# EUROPÄISCHE PATENTSCHRIFT

(11) **EP 1 773 787 B1**
(45) Veröffentlichungstag und Bekanntmachung des Hinweises auf die Patenterteilung: **02.04.2014**
(21) Anmeldenummer: 05763570.8
(22) Anmeldetag: 13.07.2005
(51) Int. Cl.: C07D 239/54, C07C 307/06

(54) **VERFAHREN ZUR HERSTELLUNG VON 3-PHENYL(THIO)URACILEN UND - DITHIOURACILEN**
METHOD FOR THE PRODUCTION OF 3-PHENYL(THIO)URACILS AND DITHIOURACILS
PROCÉDÉ DE PRODUCTION DE 3-PHENYL(THIO)URACILES ET DE 3-PHENYL-DITHIO-URACILES

(30) Priorität: 22.07.2004 DE 102004035656
(43) Veröffentlichungstag der Anmeldung: 18.04.2007
(73) Patentinhaber: BASF SE, 67056 Ludwigshafen (DE)
(72) Erfinder: LÖHR, Sandra, 67059 Ludwigshafen (DE); GEBHARDT, Joachim, 67157 Wachenheim (DE); MAYER, Guido, 67161 Gönnheim (DE); KEIL, Michael, 67251 Freinsheim (DE); SCHMIDT, Thomas, 67433 Neustadt (DE); WOLF, Bernd, 67136 Fussgönheim (DE)
(86) Internationale Anmeldenummer: PCT/EP2005/007577
(87) Internationale Veröffentlichungsnummer: WO 2006/010474

(56) Entgegenhaltungen:
- WO-A-01/83459
- WO-A-89/02891
- WO-A-98/06706
- WO-A-03/097589
- WO-A-2004/039768

## Beschreibung

Die vorliegende Erfindung betrifft ein Verfahren zur Herstellung von 3-Phenyl(thio)-uracilen und -dithiouracilen der Formel I 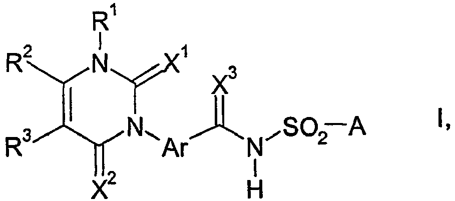 worin die Variablen die folgenden Bedeutungen haben:
- R¹: Wasserstoff, C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Halogenalkinyl, Phenyl-C₁-C₄-alkyl oder Amino;
- R² und R³: unabhängig voneinander Wasserstoff, C₁-C₆-Alkyl, C₁-C₆-Halogenalkyl, C₃-C₈-Cycloalkyl, C₂-C₆-Alkenyl, C₂-C₆-Halogenalkenyl, C₃-C₆-Alkinyl oder C₃-C₆-Halogenalkinyl;
- X¹, X² und X³: unabhängig voneinander Sauerstoff oder Schwefel;
- Ar: Phenyl, das partiell oder vollständig halogeniert sein kann und/oder ein bis drei Reste aus der Gruppe Cyano, C₁-C₄-Alkyl oder C₁-C₄-Halogenalkyl tragen kann, und
- A: ein von einem primären oder sekundären Amin abgeleiteter Rest oder NH₂.

Penyluracile, welche in meta-Position zum Uracilring am Phenylring einen Heterocyclus bzw. einen ungesättigten Ester, Thioester oder Amidrest, welcher über ein Sauer-stoff- oder Schwefelatom mit dem Phenylring verknüpft ist, tragen, sind aus WO 04/056785 bekannt

3-Phenyluracile der allgemeinen Formel I und die entsprechenden Thio- und Dithiouracile sind prinzipiell aus der WO 01/83459 bekannt.

Ihre Herstellung erfolgt gemäß der in WO 01/83459 angegebenen Lehre durch die folgenden Verfahren A bis C.

In den nachfolgenden Schemata A bis C haben die Variablen Ar und A unter anderem die zuvor genannten Bedeutungen, Hal steht für Halogen und Q steht für einen gegebenenfalls substituierten Heterocyclus:

### Verfahren A:

Kondensation einer substituierten Benzoesäure mit einem substituierten Sulfonsäurediamid in Gegenwart von N,N-Carbonyldiimidazol (CDI) oder Umwandlung der Carbonsäure in ihr Säurechlorid und anschließende Umsetzung des Säurechlorids mit einem Sulfonsäurediamid gemäß folgendem Schema A:

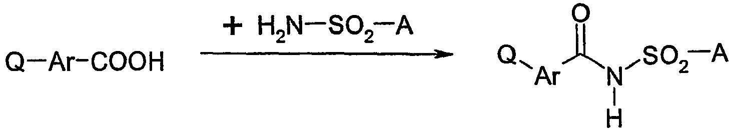

Nachteilig an dieser Vorgehensweise ist, dass die eingesetzte Benzoesäure erst durch Spaltung mit Bortribromid bei entsprechendem Salzanfall aus dem vorangehenden Ester erhältlich ist Zudem liegt die Ausbeute der Kondensation mit Sulfonsäurediamiden nur zwischen 16 und 45 %. Auch der Umweg über ein vorher hergestelltes Säurechlorid führt in nur 26 % Ausbeute zu dem gewünschten Benzoylsulfonsäurediamid.

### Verfahren B:

Ersatz eines Halogenatoms durch einen Uracil-, Thiouracil- oder Dithiouracilrest gemäß folgendem Schema B:

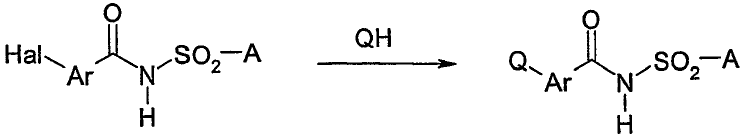

Das Verfahren B weist den Nachteil auf, dass der eingesetzte Halogenaromat erst umständlich über eine Sandmeyer-Reaktion bereitgestellt werden muss. Außerdem ist die Selektivität der Reaktion bezüglich des Halogenrestes bei Vorliegen weiterer Halogensubstituenten an Ar unbefriedigend.

### Verfahren C:

Umsetzung einer Anilinverbindung mit einem Oxazinon und anschließende Alkylierung des erhaltenen 3-Phenyluracils in Gegenwart einer Base gemäß folgendem Schema C:

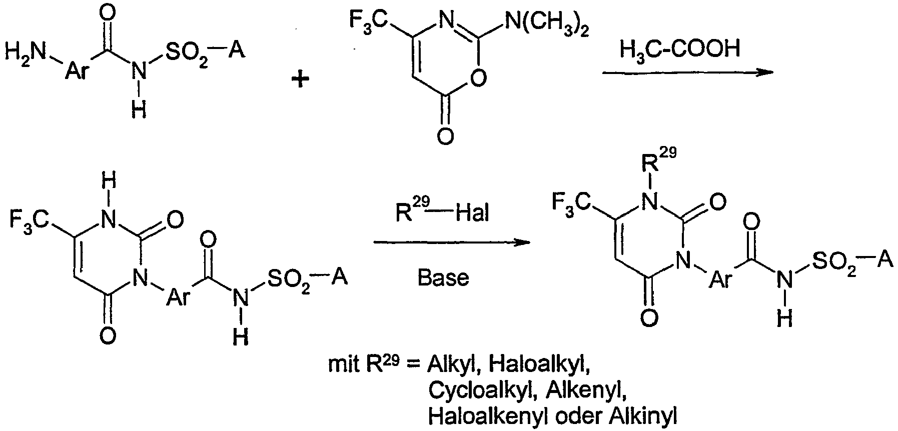

Von Nachteil ist, dass das verwendete Oxazinon aufwendig durch Umsetzung eines Aminocrotonsäureesters mit einem Dialkylcarbamoylchlorid und anschließende Zyklisierung mit Phosphoroxychlorid, Phosphorpentachlorid oder Oxalylchlorid hergestellt werden muss. Dieses Verfahren ist ebenfalls aufgrund der eingesetzten Ausgangsmaterialien und der Reaktionsstufen nicht ausreichend wirtschaftlich.

Der vorliegenden Erfindung liegt daher die Aufgabe zugrunde, Verfahren zur Herstellung von 3-Phenyl(thio)uracilen und -dithiouracilen der Formel I bereitzustellen, das die 3-Phenyl(thio)uracile und -dithiouracile der Formel I in hohen Ausbeuten und guter Reinheit liefert, und zudem die geschilderten Nachteile des Standes der Technik überwindet.

Der vorliegenden Erfindung liegt weiterhin die Aufgabe zugrunde, ein einfaches und gut zu handhabendes Verfahren zur Herstellung von Carbamaten der Formel II bereitzustellen, das die Carbamate der Formel II in hohen Ausbeuten und guter Reinheit liefert.

Weiterhin liegt der vorliegenden Erfindung die Aufgabe zugrunde, ein Verfahren zur Herstellung von 3-Phenyl(thio)uracilen und -dithiouracilen der Formel I bereitzustellen, welches zusätzlich das Verfahren zur Herstellung der Carbamate der Formel II umfasst

Es wurde überraschenderweise gefunden, dass diese Aufgabe durch ein Verfahren gelöst wird, bei dem Carbamate der Formel II 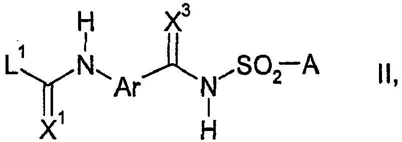 worin die Variablen X¹, X³, Ar und A die zuvor genannten Bedeutungen haben und L¹ für eine nucleophil verdrängbare Abgangsgruppe steht;
mit Enaminen der Formel III 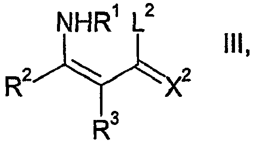 worin die Variablen X², R¹, R² und R³ die zuvor genannten Bedeutungen haben und L² für eine nucleophil verdrängbare Abgangsgruppe steht; umgesetzt werden.

Gegenstand der vorliegenden Erfindung ist daher ein Verfahren zur Herstellung der vorstehend definierten 3-Phenyl(thio)uracile und -dithiouracile der Formel I, umfassend die Umsetzung von Carbamaten der Formel II mit einem Enamin der Formel III.

Die Carbamate der Formel II können ihrerseits in Analogie zu bekannten Verfahren des Standes der Technik (z. B. Houben-Weyl, Methoden der organischen Chemie, E5, 1985, S. 972-980, sowie VIII, S. 655 und XI Teil 2, S. 10; J. B. Press et al, J. Het. Chem., 23, 6, 1986, S. 1821-1828; I. Vanthey et al., Tetrahedron Lett. 41, 33, 2000, S. 6347-6350; M. Belley et al., Synlett, 2, 2001, S. 222-225) aus Aminen der Formel IV 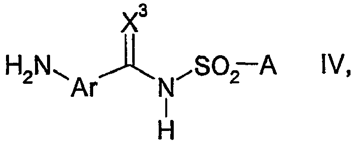 worin X³, Ar und A die zuvor genannten Bedeutungen haben, durch Umsetzung mit einer Verbindung der Formel V 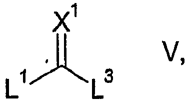 worin X¹ und L¹ die zuvor genannten Bedeutungen haben und L³ für eine nucleophil verdrängbare Abgangsgruppe steht; hergestellt werden.

Demnach umfasst das erfindungsgemäße Verfahren vorzugsweise auch die Bereitstellung der Carbamate der Formel II auf diesem Wege.

Die Carbamate der Formel II sind neu und als Ausgangs- bzw. Zwischenprodukte im erfindungsgemäßen Verfahren ebenfalls Gegenstand der vorliegenden Erfindung.

Die bei der Definition der Substituenten R¹-R³, Ar und A oder als Reste an Phenylringen genannten organischen Molekülteile stellen - wie die Bedeutung Halogen - Sammelbegriffe für individuelle Aufzählungen der einzelnen Gruppenmitglieder dar, wobei der Ausdruck Cₙ-Cₘ die mögliche Anzahl der Kohlenstoffatome im Molekülteil angibt. Sämtliche Kohlenwasserstoffketten, also alle Alkyl-, Halogenalkyl-, Cyanoalkyl-, Cyanoalkoxy-, Alkoxy-, Halogenalkoxy-, Alkylthio-, Alkylsulfinyl-, Alkylsulfonyl-, Dialkylamino-, Alkylcarbonyl-, Alkoxycarbonyl-, Alkylaminocarbonyl-, Dialkylaminocarbonyl-, Alkenyl-, Halogenalkenyl-, Alkenyloxy-, Halogenalkenyloxy-, Alkinyl-, Halogenalkinyl-, Alkinyloxy-, Halogenalkinyloxy-, Alkoxyalkoxy- und Alkylthioalkoxy-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner stehen beispielsweise:
- C₁-C₄-Alkyl: z.B. Methyl, Ethyl, n-Propyl, 1-Methylethyl, n-Butyl, 1-Methylpropyl, 2-Methylpropyl und 1,1-Dimethylethyl;
- C₁-C₆-Alkyl: C₁-C₄-Alkyl, wie voranstehend genannt, sowie z.B. n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
- C₁-C₄-Alkylcarbonyl: z.B. Methylcarbonyl, Ethylcarbonyl, Propylcarbonyl, 1-Methylethylcarbonyl, Butylcarbonyl, 1-Methylpropylcarbonyl, 2-Methylpropylcarbonyl oder 1,1-Dimethylethylcarbonyl;
- C₃-C₈-Cycloalkyl sowie die Cycloalkyteile von C₃-C₈-Cycloalkoxy: z.B. Cyclopropyl, Cyclobutyl, Cyclopentyl, Cyclohexyl, Cycloheptyl und Cycloocytyl;
- C₃-C₈-Cycloalkenyl: z.B. Cyclopropen1-yl, Cyclopropen-2-yl, Cyclobuten-1-yl, Cyclobuten-2-yl, Cyclopenten-1-yl, Cyclopent-2-en-1-yl, Cyclopent-2,4-dien-1-yl, Cyclohenex-1-yl, Cyclohen-2-en-1-yl, Cyclohen-3-en-1-yl; Cyclohepten-1-yl, Cyclohept-2-en-1-yl, Cyclohept-3-en-1-yl, Cycloocten-1-yl, Cyclooct-2-en-1-yl, Cyclooct-3-en-1-yl, Cyclooct-4-en-1-yl;
- 3- bis 6-gliedriges Heterocyclyl: ein gesättigter, partiell ungesättigter oder aromatischer 3-, 4-, 5- oder 6-gliedriger heterocyclischer Ring, der ein bis vier gleiche oder verschiedene Heteroatome, ausgewählt aus der Gruppe Sauerstoff, Schwefel, Stickstoff oder der Gruppe NR⁶ (worin R⁶ für Wasserstoff, C₁-C₆-Alkyl, C₃-C₆-Alkenyl oder C₃-C₆-Alkinyl steht) enthält, gegebenenfalls ein oder zwei Carbonylgruppen oder Thiocarbonylgrupen als Ringglieder aufweisen kann und über C oder N gebunden sein kann:
   z.B. 2-Oxrianyl, 2-Oxetanyl, 3-Oxetanyl, 2-Aziridinyl, 3-Thiethanyl, 1-Azetidinyl, 2-Azetidinyl,
   z.B. Tetrahydrofuran-2-yl, Tetrahydrofuran-3-yl, Tetrahydrothien-2-yl, Tetrahydrothien-3-yl, Tetrahydropyrrol-2-yl, Tetrahydropyrrol-3-yl, Tetrahydropyrazol-3-yl, Tetrahydro-pyrazol-4-yl, Tetrahydroisoxazol-3-yl, Tetrahydroisoxazol-4-yl, Tetrahydroisoxazol-5-yl, 1,2-Oxathiolan-3-yl, 1,2-Oxathiolan-4-yl, 1,2-Oxathiolan-5-yl, Tetrahydroisothiazol-3-yl, Tetrahydroisothiazol-4-yl, Tetrahydroisothiazol-5-yl, 1,2-Dithiolan-3-yl, 1,2-Dithiolan-4-yl, Tetrahydroimidazol-2-yl, Tetrahydroimidazol-4-yl, Tetrahydrooxazol-2-yl, Tetrahydrooxazol-4-yl, Tetrahydrooxazol-5-yl, Tetrahydrothiazol-2-yl, Tetrahydrothiazol-4-yl, Tetrahydrothiazol-5-yl, 1,3-Dioxolan-2-yl, 1,3-Dioxolan-4-yl, 1,3-Oxathiolan-2-yl, 1,3-Oxathiolan-4-yl, 1,3-Oxathiolan-5-yl, 1,3-Dithiolan-2-yl, 1,3-Dithiolan-4-yl, 1,3,2-Dioxathiolan-4-yl;
   z.B. Tetrahydropyrrol-1-yl, Tetrahydropyrazol-1-yl, Tetrahydroisoxazol-2-yl, Tetrahydroisothiazol-2-yl, Tetrahydroimidazol-1-yl, Tetrahydrooxazol-3-yl, Tetrahydrothiazol-3-yl;
   z.B. 2,3-Dihydrofuran-2-yl, 2,3-Dihydrofuran-3-yl, 2,5-Dihydrofuran-2-yl, 2,5-Di-hydrofuran-3-yl, 4,5-Dihydrofuran-2-yl, 4,5-Dihydrofuran-3-yl, 2,3-Dihydrothien-2-yl, 2,3-Dihydrothien-3-yl, 2,5-Dihydrothien-2-yl, 2,5-Dihydrothien-3-yl, 4,5-Dihydrothien-2-yl, 4,5-Dihydrothien-3-yl, 2,3-Dihydro-1H-pyrrol-2-yl, 2,3-Dihydro-1H-pyrrol-3-yl, 2,5-Dihydro-1H-pyrrol-2-yl, 2,5-Dihydro-1H-pyrrol-3-yl, 4,5-Dihydro-1H-pyrrol-2-yl, 4,5-Dihydro-1H-pyrrol-3-yl, 3,4-Dihydro-2H-pyrrol-2-yl, 3,4-Dihydro-2H-pyrrol-3-yl, 3,4-Dihydro-5H-pyrrol-2-yl, 3,4-Dihydro-5H-pyrrol-3-yl, 4,5-Dihydro-1H-pyrazol-3-yl, 4,5-Dihydro-1H-pyrazol-4-yl, 4,5-Dihydro-1H-pyrazol-5-yl, 2,5-Dihydro-1H-pyrazol-3-yl, 2,5-Dihydro-1H-pyrazol-4-yl, 2,5-Dihydro-1H-pyrazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydroisoxazol-5-yl, 2,3-Dihydroisoxazol-3-yl, 2,3-Dihydroisoxazol-4-yl, 2,3-Dihydroisoxazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydroisothiazol-3-yl, 2,3-Dihydroisothiazol-4-yl, 2,3-Dihydroisothiazol-5-yl, Δ³-1,2-Dithiol-3-yl, Δ³-1,2-Dithiol-4-yl, Δ³-1,2-Dithiol-5-yl, 4,5-Dihydro-1H-imidazol-2-yl, 4,5-Dihydro-1H-imidazol-4-yl, 4,5-Dihydro-1H-imidazol-5-yl, 2,5-Dihydro-1H-imidazol-2-yl, 2,5-Dihydro-1H-imidazol-4-yl, 2,5-Dihydro-1H-imidazol-5-yl, 2,3-Dihydro-1H-imidazol-2-yl, 2,3-Dihydro-1H-imidazol-4-yl, 4,5-Dihydro-oxazol-2-yl, 4,5-Dihydrooxazol-4-yl, 4,5-Dihydrooxazol-5-yl, 2,5-Dihydro-oxazol-2-yl, 2,5-Dihydrooxazol-4-yl, 2,5-Dihydrooxazol-5-yl, 2,3-Dihydro-oxazol-2-yl, 2,3-Dihydrooxazol-4-yl, 2,3-Dihydrooxazol-5-yl, 4,5-Dihydro-thiazol-2-yl, 4,5-Dihydrothiazol-4-yl, 4,5-Dihydrothiazol-5-yl, 2,5-Dihydro-thiazol-2-yl, 2,5-Dihydrothiazol-4-yl, 2,5-Dihydrothiazol-5-yl, 2,3-Dihydro-thiazol-2-yl, 2,3-Dihydrothiazol-4-yl, 2,3-Dihydrothiazol-5-yl, 1,3-Dioxol-2-yl, 1,3-Dioxol-4-yl, 1,3-Dithiol-2-yl, 1,3-Dithiol-4-yl, 1,3-Oxathiol-2-yl, 1,3-Oxa-thiol-4-yl, 1,3-Oxathiol-5-yl, 1,2,3-Δ²-Oxadiazolin-4-yl, 1,2,3-Δ²-Oxadiazolin-5-yl, 1,2,4-Δ⁴-Oxadiazolin-3-yl, 1,2,4-Δ⁴-Oxadiazolin-5-yl, 1,2,4-Δ²-Oxadia-zolin-3-yl, 1,2,4-Δ²-Oxadiazolin-5-yl, 1,2,4-Δ³-Oxadiazolin-3-yl, 1,2,4-Δ³-Oxadiazolin-5-yl, 1,3,4-Δ²-Oxadiazolin-2-yl, 1,3,4-Δ²-Oxadiazolin-5-yl, 1,3,4-Δ³-Oxadiazolin-2-yl, 1,3,4-Oxadiazolin-2-yl, 1,2,4-Δ⁴-Thiadiazolin-3-yl, 1,2,4-Δ⁴-Thiadiazolin-5-yl, 1,2,4-Δ³-Thiadiazolin-3-yl, 1,2,4-Δ³-Thiadiazolin-5-yl, 1,2,4-Δ²-Thiadiazolin-3-yl, 1,2,4-Δ²-Thiadiazolin-5-yl, 1,3,4-Δ²-Thiadiazolin-2-yl, 1,3,4-Δ²-Thiadiazolin-5-yl, 1,3,4-Δ³-Thiadiazolin-2-yl, 1,3,4-Thiadiazolin-2-yl, 1,2,3-Δ²-Triazolin-4-yl, 1,2,3-Δ²-Triazolin-5-yl, 1,2,4-Δ²-Triazolin-3-yl, 1,2,4-Δ²-Triazolin-5-yl, 1,2,4-Δ³-Triazolin-3-yl, 1,2,4-Δ³-Triazolin-5-yl, 1,2,4-Δ¹-Triazolin-2-yl, 1,2,4-Triazolin-3-yl, 3H-1,2,4-Dithiazol-5-yl, 2H-1,3,4-Dithiazol-5-yl, 2H-1,3,4-Oxathiazol-5-yl;
   z.B. 2,3-Dihydro-1H-pyrrol-1-yl, 2,5-Dihydro-1H-pyrrol-1-yl, 4,5-Dihydro-1H-pyrazol-1-yl, 2,5-Dihydro-1H-pyrazol-1-yl, 2,3-Dihydro-1H-pyrazol-1-yl, 2,5-Dihydroisoxazol-2-yl, 2,3-Dihydroisoxazol-2-yl, 2,5-Dihydroisothiazol-2-yl, 2,3-Dihydroisoxazol-2-yl, 4,5-Dihydro-1H-imidazol-1-yl, 2,5-Dihydro-1H-imidazol-1-yl, 2,3-Dihydro-1H-imidazol-1-yl, 2,3-Dihydrooxazol-3-yl, 2,3-Dihydrothiazol-3-yl, 1,2,4-Δ⁴-Oxadiazolin-2-yl, 1,2,4-Δ²-Oxadiazolin-4-yl, 1,2,4-Δ³-Oxadiazolin-2-yl, 1,3,4-Δ²-Oxadiazolin-4-yl, 1,2,4-Δ⁵-Thiadiazolin-2-yl, 1,2,4-Δ³-Thiadiazolin-2-yl, 1,2,4-Δ²-Thiadiazolin-4-yl, 1,3,4-Δ²-Thiadiazolin-4-yl, 1,2,3-Δ²-Triazolin-1-yl, 1,2,4-Δ²-Triazolin-1-yl, 1,2,4-Δ²-Triazolin-4-yl, 1,2,4-Δ³-Triazolin-1-yl, 1,2,4-Δ¹-Triazolin-4-yl;
   z.B. 2-Furyl, 3-Furyl, 2-Thienyl, 3-thienyl, Pyrrol-2-yl, Pyrrol-3-yl, Pyrazol-3-yl, Pyrazol-4-yl, Isoxazol-3-yl, Isoxazol-4-yl, Isoxazol-5-yl, Isothiazol-3-yl, Isothiazol-4-yl, Isothiazol-5-yl, Imidazol-2-yl, Imidazol-4-yl, Oxazol-2-yl, Oxazol-4-yl, Oxazol-5-yl, Thiazol-2-yl, Thiazol-4-yl, Thiazol-5-yl, 1,2,3-Oxadiazol-4-yl, 1,2,3-Oxadiazol-5-yl, 1,2,4-Oxadiazol-3-yl, 1,2,4,-Oxadiazol-5-yl, 1,3,4-Oxadiazol-2-yl, 1,2,3-Thiadiazol-4-yl, 1,2,3-Thiadiazol-5-yl, 1,2,4-Thiadiazol-3-yl, 1,2,4-Thiadiazol-5-yl, 1,3,4-Thiadiazolyl-2-yl, 1,2,3-Triazol-4-yl, 1,2,4-Triazol-3-yl, Tetrazol-5-yl;
   z.B. Pyrrol-1-yl, Pyrazol-1-yl, Imidazol-1-yl, 1,2,3-Triazol-1-yl, 1,2,4-Triazol-1-yl, Tetrazol-1-yl;
   z.B. Tetrahydropyran-2-yl, Tetrahydropyran-3-yl, Tetrahydropyran-4-yl, Piperidin-2-yl, Piperidin-3-yl, Piperidin-4-yl, Tetrahydrothiopyran-2-yl, Tetrahydrothiopyran-3-yl, Tetrahydrothiopyran-4-yl, 1,3-Dioxan-2-yl, 1,3-Dioxan-4-yl, 1,3-Dioxan-5-yl, 1,4-Dioxan-2-yl, 1,3-Dithian-2-yl, 1,3-Dithian-4-yl, 1,3-Dithian-5-yl, 1,4-Dithian-2-yl, 1,3-Oxathian-2-yl, 1,3-Oxathian-4-yl, 1,3-Oxathian-5-yl, 1,3-Oxathian-6-yl, 1,4-Oxathian-2-yl, 1,4-Oxathian-3-yl, 1,2-Dithian-3-yl, 1,2-Dithian-4-yl, Hexahydropyrimidin-2-yl, Hexahydropyrimidin-4-yl, Hexahydropyrimidin-5-yl, Hexahydropyrazin-2-yl, Hexahydropyridazin-3-yl, Hexahydropyridazin-4-yl, Tetrahydro-1,3-oxazin-2-yl, Tetrahydro-1,3-oxazin-4-yl, Tetrahydro-1,3-oxazin-5-yl, Tetrahydro-1,3-oxazin-6-yl, Tetrahydro-1,3-thiazin-2-yl, Tetrahydro-1,3-thiazin-4-yl, Tetrahydro-1,3-thiazin-5-yl, Tetrahydro-1,3-thiazin-6-yl, Tetrahydro-1,4-thiazin-2-yl, Tetrahydro-1,4-thiazin-3-yl, Tetrahydro-1,4-oxazin-2-yl, Tetrahydro-1,4-oxazin-3-yl, Tetrahydro-1,2-oxazin-3-yl, Tetrahydro-1,2-oxazin-4-yl, Tetrahydro-1,2-oxazin-5-yl, Tetrahydro-1,2-oxazin-6-yl;
   z.B. Piperidin-1-yl, Hexahydropyrimidin-1-yl, Hexahydropyrazin-1-yl, Hexahydropyridazin-1-yl, Tetrahydro-1,3-oxazin-3-yl, Tetrahydro-1,3-thiazin-3-yl, Tetrahydro-1,4-thiazin-4-yl, Tetrahydro-1,4-oxazin-4-yl, Tetrahydro-1,2-oxazin-2-yl;
   z.B. 2H-3,4-Dihydropyran-6-yl, 2H-3,4-Dihydropyran-5-yl, 2H-3,4-Dihydropyran-4-yl, 2H-3,4-Dihydropyran-3-yl, 2H-3,4-Dihydropyran-2-yl, 2H-3,4-Dihydro-pyran-6-yl, 2H-3,4-Dihydrothiopyran-5-yl, 2H-3,4-Dihydrothiopyran-4-yl, 2H-3,4-Dihydropyran-3-yl, 2H-3,4-Dihydropyran-2-yl, 1,2,3,4-Tetrahydropyridin-6-yl, 1,2,3,4-Tetrahydropyridin-5-yl, 1,2,3,4-Tetrahydropyridin-4-yl, 1,2,3,4-Tetra-hydropyridin-3-yl, 1,2,3,4-Tetrahydropyridin-2-yl, 2H-5,6-Dihydropyran-2-yl, 2H-5,6-Dihydropyran-3-yl, 2H-5,6-Dihydropyran-4-yl, 2H-5,6-Dihydropyran-5-yl, 2H-5,6-Dihydropyran-6-yl, 2H-5,6-Dihydrothiopyran-2-yl, 2H-5,6-Dihydro-thiopyran-3-yl, 2H-5,6-Dihydrothiopyran-4-yl, 2H-5,6-Dihydrothiopyran-5-yl, 2H-5,6-Dihydrothiopyran-6-yl, 1,2,5,6-Tetrahydropyridin-2-yl, 1,2,5,6 Tetrahydropyridin-3-yl, 1,2,5,6-Tetrahydropyridin-4-yl, 1,2,5,6-Tetrahydro-pyridin-5-yl, 1,2,5,6-Tetrahydropyridin-6-yl, 2,3,4,5-Tetrahydropyridin-2-yl, 2,3,4,5-Tetrahydropyridin-3-yl, 2,3,4,5-Tetrahydropyridin-4-yl, 2,3,4,5-Tetrahydropyridin-5-yl, 2,3,4,5-Tetrahydropyridin-6-yl, 4H-Pyran-2-yl, 4H-Pyran-3-yl-, 4H-Pyran-4-yl, 4H-Thiopyran-2-yl, 4H-Thiopyran-3-yl, 4H-Thiopyran-4-yl, 1,4-Dihydropyridin-2-yl, 1,4-Dihydropyridin-3-yl, 1,4-Dihydropyridin-4-yl, 2H-Pyran-2-yl, 2H-Pyran-3-yl, 2H-Pyran-4-yl, 2H-Pyran-5-yl, 2H-Pyran-6-yl, 2H-Thiopyran-2-yl, 2H-Thiopyran-3-yl, 2H-Thiopyran-4-yl, 2H-Thiopyran-5-yl, 2H-Thiopyran-6-yl, 1,2-Dihydropyridin-2-yl, 1,2-Dihydro-pyridin-3-yl, 1,2-Dihydropyridin-4-yl, 1,2-Dihydropyridin-5-yl, 1,2-Dihydro-pyridin-6-yl, 3,4-Dihydropyridin-2-yl, 3,4-Dihydropyridin-3-yl, 3,4-Dihydro-pyridin-4-yl, 3,4-Dihydropyridin-5-yl, 3,4-Dihydropyridin-6-yl, 2,5-Dihydro-pyridin-2-yl, 2,5-Dihydropyridin-3-yl, 2,5-Dihydropyridin-4-yl, 2,5-Dihydro-pyridin-5-yl, 2,5-Dihydropyridin-6-yl, 2,3-Dihydropyridin-2-yl, 2,3-Dihydro-pyridin-3-yl, 2,3-Dihydropyridin-4-yl, 2,3-Dihydropyridin-5-yl, 2,3-Dihydro-pyridin-6-yl, 2H-5,6-Dihydro-1,2-oxazin-3-yl, 2H-5,6-Dihydro-1,2-oxazin-4-yl, 2H-5,6-Dihydro-1,2-oxazin-5-yl, 2H-5,6-Dihydro-1,2-oxazin-6-yl, 2H-5,6-Dihydro-1,2-thiazin-3-yl, 2H-5,6-Dihydro-1,2-thiazin-4-yl, 2H-5,6-Dihydro-1,2-thiazin-5-yl, 2H-5,6-Dihydro-1,2-thiazin-6-yl, 4H-5,6-Dihydro-1,2-oxazin-3-yl, 4H-5,6-Dihydro-1,2-oxazin-4-yl, 4H-5,6-Dihydro-1,2-oxazin-5-yl, 4H-5,6-Dihydro-1,2-oxazin-6-yl, 4H-5,6-Dihydro-1,2-thiazin-3-yl, 4H-5,6-Dihydro-1,2-thiazin-4-yl, 4H-5,6-Dihydro-1,2-thiazin-5-yl, 4H-5,6-Dihydro-1,2-thiazin-6-yl, 2H-3,6-Dihydro-1,2-oxazin-3-yl, 2H-3,6-Dihydro-1,2-oxazin-4-yl, 2H-3,6-Dihydro-1,2-oxazin-5-yl, 2H-3,6-Dihydro-1,2-oxazin-6-yl, 2H-3,6-Dihydro-1,2-thiazin-3-yl, 2H-3,6-Dihydro-1,2-thiazin-4-yl, 2H-3,6-Di-hydro-1,2-thiazin-5-yl, 2H-3,6-Dihydro-1,2-thiazin-6-yl, 2H-3,4-Dihydro-1,2-oxazin-3-yl, 2H-3,4-Dihydro-1,2-oxazin-4-yl, 2H-3,4-Dihydro-1,2-oxazin-5-yl, 2H-3,4-Dihydro-1,2-oxazin-6-yl, 2H-3,4-Dihydro-1,2-thiazin-3-yl, 2H-3,4-Dihydro-1,2-thiazin-4-yl, 2H-3,4-Dihydro-1,2-thiazin-5-yl, 2H-3,4-Dihydro-1,2-thiazin-6-yl, 2,3,4,5-Tetrahydropyridazin-3-yl, 2,3,4,5-Tetrahydropyridazin-4-yl, 2,3,4,5-Tetrahydropyridazin-5-yl, 2,3,4,5-Tetrahydropyridazin-6-yl, 3,4,5,6-Tetrahydropyridazin-3-yl, 3,4,5,6-Tetrahydropyridazin-4-yl, 1,2,5,6-Tetrahydropyridazin-3-yl, 1,2,5,6-Tetrahydropyridazin-4-yl, 1,2,5,6-Tetra-hydropyridazin-5-yl, 1,2,5,6-Tetrahydropyridazin-6-yl, 1,2,3,6-Tetrahydro-pyridazin-3-yl, 1,2,3,6-Tetrahydropyridazin-4-yl, 4H-5,6-Dihydro-1,3-oxazin-2-yl, 4H-5,6-Dihydro-1,3-oxazin-4-yl, 4H-5,6-Dihydro-1,3-oxazin-5-yl, 4H-5,6-Dihydro-1,3-oxain-6-yl, 4H-5,6-Dihydro-1,3-thiazin-2-yl, 4H-5,6-Dihydro-1,3-thiazin-4-yl, 4H-5,6-Dihydro-1,3-thiazin-5-yl, 4H-5,6-Dihydro-1,3-thiazin-6-yl, 3,4,5-6-Tetrahydropyrimidin-2-yl, 3,4,5,6-Tetrahydropyrimidin-4-yl, 3,4,5,6-Tetrahydropyrimidin-5-yl, 3,4,5,6-Tetrahydropyrimidin-6-yl, 1,2,3,4-Tetrahydropyrazin-2-yl, 1,2,3,4-Tetrahydropyrazin-5-yl, 1,2,3,4-Tetrahydro-pyrimidin-2-yl, 1,2,3,4-Tetrahydropyrimidin-4-yl, 1,2,3,4-Tetrahydropyrimidin-5-yl, 1,2,3,4-Tetrahydropyrimidin-6-yl, 2,3-Dihydro-1,4-thiazin-2-yl, 2,3-Dihydro-1,4-thiazin-3-yl, 2,3-Dihydro-1,4-thiazin-5-yl, 2,3-Dihydro-1,4-thiazin-6-yl, 2H-1,2-Oxazin-3-yl, 2H-1,2-Oxazin-4-yl, 2H-1,2-Oxazin-5-yl, 2H-1,2-Oxazin-6-yl, 2H-1,2-Thiazin-3-yl, 2H-1,2-Thiazin-4-yl, 2H-1,2-Thiazin-5-yl, 2H-1,2-Thiazin-6-yl, 4H-1,2-Oxazin-3-yl, 4H-1,2-Oxazin-4-yl, 4H-1,2-Oxazin-5-yl, 4H-1,2-Oxazin-6-yl, 4H-1,2-Thiazin-3-yl, 4H-1,2-Thiazin-4-yl, 4H-1,2-Thiazin-5-yl, 4H-1,2-Thiazin-6-yl, 6H-1,2-Oxazin-3-yl, 6H-1,2-Oxazin-4-yl, 6H-1,2-Oxazin-5-yl, 6H-1,2-Oxazin-6-yl, 6H-1,2-Thiazin-3-yl, 6H-1,2-Thiazin-4-yl, 6H-1,2-Thiazin-5-yl, 6H-1,2-Thiazin-6-yl, 2H-1,3-Oxazin-2-yl, 2H-1,3-Oxazin-4-yl, 2H-1,3-Oxazin-5-yl, 2H-1,3-Oxazin-6-yl, 2H-1,3-Thiazin-2-yl, 2H-1,3-Thiazin-4-yl, 2H-1,3-Thiazin-5-yl, 2H-1,3-Thiazin-6-yl, 4H-1,3-Oxazin-2-yl, 4H-1,3-Oxazin-4-yl, 4H-1,3-Oxazin-5-yl, 4H-1,3-Oxazin-6-yl, 4H-1,3-Thiazin-2-yl, 4H-1,3-Thiazin-4-yl, 4H-1,3-Thiazin-5-yl, 4H-1,3-Thiazin-6-yl, 6H-1,3-Oxazin-2-yl, 6H-1,3-Oxazin-4-yl, 6H-1,3-Oxazin-5-yl, 6H-1,3-Oxazin-6-yl, 6H-1,3-Thiazin-2-yl, 6H-1,3-Oxazin-4-yl, 6H-1,3-Oxazin-5-yl, 6H-1,3-Thiazin-6-yl, 2H-1,4-Oxazin-2-yl, 2H-1,4-Oxazin-3-yl, 2H-1,4-Oxazin-5-yl, 2H-1,4-Oxazin-6-yl, 2H-1,4-Thiazin-2-yl, 2H-1,4-Thiazin-3-yl, 2H-1,4-Thiazin-5-yl, 2H-1,4-Thiazin-6-yl, 4H-1,4-Oxazin-2-yl, 4H-1,4-Oxazin-3-yl, 4H-1,4-Thiazin-2-yl, 4H-1,4-Thiazin-3-yl, 1,4-Dihydropyridazin-3-yl, 1,4-Dihydropyridazin-4-yl, 1,4-Dihydropyridazin-5-yl, 1,4-Dihydropyridazin-6-yl, 1,4-Dihydropyrazin-2-yl, 1,2-Dihydropyrazin-2-yl, 1,2-Dihydropyrazin-3-yl, 1,2-Dihydropyrazin-5-yl, 1,2-Dihydropyrazin-6-yl, 1,4-Dihydropyrimidin-2-yl, 1,4-Dihydropyrimidin-4-yl, 1,4-Dihydropyrimidin-5-yl, 1,4-Dihydropyrimidin-6-yl, 3,4-Dihydropyrimidin-2-yl, 3,4-Dihydropyrimidin-4-yl, 3,4-Dihydropyrimidin-5-yl oder 3,4-Dihydropyrimidin-6-yl;
   z.B. 1,2,3,4-Tetrahydropyridin-1-yl, 1,2,5,6-Tetrahydropyridin-1-yl, 1,4-Dihydropyridin-1-yl, 1,2-Dihydropyridin-1-yl, 2H-5,6-Dihydro-1,2-oxazin-2-yl, 2H-5,6-Dihydro-1,2-thiazin-2-yl, 2H-3,6-Dihydro-1,2-oxazin-2-yl, 2H-3,6-Dihydro-1,2-thiazin-2-yl, 2H-3,4-Dihydro-1,2-oxazin-2-yl, 2H-3,4-Dihydro-1,2-thiazin-2-yl, 2,3,4,5-Tetrahydropyridazin-2-yl, 1,2,5,6-Tetrahydropyridazin-1-yl, 1,2,5,6-Tetrahydropyridazin-2-yl, 1,2,3,6-Tetrahydropyridazin-1-yl, 3,4,5,6-Tetrahydropyrimidin-3-yl, 1,2,3,4-Tetrahydropyrazin-1-yl, 1,2,3,4-Tetrahydropyrimidin-1-yl, 1,2,3,4-Tetrahydropyrimidin-3-yl, 2,3-Dihdro-1,4-thiazin-4-yl, 2H-1,2-Oxazin-2-yl, 2H-1,2-Thiazin-2-yl, 4H-1,4-Oxazin-4-yl, 4H-1,4-Thiazin-4-yl, 1,4-Dihydropyridazin-1-yl, 1,4-Dihydropyrazin-1-yl, 1,2-Dihydropyrazin-1-yl, 1,4-Dihydropyrimidin-1-yl oder 3,4-Dihydropyrimidin-3-yl;
   z.B. Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl, 1,2,4-Triazin-3-yl, 1,2,4-Triazin-5-yl, 1,2,4-Triazin-6-yl, 1,2,4,5-Tetrazin-3-yl;
- C₃-C₆-Alkenyl: z.B. 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
- C₂-C₆-Alkenyl: C₃-C₆-Alkenyl wie voranstehend genannt sowie Ethenyl,
- C₃-C₆-Alkinyl: z.B. 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
- C₂-C₆-Alkinyl: C₃-C₆-Akinyl wie voranstehend genannt sowie Ethinyl;
- C₁-C₄-Halogenalkyl: einen C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-lodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
- C₁-C₆-Halogenalkyl: C₁-C₄-Halogenalkyl wie voranstehend genannt, sowie z.B. 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-lodhexyl und Dodecafluorhexyl;
- C₂-C₆-Halogenalkenyl sowie die Halogenalkenylteile von C₂-C₆-Halogenalkenyloxy: ein C₂-C₆-Alkenylrest, wie voranstehend genannt, der partiell oder vollständig durch Fluor, Chlor, Brom und/oder Iod substituiert ist, z.B. 2-Chlorvinyl, 2-Chlorallyl, 3-Chlorallyl, 2,3-Dichlorallyl, 3,3-Dichlorallyl, 2,3,3-Trichlorallyl, 2,3-Dichlorbut-2-enyl, 2-Bromvinyl, 2-Bromallyl, 3-Bromallyl, 2,3-Dibromallyl, 3,3-Dibromallyl, 2,3,3-Tribromallyl oder 2,3-Dibrombut-2-enyl;
- C₃-C₆-Halogenalkinyl sowie die Halogenalkinylteile von C₃-C₆-Halogenalkinyloxy: ein C₃-C₆-Alkinylrest, wie voranstehend genannt, der partiell oder vollständig durch Fluor, Chlor, Brom und/oder Iod substituiert ist, z.B. 1,1-Difluor-prop-2-in-1-yl, 3-Iod-prop-2-in-1-yl, 4-Fluorbut-2-in-1-yl, 4-Chlorbut-2in-1-yl, 1,1-Difluorbut-2-in-1-yl, 4-Iodbut-3-in-1-yl, 5-Fluorpent-3-in-1-yl, 5-Iodpent-4-in-1-yl, 6-Fluorhex-4-in-1-yl oder 6-Iodhex-5-in-1-yl;
- C₁-C₄-Cyanoalkyl: z.B. Cyanomethyl, 1-Cyanoeth-1-yl, 2-Cyanoeth-1-yl, 1-Cyanoprop-1-yl, 2-Cyanoprop-1-yl, 3-Cyanoprop-1-yl, 1-Cyanoprop-2-yl, 2-Cyanoprop-2-yl, 1-Cyanobut-1-yl, 2-Cyanobut-1-yl, 3-Cyanobut-1-yl, 4-Cyanobut-1-yl, 1-Cyanobut-2-yl, 2-Cyanobut-2-yl, 1-Cyanobut-3-yl, 2-Cyanobut-3-yl, 1-Cyano-2-methyl-prop-3-yl, 2-Cyano-2-methyl-prop-3-yl, 3-Cyano-2-methyl-prop-3-yl und 2-Cyano-methyl-prop-2-yl;
- C₁-C₆-Cyanoalkyl sowie die Cyanoalkylteile von C₁-C₆-Cyanoalkoxy: C₁-C₄-Cyanoalkyl wie voranstehend genannt sowie 5-Cyanopentyl, 6-Cyanohexyl;
- C₁-C₄-Alkoxy z.B. Methoxy, Ethoxy, Propoxy, 1-Methyl-ethoxy, Butoxy, 1-Methylpropoxy, 2-Methylpropoxy und 1,1-Dimethylethoxy;
- C₁-C₆-Alkoxy: C₁-C₄-Alkoxy wie voranstehend genannt, sowie z.B. Pentoxy, 1-Methylbutoxy, 2-Methylbutoxy, 3-Methoxylbutoxy, 1,1-Dimethyl-propoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Di-methylbutoxy,1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Tri-methylpropoxy, 1,2,2-Trimethylpropoxy, 1-Ethyl-1-methylpropoxy und 1-Ethyl-2-methylpropoxy;
- C₂-C₆-Alkenyloxy: z.B. Ethen-1-yloxy, Ethen-2-yloxy, Prop-1-en-1-yloxy, Prop-2-en-1-yloxy, 1-Methylethenyloxy, Buten-1-yloxy, Buten-2-yloxy, Buten-3-yloxy, 1-Methylprop-1-en-1-yloxy, 2-Methyl-prop-1-en-1-yloxy, 1-Methyl-prop-2-en-1-yloxy, 2-Methyl-prop-2-en-1-yloxy, Penten-1-yloxy, Penten-2-yloxy, Penten-3-yloxy, Peten-4-yloxy, 1-Methyl-but-1-en-1-yloxy, 2-Methyl-but-1-en-1-yloxy, 3-Methyl-but-1-en-1-yloxy, 1-Methyl-but-2-en-1-yloxy, 2-Methyl-but-2-en-1-yloxy, 3-Methyl-but-2-en-1-yloxy, 1-Methyl-but-3-en-1-yloxy, 2-Methyl-but-3-en-1-yloxy, 3-Methyl-but-3-en-1-yloxy, 1,1-Dimethyl-prop-2-en-1-yloxy, 1,2-Dimethyl-prop-1-en-1-yloxy, 1,2-Dimethyl-prop-2-en-1-yloxy, 1-Ethyl-prop-1-en-2-yloxy, 1-Ethyl-prop-2-en-1-yloxy, Hex-1-en-1-yloxy, Hex-2-en-1-yloxy, Hex-3-en-1-yloxy, Hex-4-en-1-yloxy, Hex-5-en-1-yloxy, 1-Methyl-pent-1-en-1-yloxy, 2-Methyl-pent-1-en-1-yloxy, 3-Methyl-pent-1-en-1-yloxy, 4-Methyl-pent-1-en-1-yloxy, 1-Methyl-pent-2-en-1-yloxy, 2-Methyl-pent-2-en-1-yloxy, 3-Methyl-pent-2-en-1-yloxy, 4-Methyl-pent-2-en-1-yloxy, 1-Methyl-pent-3-en-1-yloxy, 2-Methyl-pent-3-en-1-yloxy, 3-Methyl-pent-3-en-1-yloxy, 4-Methyl-pent-3-en-1-yloxy, 1-Methyl-pent-4-en-1-yloxy, 2-Methyl-pent-4-en-1-yloxy, 3-Methyl-pent-4-en-1-yloxy, 4-Methyl-pent-4-en-1-yloxy, 1,1-Dimethyl-but-2-en-1-yloxy, 1,1-Dimethyl-but-3-en-1-yloxy, 1,2-Dimethyl-but-1-en-1-yloxy, 1,2-Dimethyl-but-2-en-1-yloxy, 1,2-Dimethyl-but-3-en-1-yloxy, 1,3-Dimethyl-but-1-en-1-yloxy, 1,3-Dimethyl-but-2-en-1-yloxy, 1,3-Dimethyl-but-3-en-1-yloxy, 2,2-Dimethyl-but-3-en-1-yloxy, 2,3-Dimethyl-but-1-en-1-yloxy, 2,3-Dimethyl-but-2-en-1-yloxy, 2,3-Dimethyl-but-3-en-1-yloxy, 3,3-Dimethyl-but-1-en-1-yloxy, 3,3-Dimethyl-but-2-en-1-yloxy, 1-Ethyl-but-1-en-1-yloxy, 1-Ethyl-but-2-en-1-yloxy, 1-Ethyl-but-3-en-1-yloxy, 2-Ethyl-but-1-en-1-yloxy, 2-Ethyl-but-2-en-1-yloxy, 2-Ethyl-but-3-en-1-yloxy, 1,1,2-Trimethyl-prop-2-en-1-yloxy, 1-Ethyl-1-methyl-prop-2-en-1-yloxy, 1-Ethyl-2-methyl-prop-1-en-1-yloxy und 1-Ethyl-2-methyl-prop-2-en-1-yloxy;
- C₃-C₆-Alkinyloxy: z.B. Prop-1-in-1-yloxy, Prop-2-in-1-yloxy, But-1-in-1-yloxy, But-1-in-3-yloxy, But-1-in-4-yloxy, But-2-in-1-yloxy, Pent-1-in-1-yloxy, Pent-1-in-3-yloxy, Pent-1-in-4-yloxy, Pent-1-in-5-yloxy, Pent-2-in-1-yloxy, Pent-2-in-4-yloxy, Pent-2-in-5-yloxy, 3-Methyl-but-1-in-3-yloxy, 3-Methyl-but-1-in-4-yloxy, Hex-1-in-1-yloxy, Hex-1-in-3-yloxy, Hex-1-in-4-yloxy, Hex-1-in-5-yloxy, Hex-1-in-6-yloxy, Hex-2-in-1-yloxy, Hex-2-in-4-yloxy, Hex-2-in-5-yloxy, Hex-2-in-6-yloxy, Hex-3-in-1-yloxy, Hex-3-in-2-yloxy, 3-Methylpent-1-in-1-yloxy, 3-Methyl-pent-1-in-3-yloxy, 3-Methyl-pent-1-in-4-yloxy, 3-Methyl-pent-1-in-5-yloxy, 4-Methyl-pent-1-in-1-yloxy, 4-Methyl-pent-2-in-4-yloxy und 4-Methylpent-2-in-5-yloxy;
- C₁-C₄-Halogenalkoxy: einen C₁-C₄-Alkoxyrest wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Brommethoxy, 2-lodethoxy, 2,2-Difluorethoxy, 2,2,2-Trifluorethoxy, 2-Chlor-2-fluorethoxy, 2-Chlor-2,2-difluor-ethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy und Nonafluorbutoxy;

- C₁-C₆-Halogenalkoxy: C₁-C₄-Halogenalkoxy wie voranstehend genannt, sowie z.B. 5-Fluorpentoxy, 5-Chlorpentoxy, 5-Brompentoxy, 5-Iodpentoxy, Undecafluorpentoxy, 6-Fluorhexoxy, 6-Chlorhexoxy, 6-Bromhexoxy, 6-lodhexoxy und Dodecafluorhexoxy;
- C₁-C₄-Alkoxy-C₂-C₄-alkoxy: durch C₁-C₄-Alkoxy wie vorstehend genannt substituiertes C₂-C₄-Alkoxy, also z.B. für 2-(Methoxy)ethoxy, 2-(Ethoxy)ethoxy, 2-(Propoxy)-ethoxy, 2-(1-Methylethoxy)ethoxy, 2-(Butoxy)ethoxy, 2-(1-Methylpropoxy)-ethoxy, 2-(2-Methyl-propoxy)ethoxy, 2-(1,1-Dimethylethoxy)ethoxy, 2-(Methoxy)-propoxy, 2-(Ethoxy)-propoxy, 2-(Propoxy)propoxy, 2-(1-Methylethoxy)propoxy, 2-(Butoxy)-propoxy, 2-(1-Methylpropoxy)propoxy, 2-(2-Methylpropoxy)propoxy, 2-(1,1-Di-methylethoxy)-propoxy, 3-(Methoxy)propoxy, 3-(Ethoxy)propoxy, 3-(Propoxy)-propoxy, 3-(1-Methylethoxy)-propoxy, 3-(Butoxy)propoxy, 3-(1-Methylpropoxy)-propoxy, 3-(2-Methylpropoxy)-propoxy, 3-(1,1-Dimethylethoxy)propoxy, 2-(Meth-oxy)butoxy, 2-(Ethoxy)butoxy, 2-(Propoxy)butoxy, 2-(1-Methylethoxy)-butoxy, 2-(Butoxy)-butoxy, 2-(1-Methylpropoxy)-butoxy, 2-(2-Methylpropoxy)-butoxy, 2-(1,1-Dimethytethoxy)butoxy, 3-(Methoxy)butoxy, 3-(Ethoxy)-butoxy, 3-(Propoxy)butoxy, 3-(1-Methylethoxy)butoxy, 3-(Butoxy)butoxy, 3-(1-Methylpropoxy)butoxy, 3-(2-Methylpropoxy)butoxy, 3-(1,1-Dimethylethoxy)butoxy, 4-(Methoxy)-butoxy, 4-(Ethoxy)butoxy, 4-(Propoxy)butoxy, 4-(1-Methylethoxy)-butoxy, 4-(Butoxy)butoxy, 4-(1-Methylpropoxy)butoxy, 4-(2-Methylpropoxy)butoxy und 4-(1,1-Dimethyl-ethoxy)butoxy;
- C₁-C₄-Alkoxycarbonyl: z.B. Methoxycarbonyl, Ethoxycarbonyl, Propoxycarbonyl, 1-Methylethoxycarbonyl, Butoxycarbonyl, 1-Methylpropoxycarbonyl, 2-Methylpropoxycarbonyl oder 1,1-Dimethylethoxycarbonyl;
- C₁-C₄-Alkylthio: z.B. Methylthio, Ethylthio, Propylthio, 1-Methylethylthio, Butylthio, 1-Methylpropylthio, 2-Methylpropylthio und 1,1-Dimethylethylthio;
- C₁-C₆-Alkylthio: C₁-C₄-Alkylthio wie voranstehend genannt, sowie z.B. Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio;
- C₁-C₄-Alkylsulfinyl (C₁-C₄-Alkyl-S(=O)-): z.B. Methylsulfinyl, Ethylsulfinyl, Propylsulfinyl, 1-Methylethylsulfinyl, Butylsulfinyl, 1-Methylpropylsulfinyl, 2-Methylpropyl-sulfinyl, 1,1-Dimethylethylsulfinyl;
- C₁-C₄-Alkylsulfonyl (C₁-C₄-Alkyl-S(=O)₂-): z.B. Methylsulfonyl, Ethylsulfonyl, Propylsulfonyl, 1-Methylethylsulfonyl, Butylsulfonyl, 1-Methylpropylsulfonyl, 2-Methylpropylsulfonyl, 1,1-Dimethylethylsulfonyl;
- C₁-C₄-Alkylamino:z.B. Methylamino, Ethylamino, Propylamino, 1-Methylethylamino, Butylamino, 1-Methylpropylamino, 2-Methylpropylamino, 1,1-Dimethylethylamino;
- Di-(C₁-C₄-alkyl)-amino: z.B. N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)-amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl)amino, N-(1-Methylpropyl)-N-(2-methylpropyl)amino, N-(1,1-Dimethylethyl)-N-(1-methylpropyl)amino und N-(1,1-Dimethylethyl)-N-(2-methylpropyl)amino;
- (C₁-C₄-Alkylamino)carbonyl: z.B. Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl oder 1,1-Dimethylethylaminocarbonyl;
- Di-(C₁-C₄)-alkylaminocarbonyl: z.B. N,N-Dimethylaminocarbonyl, N,N-Diethylaminocarbonyl, N,N-Di-(1-methylethyl)aminocarbonyl, N,N-Dipropylaminocarbonyl, N,N-Dibutylaminocarbonyl, N,N-Di-(1-methylpropyl)aminocarbonyl, N,N-Di-(2-methylpropyl)aminocarbonyl, N,N-Di-(1,1-dimethylethyl)aminocarbonyl, N-Ethyl-N-methylaminocarbonyl, N-Methyl-N-propylaminocarbonyl, N-Methyl-N-(1-methylethyl)aminocarbonyl, N-Butyl-N-methylaminocarbonyl, N-Methyl-N-(1-methyl-propyl)aminocarbonyl, N-Methyl-N-(2-methylpropyl)aminocarbonyl, N-(1,1-Dimethylethyl)-N-methylaminocarbonyl, N-Ethyl-N-propylaminocarbonyl, N-Ethyl-N-(1-methylethyl)aminocarbonyl, N-Butyl-N-ethylaminocarbonyl, N-Ethyl-N-(1-methyl-propyl)aminocarbonyl, N-Ethyl-N-(2-methylpropyl)aminocarbonyl, N-Ethyl-N-(1,1-dimethylethyl)aminocarbonyl, N-(1-Methylethyl)-N-propylaminocarbonyl, N-Butyl-N-propylaminocarbonyl, N-(1-Methylpropyl)-N-propylaminocarbonyl, N-(2-Methyl-propyl)-N-propylaminocarbonyl, N-(1,1-Dimethylethyl)-N-propylaminocarbonyl, N-Butyl-N-(1-methylethyl)aminocarbonyl, N-(1-Methylethyl)-N-(1-methylpropyl)-aminocarbonyl, N-(1-Methylethyl)-N-(2-methylpropyl)aminocarbonyl, N-(1,1-Dimethylethyl)-N-(1-methylethyl)aminocarbonyl, N-Butyl-N-(1-methylpropyl)-aminocarbonyl, N-Butyl-N-(2-methylpropyl)aminocarbonyl, N-Butyl-N-(1,1-dimethylethyl)aminocarbonyl, N-(1-Methylpropyl)-N-(2-methylpropyl)aminocarbonyl, N-(1,1-Dimethylethyl)-N-(1-methylpropyl)aminocarbonyl oder N-(1,1-Dimethylethyl)-N-(2-methylpropyl)aminocarbonyl;

Alle Phenylringe sind vorzugsweise unsubstituiert oder tragen ein bis drei Halogenatome und/oder eine Nitrogruppe, einen Cyanorest und/oder einen oder zwei Methyl-, Trifluormethyl-, Methoxy- oder Trifluormethoxysubstituenten.

In einer besonders bevorzugten Ausführungsform des erfindungsgemäßen Verfahrens weisen die Variablen R¹, R² und R³ jeweils für sich allein oder in Kombination die folgenden Bedeutungen auf:
R¹ Wasserstoff, C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl;
   sehr bevorzugt Wasserstoff oder C₁-C₄-Alkyl,
   besonders bevorzugt Wasserstoff, Methyl oder Ethyl,
   insbesondere bevorzugt Methyl;
   ebenso bevorzugt Wasserstoff, Amino oder C₁-C₄-Alkyl,
   besonders bevorzugt Wasserstoff, Amino, Methyl oder Ethyl,
   insbesondere bevorzugt Amino oder Methyl;
   ebenso bevorzugt Wasserstoff, Amino oder C₁-C₄-Alkyl,
   besonders bevorzugt Wasserstoff oder Amino,
   insbesondere bevorzugt Wasserstoff;
R² Wasserstoff, C₁-C₄-Alkyl oder C₁-C₄-Halogenalkyl,
   besonders bevorzugt Wasserstoff, Methyl, Difluormethyl oder Trifluormethyl, insbesondere bevorzugt Trifluormethyl;
R³ Wasserstoff oder C₁-C₄-Alkyl,
   besonders bevorzugt Wasserstoff.

In einer weiteren bevorzugten Ausführungsform des erfindungsgemäßen Verfahrens stehen X¹, X² und X³ jeweils für Sauerstoff.

Die angegebene Gruppe Ar steht bevorzugt für eine Gruppe der allgemeinen Formel Ar-1 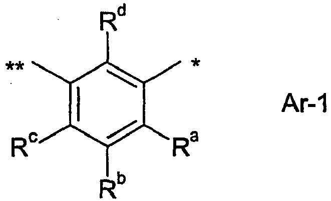 wobei
* die Verknüpfung von Ar mit der C(X³)-Gruppe,
   wobei X bevorzugt für Sauerstoff steht;
** die Verknüpfung von Ar mit dem direkt benachbarten Stickstoffatom kennzeichnet; und
R^{a}, R^{b}, R^{c} und R^{d} unabhängig voneinander Wasserstoff, Halogen, Cyano oder C₁-C₄-Halogenalkyl
bedeuten.

In einer besonders bevorzugten Ausführungsform des erfindungsgemäßen Verfahrens weisen die Variablen R^{a}, R^{b}, R^{c} und R^{d} jeweils für sich allein oder in Kombination die folgenden Bedeutungen auf:
- R^{a}: Wasserstoff, Halogen oder Cyano,
insbesondere bevorzugt Wasserstoff, Fluor, Chlor oder Cyano, sehr bevorzugt Wasserstoff, Chlor oder Cyano, außerordentlich bevorzugt Wasserstoff oder Chlor;
- R^{b}: Wasserstoff;
- R^{c}: Wasserstoff oder Halogen,
insbesondere bevorzugt Wasserstoff, Fluor oder Chlor, sehr bevorzugt Wasserstoff oder Fluor, außerordentlich bevorzugt Fluor;
- R^{d}: Wasserstoff.

Der angegebene, von einem primären oder sekundären Amin abgeleitete Rest A steht in der Regel für eine Gruppe der Formel -NR⁴R⁵, worin die Variablen R⁴ und R⁵ unabhängig voneinander die folgenden Bedeutungen aufweisen:
- R⁴, R⁵: Wasserstoff, C₁-C₆-Alkyl, C₂-C₆-Alkenyl oder C₂-C₆-Alkinyl,
wobei die drei letztgenannten Reste durch einen Rest ausgewählt aus der Gruppe CN, NO₂, C₁-C₄-Alkoxy, C₁-C₄-Alkylthio, Formyl, C₁-C₄-Alkylcarbonyl, C₁-C₄-Alkoxycarbonyl, (C₁-C₄-Alkylamino)carbonyl, (C₁-C₄-Dialkylamino)carbonyl, C₁-C₄-Alkylsulfinyl, C₁-C₄-Alkylsulfonyl, C₃-C₈-Cycloalkyl, 3- bis 6-gliedriges Heterocyclyl mit ein bis vier unter Sauerstoff, Schwefel, Stickstoff und einer Gruppe NR⁶ ausgewählten Heteroatomen,
wobei R⁶ für Wasserstoff, C₁-C₆-Alkyl, C₃-C₆-Alkenyl oder C₃-C₆-Alkinyl steht; oder
Phenyl, das seinerseits partiell oder vollständig halogeniert sein kann und/oder ein bis drei Substituenten ausgewählt aus der Gruppe Cyano, Nitro, C₁-C₄-Alkyl, C₁-C₄-Fluoralkyl, C₁-C₄-Alkoxy, (C₁-C₄-Alkyl)amino, (C₁-C₄-Dialkyl)amino, Trifluormethylsulfonyl, Formyl oder C₁-C₄-Alkyloxycarbonyl, tragen kann;
substituiert sein können;
C₁-C₆-Halogenalkyl, C₂-C₆-Halogenalkenyl, C₃-C₆-Halogenalkinyl;
C₃-C₈-Cycloalkyl, C₃-C₈-Cycloalkenyl, 3- bis 6-gliedriges Heterocyclyl mit ein bis vier unter Sauerstoff, Schwefel, Stickstoff und einer Gruppe NR⁶ ausgewählten Heteroatomen,
wobei R⁶ für Wasserstoff, C₁-C₆-Alkyl, C₃-C₆-Alkenyl oder C₃-C₆-Alkinyl steht;
Phenyl oder Naphthyl;
wobei die fünf letztgenannten Reste C₃-C₈-Cycloalkyl, C₃-C₈-Cycloalkenyl, 3- bis 6-gliedriges Heterocyclyl, Phenyl und Naphthyl ihrerseits partiell oder vollständig halogeniert sein können und/oder ein bis drei Substituenten ausgewählt aus der Gruppe Cyano, Nitro, C₁-C₄-Alkyl, C₁-C₄-Fluoralkyl, C₁-C₄-Alkoxy, (C₁-C₄-Alkyl)amino, (C₁-C₄-Dialkyl)amino, Trifluormethylsulfonyl, Formyl, C₁-C₄-Alkyloxycarbonyl oder Phenoxy tragen können; oder
- R⁴ und R⁵: bilden gemeinsam einen gesättigten oder teilweise ungesättigten 5- bis 6-gliedrigen Stickstoffheterocyclus, der ein oder zwei Carbonylgruppen, Thiocarbonylgruppen und/oder ein oder zwei weitere Heteroatome, ausgewählt unter O, S, N und einer Gruppe NR⁶ als Ringglieder aufweisen kann,
wobei R⁶ für Wasserstoff, C₁-C₆-Alkyl, C₃-C₆-Alkenyl oder C₃-C₆-Alkinyl steht,
und welcher seinerseits durch C₁-C₄-Alkyl, C₁-C₄-Alkoxy und/oder C₁-C₄-Halogenalkyl substituiert sein kann.

Für das erfindungsgemäße Verfahren hat es sich als besonders vorteilhaft erwiesen, wenn A für eine Gruppe der Formel -NR⁴R⁵ steht, worin die Substituenten R⁴ und R⁵ unabhängig voneinander
Wasserstoff oder C₁-C₆-Alkyl, das seinerseits durch einen Substituenten ausgewählt aus der Gruppe
Halogen, Cyano, C₃-C₈-Cycloalkyl, C₁-C₄-Alkoxy, C₁-C₄-Alkylthio, C₁-C₄-Alkoxycarbonyl, Phenyl, das seinerseits ein bis drei Reste aus der Gruppe Halogen oder C₁-C₄-Alkoxy tragen kann, Furyl, Thienyl und 1,3-Dioxolanyl;
bevorzugt Halogen, Cyano und C₁-C₄-Akoxy;
sehr bevorzugt Halogen;
substituiert sein kann,
bedeuten.

In einer bevorzugten Ausführungsform der Erfindung steht A für eine Gruppe der Formel -NR⁴R⁵, worin die Substituenten R⁴ und R⁵ unabhängig voneinander Wasserstoff oder C₁-C₆-Alkyl bedeuten.

In einer besonders bevorzugten Ausführungsform der Erfindung steht A für eine Gruppe der Formel -NR⁴R⁵, worin
- R⁴: Wasserstoff, C₁-C₆-Alkyl, C₁-C₆-Halogenalkyl, C₂-C₆-Alkenyl oder C₂-C₆-Alkinyl, insbesondere bevorzugt Wasserstoff oder C₁-C₆-Alkyl, sehr bevorzugt C₁-C₆-Alkyl,
besonders bevorzugt Methyl; und
- R⁵: C₁-C₆-Alkyl, C₁-C₄-Halogenalkyl, C₃-C₈-Cycloalkyl oder Phenyl, insbesondere bevorzugt C₁-C₆-Alkyl,
sehr bevorzugt C₁-C₄-Alkyl;
bedeuten.

Die erfindungsgemäßen Verfahrensschritte können sowohl diskontinuierlich als auch kontinuierlich in hierfür geeigneten Reaktionsgefäßen betrieben werden.

Bei diskontinuierlicher Vorgehensweise wird man üblicherweise Rührkessel und Rührreaktoren einsetzen. Diese sind in der Regel zur Abfuhr der Reaktionswärme mit geeigneten Wärmetauschern oder einer Mantelkühlung ausgestattet Die kontinuierliche Durchführung der erfindungsgemäßen Reaktionsschritte erfolgt ebenfalls in den hierfür üblichen Reaktoren, beispielsweise in Rührkesseln, Rührkesselkaskaden und Rohrreaktoren, wobei Reaktoren mit geringer Rückvermischung bevorzugt sind.

Die Menge an Lösungs- bzw. Verdünnungsmittel wird in der Regel so gewählt, dass die Reaktionsmischungen während der Umsetzung fließfähig bleiben.

Die 3-Phenyl(thio)uracile und -dithiouracile der Formel I werden durch die Umsetzung eines Carbamats der Formel II mit einem Enamin der Formel III hergestellt:

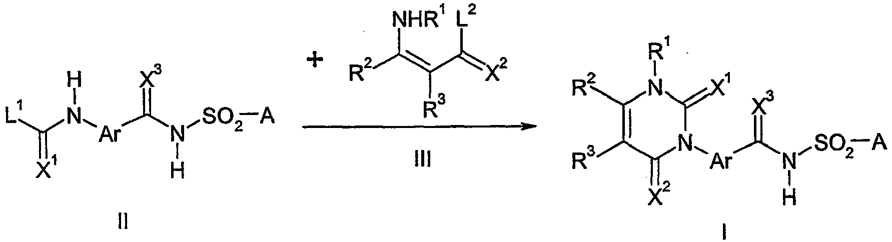

Die Variablen X¹, X², X³, R¹, R², R³ sowie Ar und A haben die zuvor genannten Bedeutungen, insbesondere bevorzugt die in der Beschreibung als bevorzugt genannten Bedeutungen.
- L¹: steht für eine nucleophil verdrängbare Abgangsgruppe,
bevorzugt für C₁-C₆-Alkoxy oder C₁-C₆-Alkylthio,
besonders bevorzugt für C₁-C₆-Alkoxy.
- L²: steht für eine nucleophil verdrängbare Abgangsgruppe;
bevorzugt für C₁-C₆-Alkoxy, C₁-C₆-Halogenalkoxy, C₁-C₄-Alkoxy-C₂-C₄-alkoxy, C₁-C₄-Alkylthio-C₂C₄-alkoxy, C₂-C₆-Alkenyloxy, C₂-C₆-Halogenalkenyloxy, C₃-C₆-Alkinyloxy, C₃-C₈-Halogenalkinyloxy, C₃-C₈-Cycloalkyloxy, C₁-C₆-Cyanoalkoxy oder Benzyloxy,
das seinerseits am Phenylring partiell oder vollständig halogeniert sein kann und/oder durch ein bis drei Reste aus der Gruppe Cyano, Nitro, C₁-C₄-Alkyl, C₁-C₄-Alkoxy und C₁-C₄-Alkylthio substituiert sein kann;
vorzugsweise für C₁-C₆-Alkoxy, C₁-C₆-Halogenalkoxy, C₁-C₄-Alkoxy-C₂-C₄-alkoxy, C₂-C₆-Alkenyloxy, C₂-C₆-Halogenalkenyloxy, C₃-C₆-Alkinyloxy oder C₃-C₆-Halogenalkinyloxy;
sehr bevorzugt für C₁-C₆-Alkoxy, C₁-C₄-Alkoxy-C₂-C₄-alkoxy, C₂-C₆-Alkenyloxy oder C₃-C₆-Alkinyloxy;
außerordentlich bevorzugt für C₁-C₆-Alkoxy.

Diese Umsetzung der Carbamate der Formel II mit Enaminen der Formel III erfolgt üblicherweise bei Temperaturen oberhalb Raumtemperatur, z.B. von 25°C bis 200°C, vorzugsweise 90 °C bis 190°C, besonders bevorzugt 100 °C bis 140°C in einem inerten organischen Lösungsmittel in Gegenwart einer Base (vgl. z.B. WO 99/31091).

Der Reaktionsdruck ist für den Erfolg des erfindungsgemäßen Verfahrens von untergeordneter Bedeutung und kann beispielsweise im Bereich von 500 mbar bis 10 bar liegen. Vorzugsweise wird die Reaktion im Bereich des Normaldrucks, d.h. im Bereich von 0,9 bis 1,2 bar durchgeführt.

Die für die Umsetzung erforderliche Reaktionszeit liegt in der Regel im Bereich von 1 h bis 24h, und insbesondere im Bereich von 2h bis 8h.

Die Reaktion kann grundsätzlich in Substanz durchgeführt werden. Vorzugsweise setzt man jedoch die Carbamate der Formel II mit den Enaminen der Formel III in einem organischen Lösungsmittel um. Grundsätzlich geeignet sind alle Lösungsmittel, welche die Carbamate der Formel II und die Enamine der Formel III zumindest teilweise und vorzugsweise vollständig unter Reaktionsbedingungen zu lösen vermögen. Bevorzugte Lösungsmittel sind polar protische Lösungsmittel.

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Diethylenglycoldimethylether, Anisol und Tetrahydrofuran, Nitrile wie Acetonitril und Propionitril, Alkohole wie Methanol, Ethanol, n-Propanol, Isopropanol, n-Butanol und tert.-Butanol, Carbonsäureester wie Butylacetat, sowie Dimethylsulfoxid, Dimethylformamid, Dimethylacetamid und N-Methylpyrrolidon; besonders bevorzugt Dimethylformamid, Dimethylacetamid und N-Methylpyrrolidon.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetallamide wie Lithiumamid, Natriumamid und Kaliumamid, Alkalimetall- und Erdalkalimetallcarbonate wie Lithiumcarbonat, Natriumcarbonat, Kaliumcarbonat, Calciumcarbonat und Caesiumcarbonat, sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkalimetall- und Erdalkalimetallalkoholate wie Lithiummethanolat, Natriummethanolat, Natriumethanolat, Kaliumethanolat, Kalium- tert.-Butanolat, Kalium-tert.-Pentanolat und Dimethoxymagnesium, außerdem organische Basen, z.B. tertiäre Amine wie Trimethylamin, Triethylamin, Diisopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Alkalimetall- und Erdalkalimetallhydroxide, Alkalimetall- und Erdalkalimetallcarbonate sowie Alkalimetall- und Erdalkalimetallalkoholate.

Die Basen werden im allgemeinen im Überschuß, besonders bevorzugt mit 1,1 bis 3 Äquivalenten bzgl. des Carbamats der Formel II eingesetzt, sie können aber auch als Lösungsmittel verwendet werden. Es kann von Vorteil sein, die Base über einen Zeitraum versetzt zuzugeben.

Bevorzugt werden die Basen mit 1,1 bis 2,4 Äquivalenten, sehr bevorzugt mit 2,2 bis 2,4 Äquivalenten, besonders bevorzugt mit 2,3 Äquivalenten bzgl. des Carbamats II eingesetzt.

Die Edukte werden im allgemeinen in äquimolaren Mengen miteinander umgesetzt. Es kann vorteilhaft sein, die eine Komponente in einem Überschuß bezogen auf die andere Komponente einzusetzen. Vorzugsweise wird man die Verbindungen in einem Molverhältnis II : III im Bereich von 1,5 : 1 bis 1 : 1,5, besonders bevorzugt 1 : 1 bis 1 : 1,2, insbesondere bevorzugt 1: 1 einsetzen.

Vorzugsweise wird man die bei der Umsetzung der Carbamate der Formel II mit den Enaminen der Formel III gebildeten Verbindungen L¹-H und L²-H während der Umsetzung teilweise aus der Reaktionsmischung entfernen, insbesondere wenn es sich bei den Verbindungen L¹-H und L²⁻H um ein C₁-C₄-Alkanol wie Methanol oder Ethanol handelt. Hierzu wird man in an sich bekannter Weise die Reaktion bei einer Temperatur und einem Druck durchführen, bei der die Verbindungen L¹-H und L²-H gegebenenfalls als Azeotrop mit dem Lösungsmittel aus der Reaktionsmischung abdestilliert. Gegebenenfalls wird man zum Ausgleich frisches Lösungsmittel in die Reaktion einbringen oder das mit den Verbindungen L¹-H und L²-H abdestillierte Lösungsmittel nach ggf. destillativer Abreicherung der Verbindungen L¹-H und L²-H in die Reaktion zurückführen.

Aus diesen Gründen ist es von Vorteil, wenn das eingesetzte Lösungsmittel einen Siedepunkt von wenigstens 10 °C, insbesondere wenigstens 30 °C, oberhalb des Siedepunktes der bei der Reaktion gebildeten Verbindungen L¹-H und L²-H aufweist (jeweils bei Normaldruck).

Zweckmäßigerweise führt man die Umsetzung der Carbamate der Formel II mit den Enaminen der Formel III in einer Apparatur durch, die mit wenigstens einer Destillations- oder Rektifikationsvorrichtung, z.B. einer Destillationskolonne, versehen ist, welche zum einen ein Abdestillieren der Verbindungen L¹-H und L²-H, ggf. zusammen mit Lösungsmittel erlaubt und gleichzeitig eine Abtrennung und Rückführung des gegebenenfalls mit den Verbindungen L¹-H und L²-H abdestillierten Lösungsmittels ermöglicht.

Zur Umsetzung können die Verbindungen II und III in an sich beliebiger Weise miteinander in Kontakt gebracht werden, d.h., die Reaktanden und die Base können getrennt, gleichzeitig oder nacheinander in das Reaktionsgefäß eingebracht und zur Reaktion geführt werden. Beispielsweise kann man die Verbindungen II und III in einem Reaktionsgefäß, gegebenenfalls mit dem gewünschten Lösungsmittel, vorlegen und dann die gewünschten Reaktionsbedingungen einstellen. Man kann jedoch auch die Hauptmenge oder Gesamtmenge an Verbindungen II und III, gegebenenfalls in einem Lösungsmittel unter Reaktionsbedingungen in das Reaktionsgefäß eintragen.

In einer bevorzugten Ausführungsform der Erfindung legt man die Hauptmenge, insbesondere wenigstens 80 % und besonders bevorzugt die Gesamtmenge oder nahezu die Gesamtmenge (> 95 %) der Carbamate der Formel II vor, und gibt hierzu im Verlauf der Reaktion, z.B. über einen Zeitraum von 0,5 bis 20 h und insbesondere 1 bis 10 h, die Hauptmenge, insbesondere wenigstens 80 % und besonders bevorzugt die Gesamtmenge oder nahezu die Gesamtmenge (> 95 %) des Enamins der Formel III unter Reaktionsbedingungen zu. Hierzu wird man die Enamine der Formel III vorzugsweise in einem Lösungsmittel lösen.

In einer weiteren bevorzugten Ausführungsform der Erfindung werden die Verbindungen II und III vorgelegt und dann hierzu die Hauptmenge, insbesondere wenigstens 80 % und besonders bevorzugt die Gesamtmenge oder nahezu die Gesamtmenge (> 95 %) der Base zugegeben. Die Reaktion kann gegebenenfalls durch Nachdosieren von Base vervollständigt werden.

Die Isolierung der 3-Phenyl(thio)uracile und -dithiouracile der Formel I aus der Reaktionsmischung kann in an sich bekannter Weise erfolgen.

Sofern die Umsetzung in einem Lösungsmittel durchgeführt wurde, wird man in der Regel die Reaktionsmischung einengen und/oder abkühlen und/oder ein Fällungsmittel zugeben. Geeignete Fällungsmittel sind Lösungsmittel, in welchen sich die 3-Phenyl-(thio)uracile und -dithiouracile der Formel I zumindest bei Temperaturen unterhalb 25°C nicht oder nur in geringem Ausmaß löst. Hierzu zählen insbesondere aliphatische und cycloaliphatische Kohlenwasserstoff wie Pentan, Hexan, Cyclohexan, Heptan, Petrolether, Toluol und dergleichen. Der Fällung/Kristallisation können sich weitere Reinigungsmaßnahmen anschließen. Wird die Umsetzung wie bevorzugt in einem Alkohol, insbesondere in Methanol oder Ethanol, oder in einem Alkylbenzol durchgeführt, so ist in der Regel die Zugabe eines Fällungsmittels nicht erforderlich.

Zur Aufarbeitung ist es weiterhin vorteilhaft, den pH-Wert der Reaktionsmischung durch Säure, bevorzugt durch anorganische Säuren wie z.B. Salzsäure oder Schwefelsäure, auf pH < 7 einzustellen. Insbesondere ist es vorteilhaft, wenn am Ende der pH-Wert der Reaktionsmischung < 2 ist.

Die für die Herstellung der 3-Phenyl(thio)uracile und -dithiouracile der Formel I benötigten Enamine der Formel III sind in der Literatur bekannt (z. B. A. Lutz, A. und S. Trotto, J. of Heterocyclic Chem. 1972, 9, 3, 513-522) und können gemäß der zitierten Literatur hergestellt werden.

Im Besonderen können auf diesem Weg 3-Phenyluracile der Formel I.A.1, 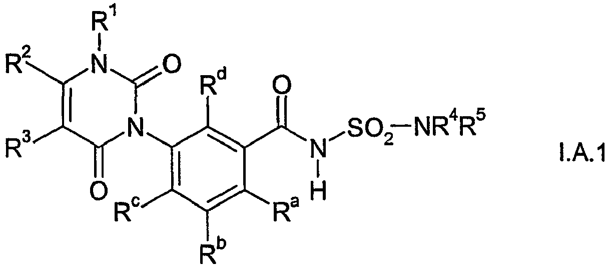 wobei R^{b} und R^{d} Wasserstoff bedeuten,
hergestellt werden, indem entsprechende Carbamate der Formel II.A.1, 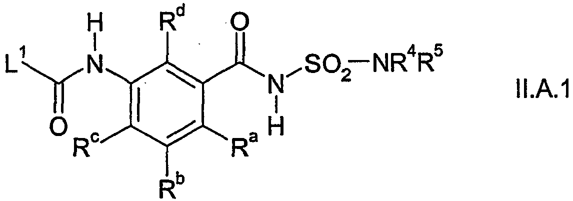 wobei R^{b} und R^{d} Wasserstoff bedeuten,
mit Enaminen der Formel III 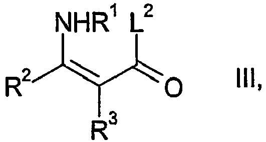 wobei X² für Sauerstoff steht,
umgesetzt werden: 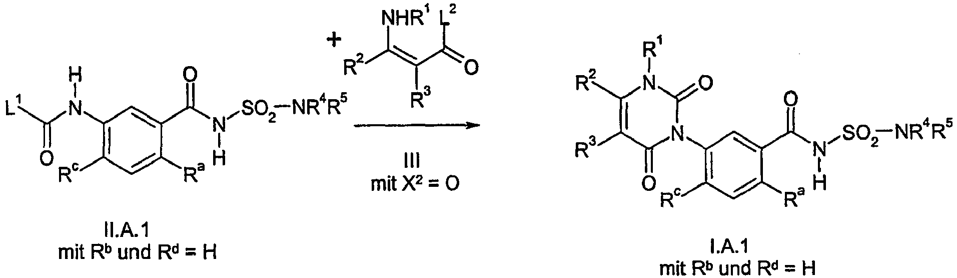
- L¹: steht für eine nucleophil verdrängbare Abgangsgruppe,
bevorzugt C₁-C₆-Alkoxy oder C₁-C₆-Alkylthio,
besonders bevorzugt C₁-C₆-Alkoxy,
insbesondere bevorzugt C₁-C₄-Alkoxy,
sehr bevorzugt Methoxy oder Ethoxy;
- L²: steht für eine nucleophil verdrängbare Abgangsgruppe;
bevorzugt für C₁-C₆-Alkoxy, C₁-C₆-Halogenalkoxy, C₁-C₄-Alkoxy-C₂-C₄-alkoxy, C₁-C₄-Alkylthio-C₂C₄-alkoxy, C₂-C₆-Alkenyloxy, C₂-C₆-Halogenalkenyloxy, C₃-C₆-Alkinyloxy, C₃-C₆-Halogenalkinyloxy, C₃-C₈-Cycloalkyloxy, C₁-C₆-Cyanoalkoxy oder Benzyloxy,
das seinerseits am Phenylring partiell oder vollständig halogeniert sein kann und/oder durch ein bis drei Reste aus der Gruppe Cyano, Nitro, C₁-C₄-Alkyl, C₁-C₄-Alkoxy und C₁-C₄-Alkylthio substituiert sein kann;
vorzugsweise für C₁-C₆-Alkoxy, C₁-C₆-Halogenalkoxy, C₁-C₄-Alkoxy-C₂-C₄-alkoxy, C₂-C₆-Alkenyloxy, C₂-C₆-Halogenalkenyloxy, C₃-C₆-Alkinyloxy oder C₃-C₆-Halogenalkinyloxy;
sehr bevorzugt für C₁-C₆-Alkoxy, C₁-C₄-Alkoxy-C₂-C₄-alkoxy, C₂-C₆-Alkenyloxy oder C₃-C₆-Alkinyloxy;
außerordentlich bevorzugt für C₁-C₆-Alkoxy.

Dabei ist die Herstellung insbesondere solcher 3-Phenyluracile der Formel I.A.1 mit R^{b} und R^{d} = Wasserstoff bevorzugt, in denen die Variablen R¹, R², R³, R^{a}, R^{c} sowie R⁴ und R⁵ jeweils für sich alleine als auch in Kombination miteinander folgende Bedeutungen haben:
- R¹: Wasserstoff, C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl;
sehr bevorzugt Wasserstoff oder C₁-C₄-Alkyl,
besonders bevorzugt Wasserstoff, Methyl oder Ethyl,
insbesondere bevorzugt Methyl;
ebenso bevorzugt Wasserstoff, Amino oder C₁-C₄-Alkyl,
besonders bevorzugt Wasserstoff, Amino, Methyl oder Ethyl,
insbesondere bevorzugt Amino oder Methyl;
ebenso bevorzugt Wasserstoff, Amino oder C₁-C₄-Alkyl,
besonders bevorzugt Wasserstoff oder Amin,
insbesondere bevorzugt Wasserstoff;
- R²: Wasserstoff, C₁-C₄-Alkyl oder C₁-C₄-Halogenalkyl,
besonders bevorzugt Wasserstoff, Methyl, Difluormethyl oder Trifluormethyl, insbesondere bevorzugt Trifluormethyl;
- R³: Wasserstoff oder C₁-C₄-Alkyl,
besonders bevorzugt Wasserstoff.
- R^{a}: Wasserstoff, Halogen oder Cyano,
insbesondere bevorzugt Wasserstoff, Fluor, Chlor oder Cyano,
sehr bevorzugt Wasserstoff, Chlor oder Cyano,
außerordentlich bevorzugt Wasserstoff oder Chlor,
sehr außerordentlich bevorzugt Wasserstoff;
- R^{c}: Wasserstoff oder Halogen,
insbesondere bevorzugt Wasserstoff, Fluor oder Chlor,
sehr bevorzugt Wasserstoff oder Fluor,
außerordentlich bevorzugt Fluor;
- R⁴ und R⁵: unabhängig voneinander
Wasserstoff oder C₁-C₆-Alkyl, das seinerseits durch einen Substituenten ausgewählt aus der Gruppe
Halogen, Cyano und C₁-C₄-Alkoxy, bevorzugt Halogen,
substituiert sein kann;
insbesondere bevorzugt
- R⁴: Wasserstoff, C₁-C₆-Alkyl, C₂-C₆-Alkenyl oder C₂-C₆-Alkinyl,
sehr bevorzugt Wasserstoff oder C₁-C₆-Alkyl,
besonders bevorzugt C₁-C₄-Alkyl,
außerordentlich bevorzugt Methyl; und
- R⁵: C₁-C₆-Alkyl, C₃-C₈-Cycloalkyl oder Phenyl,
sehr bevorzugt C₁-C₆-Alkyl,
besonders bevorzugt C₁-C₄-Alkyl.

Insbesondere können 3-Phenyl(thio)uracile und -dithiouracile der Formel I, worin
- R¹: für C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Halogenalkinyl, Phenyl-C₁-C₄-alkyl oder Amino;
steht, hergestellt werden, indem Carbamate der Formel II 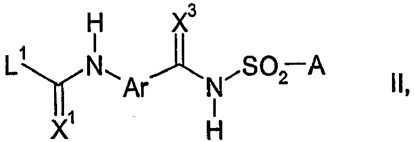 wobei die Variablen X¹, X³, Ar und A die zuvor genannten Bedeutungen haben und L¹ für eine nucleophil verdrängbare Abgangsgruppe steht,
mit Enaminen der Formel III 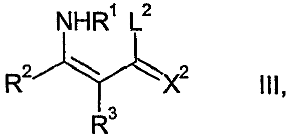 wobei R¹ für Wasserstoff steht, die Variablen X², R² und R³ die zuvor genannten Bedeutungen haben und L² für eine nucleophil verdrängbare Abgangsgruppe steht, umgesetzt werden; und
das entstandene 3-Phenyl(thio)uracil und -dithiouracil der Formel I, wobei R¹ für Wasserstoff steht, anschließend
- mit einem Alkylierungsmittel der Formel VI

   R¹-L⁴ VI,

   worin R¹ C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl; und
   L⁴ eine nucleophil verdrängbare Abgangsgruppe; bedeutet, zu 3-Phenyl(thio)uracilen und -dithiouracilen der Formel I
   worin R¹ für C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl steht,
   alkyliert;
   oder
- mit einem Aminierungsmittel der Formel VII

   H₂N-L⁵ VII,
worin L⁵ für eine nucleophil verdrängbare Abgangsgruppe steht,
zu 3-Phenyl(thio)uracilen und -dithiouracilen der Formel I, worin R¹ für NH₂ steht, aminiert.

Für die Herstellung der 3-Phenal(thio)uracile und -dithioracile der Formel I, worin R¹ für Wasserstoff steht, gelten die voranstehend genannten, insbesondere die voranstehend als bevorzugt genannten Reditionsbedingungen.
- L⁴: im Alkylierungsmittel der Formel VI steht für eine nucleophil verdrängbare Abgangsgruppe,
bevorzugt für Halogen, Hydrogensulfat, C₁-C₆-Alkylsulfat, Sulfat, C₁-C₆- Alkylsulfonyloxy, C₁-C₆-Halogenalkylsulfonyloxy oder Phenylsulfonyloxy,
wobei der Phenylring gegebenenfalls mit Halogen, Nitro, C₁-C₆-Alkyl oder C₁-C₆-Halogenalkyl ein- oder mehrfach substituiert ist,
besonders bevorzugt für Halogen, Hydrogensulfat, C₁-C₆-Alkylsulfonyloxy, C₁-C₆-Halogenalkylsulfonyloxy, Phenylsulfonyloxy, p-Toluolsulfonyloxy, p-Chlorphenylsulfonyloxy, p-Bromphenylsulfonyloxy, oder p-Nitrophenylsulfonyloxy, insbesondere bevorzugt Chlor, Methylsulfonyloxy, Trifluormethylsulfonyloxy oder Phenylsulfonyloxy.
- L⁵: im Aminierungsmittel der Formel VII steht für eine nucleophil verdrängbare Abgangsgruppe,
bevorzugt für Halogen, Hydrogensulfat, C₁-C₆-Alkylsulfonyloxy, C₁-C₆-Halogenalkylsulfonyloxy, Phenylsulfonyloxy oder Phenyloxy,
wobei der Phenylring gegebenenfalls mit Halogen, Nitro, C₁-C₆-Alkyl oder C₁-C₆-Halogenalkyl ein- oder mehrfach substituiert ist,
besonders bevorzugt für Halogen, Hydrogensulfat, C₁-C₆-Alkylsulfonyloxy, C₁-C₆-Halogenalkylsulfonyloxy, Phenylsulfonyloxy, p-Toluolsulfonyloxy, p-Chlorphenylsulfonyloxy, p-Bromphenylsulfonyloxy, oder p-Nitrophenylsulfonyloxy, insbesondere bevorzugt Chlor, Methylsulfonyloxy, Trifluormethylsulfonyloxy oder Phenylsulfonyloxy.

Das Verfahren zur Alkylierung oder Aminierung der Verbindung I mit R¹ = Wasserstoff ist insofern überraschend, da man die Bildung entsprechender N-Alkylsulfonamide oder Gemische aus N-Alkylsulfonamiden oder N-Alkyl substituierten (Thio)Uracilen bzw. Dithiouracilen erwartet hätte. Es ist bekannt, dass Schwefelsäurediamide in einfacher Weise mit Schwefelsäurediestern oder Arensulfonsäureestern in Gegenwart einer Base alkyliert werden, siehe beispielsweise R. Sowada, J. Prakt. Chem. 25, 88 (1964). Im Falle von trisubstituierten Schwefelsäurediamiden ist die Bildung von tetrasubstituierten Schwefelsäurediamiden bekannt, siehe B. Unterhalt, E. Seebach, Arch. Pharm. 314, 51 (1981). Ebenso lassen sich Schwefelsäurediamide, bei denen die Amidfunktion bereits einen Acylrest trägt, alkylieren, siehe K. C. C. Bancroft et al., J. Heterocycl. Chem. 15, 1521 (1978); A. Martinez et al., Bioorg. Med. Chem. Lett. 9 (21), 3133 (1999). Der Fachmann hätte daher - aufgrund der leichten Alkylierbarkeit der Sulfamindseitenkette - die bevorzugte Alkyierung am Sulfonamidstickktoffatom oder zumindest die Bildung dialkylierter Produkte erwartet.

Die N-Alkylierung der Verbindung I am freien (Thio)Uracilstickstoffatom gelingt in für Uracile an sich bekannter Weise durch Umsetzung der Verbindung I mit R¹ = Wasser-stoff mit einem Alkylierungsmittel R¹-L⁴ (VI), wie sie beispielsweise in der US 4,943,309 beschrieben sind, auf deren Offenbarung zur Alkylierung hiermit im vollen Umfang Bezug genommen.

Bevorzugte Alkylierungsmittel sind C₁-C₄-Alkylhalogenide, Di-C₁-C₄-alkylsulfate, Phenylsulfonsäure-C₁-C₄-alkylester, wobei der Phenylrest gegebenenfalls mit Halogen, Nitro oder C₁-C₆-Alkyl ein- oder zweifach substituiert ist. Besonders bevorzugte Alkylierungsmittel sind Methylierungsmittel oder Ethylierungsmittel wie Dimethylsulfat, Diethylsulfat, Methyliodid, Ethyliodid, Methylbromid, Methylchlorid, Ethylbromid, Ethylchlorid, C₁-C₆-Alkylsulfonsäuremethylester oder -ethylester oder die Methyl- oder Ethylester der zuvor genannten Phenylsulfonsäuren. Ein ganz besonders bevorzugtes Methylierungsmittel ist Dimethylsulfat.

Im erfindungsgemäßen Verfahren kann man das Alkylierungsmittel sowohl in äquimolarer Menge, bezogen auf die Verbindung I, als auch in substöchiometrischer Menge oder überstöchiometrischer Menge einsetzen. Üblicherweise setzt man wenigstens eine äquimolare Menge an Alkylierungsmittel VI, bezogen auf die Verbindung I ein. Die molaren Verhältnisse, in denen die Verbindung I mit R¹ = Wasserstoff zu Alkylierungsmittel VI eingesetzt werden, liegen in der Regel im Bereich von 1:1 bis 1:3, vorzugsweise 1:1 1 bis 1:1,3 für das Verhältnis von Verbindung I zu Alkylierungsmittel VI.

Üblicherweise führt man die Alkylierung in Gegenwart einer Base durch. Als Base kommen grundsätzlich alle Verbindungen in Betracht, die in der Lage sind, das Lactamstickstoffatom zu deprotonieren Geeignete Basen sind z.B. die im Zusammenhang mit der Herstellung der Verbindung I durch Umsetzung von II mit III genannten Basen. Bevorzugt ist die Base ausgewählt unter Alkali- und Erdalkalimetallhydroxiden wie Natriumhydroxid, Kaliumhydroxid und Lithiumhydroxid, Alkali- und Erdalkalimetalloxiden wie Calciumoxid, Alkali- und Erdalkalimetallcarbonaten wie Lithiumcarbonat, Natriumcarbonat, Kaliumcarbonat, Cäsiumcarbonat, Magnesiumcarbonat, Calciumcarbonat, Zinkcarbonat oder Bariumcarbonat. In einer besonders bevorzugten Ausführungsform des erfindungsgemäßen Verfahrens setzt man als Base Natriumhydroxid oder Kaliumcarbonat ein.

Die Base kann in substöchiometrischer, überstöchiometrischer oder äquimolarer Menge, bezogen auf die Verbindung I, eingesetzt werden. Vorzugsweise setzt man wenigstens eine äquimolare Menge an Base, bezogen auf die Verbindung I ein. Die Menge an Base wird in der Regel nicht mehr als 1,3 mol, bezogen auf 1 mol der Verbindung I, betragen.

Die Umsetzung der Verbindungen I mit R¹ = Wasserstoff mit dem Alkylierungsmittel der Formel VI wird vorteilhaft in Gegenwart eines Lösungsmittels durchgeführt. Als Lösungsmittel für diese Umsetzungen verwendet man - je nach Temperaturbereich-aliphatische, cycloaliphatische oder aromatische Kohlenwasserstoffe wie Pentan, Hexan, Cyclopentan, Cyclohexan, Toluol, Xylol, chlorierte aliphatische und aromatische Kohlenwasserstoffe wie Dichlormethan, Trichlormethan, 1,2-Dichlorethan, 1,1,2,2-Tetrachlorethan, Chlorbenzol, 1,2-, 1,3- oder 1,4-Dichlorbenzol, Chlortoluole, Dichlortoluole, offfenkettige Dialkylether wie Diethylether, Di-n-proplyether, Di-n-isopropylether, Methyl-tert-butylether, cyclische Ether wie Tetrahydrofuran, 1,4-Dioxan, Anisol, Glykolether wie Dimethylglykolether, Diethylglykolether, Diethylenglykoldimethylether, Diethylenglykoldiethylether, C₁-C₄-Alkohole wie Methanol, Ethanol, n-Propanol, iso-Propanol, n-Butanol, Ketone wie Aceton, Methylethylketon, Methylisopropylketon, Methylisobutylketon, Butanon, Carbonate wie Diethylcarbonat und Ethylencarbonat, N,N-Dialkylamide wie N,N-Dimethylformamid oder N,N-Dimethylacetamid, N-Alkyllactame wie N-Methylpyrrolidon, Sulfoxide wie Dimethylsulfoxid, Tetraalkylhamstoffe wie Tetramethylharnstoff, Tetraethylhamstoff, Tetrabutylharnstoffe, Dimethylethylenharnstoff, Dimethylpropylenharnstoff oder Gemische dieser Lösungsmittel. Als Lösungsmittel bevorzugt sind N,N-Dimethylformamid, N-Methylpyrrolidon, Aceton, Dichlormethan, Tetrahydrofuran, Toluol oder Gemische dieser Lösungsmittel.

Vorzugsweise führt man die Alkylierung der Verbindung I bei Temperaturen zwischen -5 °C und 100 °C, vorzugsweise bei Temperaturen zwischen 0 °C und 80 °C und insbesondere bei Temperaturen zwischen 20 °C und 50 °C durch. Die Reaktionszeit kann der Fachmann in an sich üblicher Weise durch Routinemethoden wie Dünnschichtchromatographie oder HPLC ermitteln.

Die Zugabe von Verbindung I, Alkylierungsmittel VI und Base kann getrennt, gleichzeitig oder nacheinander erfolgen.

Vorteilhaft kann man das mehrstufige Verfahren zur Herstellung der Verbindung I mit R¹ ≠ Wasserstoff auch als Eintopfreaktion durchführen. Bei der Umsetzung des Carbamats der Formel II mit dem Enamin der Formel III mit R¹ = Wasserstoff in Gegenwart eines Überschusses an Base entsteht zunächst das Uracilsalz, das ohne Isolierung oder Reinigung anschließend mit dem Alkylierungsmittel der Formel VI umgesetzt wird. Danach führt man die Reaktion im angegebenen Temperaturbereich zu Ende.

In einer anderen Variante des erfindungsgemäßen Verfahrens kann man die Umsetzug auch in einem wässrigen Mehrphasensystem durchführen, vorzugsweise in Gegenwart von Phasentransferkatalysatoren wie quartären Ammoniumsalzen oder Phosphoniumsalzen. Geeignete quartäre Ammoniumsalze umfassen Tetraalkyl-(C₁-C₁₈)-ammoniumchloride, -bromide, fluoride oder -tetrafluoroborate wie Tetraethylammoniumchlorid, Tetrabutylammoniumbromid, Tetrabutylammoniumiodid, Tetrabutylammoniumtetrafluoroborat, N-Benzyltrialkyl-(C₁-C₁₈)-ammoniumchloride, -bromide oder -fluoride wie Benzyltriethylammoniumchlorid, vorzugsweise Tetrabutylammoniumbromid oder Tetrabutylammoniumiodid. Geeignete Phosphoniumsalze sind beispielsweise Tetraphenylphosphoniumchlorid oder -bromid, Tetraalkyl-(C₁-C₁₈)-phosphoniumchlorid oder -bromid wie Tetrabutylphosphoniumbromid. In der Regel setzt man den Phasentransferkatalysator in einer Menge von bis zu 20 mol-%, vorzugsweise zwischen 1 und 15 mol-% und insbesondere zwischen 2 und 12 mol.-%, bezogen auf die Verbindung I mit R¹ = Wasserstoff, ein.

Das Mehrphasensystem umfasst eine wässrige Phase und wenigstens eine organische flüssige Phase. Daneben können im Verlauf der Umsetzung auch feste Phasen auftreten. Die wässrige Phase ist vorzugsweise eine Lösung von Alkall- oder Erdalkalihydroxiden oder -carbonaten in Wasser. Bezüglich geeigneter Alkali- oder Erdalkalihydroxide oder -carbonate wird auf das zuvor Gesagte verwiesen. Besonders bevorzugt verwendet man Alkali- oder Erdalkalihydroxide, speziell Natriumhydroxid. Vorzugsweise kommen für die organische Phase aliphatische, cycloaliphatische oder aromatische, gegebenenfalls halogenierte Kohlenwasserstoffe, cyclische oder offenkettige Ether oder Gemische davon in Betracht, wobei bezüglich der aliphatischen, cycloaliphatischen oder aromatischen, gegebenenfalls halogenierten Kohlenwasserstoffe, cyclischen oder offenkettigen Ether auf das zuvor Gesagte Bezug genommen wird. In einer bevorzugten Ausführungsform des erfindungsgemäßen Verfahrens besteht das Mehrphasensystem aus wässriger Natriumhydroxid-Lösung als wässrige Phase und aus Toluol und Tetrahydrofuran oder Dichlormethan und Tetrahydrofuran als organische Phase.

Bei Verwendung eines Mehrphasensystems kann man beispielsweise die Verbindung I in einem der vorgenannten organischen Lösungsmittel oder Lösungsmittelgemische vorlegen. Danach gibt man die wässrige Lösung der Base, das Alkylierungsmittel VI und den Phasentransferkatalysator unter Durchmischen zu und bringt dann im genannten Temperaturbereich die Umsetzung zu Ende.

Die Umsetzung kann bei Normaldruck, vermindertem Druck oder unter erhöhtem Druck, gegebenenfalls unter Inertgas kontinuierlich oder diskontinuierlich durchgeführt werden.

Im Besonderen können auf diesem Weg 3-Phenyluracile der Formel I.A.1, 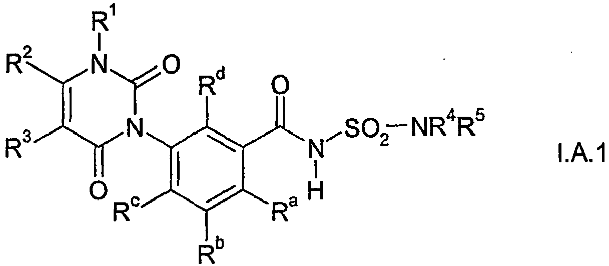 wobei
- R¹: C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl; und
- R^{b} und R^{d}: Wasserstoff bedeuten,
hergestellt werden, indem entsprechende Carbamate der Formel II.A.1, 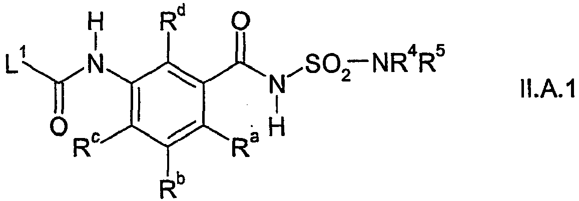 wobei R^{b} und R^{d} Wasserstoff bedeuten,
mit Enaminen der Formel III 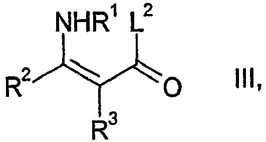 wobei
- R¹: für Wasserstoff; und
- X²: für Sauerstoff steht,
umgesetzt werden, und
das so entstandene 3-Phenyluracil der Formel I.A.1,
wobei R¹, R^{b} und R^{d} Wasserstoff bedeuten, anschließend
mit einem Alkylierungsmittel der Formel VI,

R¹-L⁴ VI,

worin
- R¹: C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl, und
- L⁴: eine nucleophil verdrängbare Abgangsgruppe, bevorzugt die als voranstehend bevorzugt genannten Bedeutungen
bedeutet;
zu 3-Phenyluracilen der Formel I.A.1,
wobei
- R¹: C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl; und
- R^{b} und R^{d}: Wasserstoff bedeuten;
alkyliert wird: 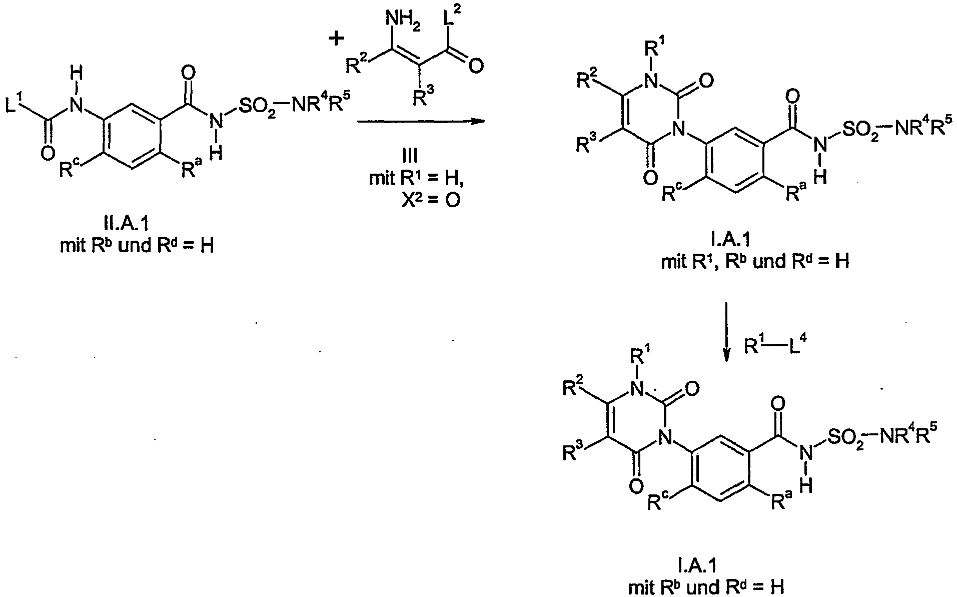
- L¹: steht für eine nucleophil verdrängbare Abgangsgruppe,
bevorzugt C₁-C₆-Alkoxy oder C₁-C₆-Alkylthio,
besonders bevorzugt C₁-C₆-Alkoxy,
insbesondere bevorzugt C₁-C₄-Alkoxy,
sehr bevorzugt Methoxy oder Ethoxy;
- L²: steht für eine nucleophil verdrängbare Abgangsgruppe;
bevorzugt für C₁-C₆-Alkoxy, C₁-C₆-Halogenalkoxy, C₁-C₄-Alkoxy-C₂-C₄-alkoxy, C₁-C₄-Alkylthio-C₂C₄-alkoxy, C₂-C₆-Alkenyloxy, C₂-C₆-Halogenalkenyloxy, C₃-C₆-Alkinyloxy, C₃-C₆-Halogenalkinyloxy, C₃-C₈-Cycloalkyloxy, C₁-C₆-Cyanoalkoxy oder Benzyloxy,
das seinerseits am Phenylring partiell oder vollständig halogeniert sein kann und/oder durch ein bis drei Reste aus der Gruppe Cyano, Nitro, C₁-C₄-Alkyl, C₁-C₄-Alkoxy und C₁-C₄-Alkylthio substituiert sein kann;
vorzugsweise für C₁-C₆-Alkoxy, C₁-C₆-Halogenalkoxy, C₁-C₄-Alkoxy-C₂-C₄-alkoxy, C₂-C₆-Alkenyloxy, C₂-C₆-Halogenalkenyloxy, C₃-C₆-Alkinyloxy oder C₃-C₆-Halogenalkinyloxy;
sehr bevorzugt für C₁-C₆-Alkoxy, C₁-C₄-Alkoxy-C₂-C₄-alkoxy, C₂-C₆-Alkenyloxy oder C₃-C₆-Alkinyloxy;
außerordentlich bevorzugt für C₁-C₆-Alkoxy.

Dabei ist die Herstellung insbesondere solcher 3-Phenyluracile der Formel I.A.1 mit R^{b} und R^{d} = Wasserstoff bevorzugt, in denen die Variablen R¹, R², R³, R^{a}, R^{c} sowie R⁴ und R⁵ jeweils für sich alleine als auch in Kombination miteinander folgende Bedeutungen haben:
- R¹: C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl;
sehr bevorzugt C₁-C₄-Alkyl,
besonders bevorzugt Methyl oder Ethyl,
insbesondere bevorzugt Methyl;
- R²: Wasserstoff, C₁-C₄-Alkyl oder C₁-C₄-Halogenalkyl,
besonders bevorzugt Wasserstoff, Methyl, Difluormethyl oder Trifluormethyl,
insbesondere bevorzugt Trifluormethyl;
- R³: Wasserstoff oder C₁-C₄-Alkyl,
besonders bevorzugt Wasserstoff.
- R^{a}: Wasserstoff, Halogen oder Cyano,
insbesondere bevorzugt Wasserstoff, Fluor, Chlor oder Cyano, sehr bevorzugt Wasserstoff, Chlor oder Cyano,
außerordentlich bevorzugt Wasserstoff oder Chlor,
sehr außerordentlich bevorzugt Wasserstoff;
- R^{c}: Wasserstoff oder Halogen,
insbesondere bevorzugt Wasserstoff, Fluor oder Chlor,
sehr bevorzugt Wasserstoff oder Fluor,
außerordentlich bevorzugt Fluor;
- R⁴ und R⁵: unabhängig voneinander
Wasserstoff oder C₁-C₆-Alkyl, das seinerseits durch einen Substituenten ausgewählt aus der Gruppe
Halogen, Cyano und C₁-C₄-Alkoxy, bevorzugt Halogen,
substituiert sein kann;
insbesondere bevorzugt
R⁴ Wasserstoff, C₁-C₆-Alkyl, C₂-C₆-Alkenyl oder C₂-C₆-Alkinyl,
sehr bevorzugt Wasserstoff oder C₁-C₆-Alkyl,
besonders bevorzugt C₁-C₄-Alkyl,
außerordentlich bevorzugt Methyl; und
R⁶ C₁-C₆-Alkyl, C₃-C₈-Cycloalkyl oder Phenyl,
sehr bevorzugt C₁-C₆-Alkyl,
besonders bevorzugt C₁-C₄-Alkyl.

Die Einführung der Aminogruppe am (Thio)Uracilring beziehungsweise Dithiouracilring gelingt überraschenderweise in Anlehnung an bekannte Verfahren zur Einführung der Aminogruppe am Uracilstickstoff. Solche Verfahren sind beispielsweise in der DE 196 52431 beschrieben, auf deren Offenbarung zur elektrophilen Aminierung hiermit im vollen Umfang Bezug genommen. Als geeignete Aminierungsmittel der Formel VII kommen beispielsweise 1-Aminooxy-2,4-dinitrobenzol oder O-Mesitylensulfonylhydroxylamin in Betracht.

Gegebenenfalls erfolgt die Umsetzung in Gegenwart einer Base. Als Base kommen alle üblichen anorganischen oder organischen Basen in Betracht. Geeignete Basen sind z.B. die im Zusammenhang mit der Herstellung der Verbindung I durch Umsetzung von II mit III genannten Basen. Bevorzugte Basen sind Alkalimetallalkoholate, insbesondere Lithium-, Natrium- oder Kaliumalkoholate wie Natriummethylat, Natriumethylat, Lithiumethylat, Kaliummethylat, Kaliumethylat, Kalium-tert.-butylat, Natrium-tert.-butylat, Natrium-isopropylat, Kalium-tert.-pentylat, Alkalimetallhydride wie Natriumhydrid, Kaliumhydrid, Alkalimetallcarbonate wie Lithiumcarbonat, Natriumcarbonat, Kaliumcarbonat, Cäsiumcarbonat oder tertiäre Amine, insbesondere Amidinbasen wie 1,8-Diazabicyclo[5.4.0]undec-7-en. In der Regel setzt man die Verbindung I mit R¹ = Wasserstoff und die Base annähernd äquimolar ein.

Die Umsetzung der Verbindung I mit R¹ = Wasserstoff mit einem Aminierungsreagenz der Formel VII erfolgt in der Regel in einem inerten organischen Lösungsmittel oder Lösungsmitteigemisch. Für diesen Zweck bevorzugte Lösungsmittel sind Nitrile wie Acetonitril, Propionitril oder Butyronitril, Ketone wie Aceton und Methylethylketon, Carbonate wie Dimethylcarbonat, Diethylcarbonat und Ethylencarbonat sowie Amide wie N,N-Dimethylformamid, N,N-Dimethylacetamid und N-Methylpyrrolidon. Geeignet sind auch organische Lösungsmittel mit basischem Charakter, z. B. die vorgenannten tertiären Amine wie Trialkylamine und Pyridinverbindungen.

In der Regel wird man die Umsetzung bei Temperaturen von 0 bis 80 °C, vorzugsweise zwischen 10 und 60 °C durchführen. Hierfür setzt man in der Regel die Verbindung I mit R¹ = Wasserstoff und das Aminierungsreagenz der Formel VII annähernd äquimolar ein. Es ist jedoch auch möglich, eine der Komponenten in einem größeren Überschuss zu verwenden, wobei der Überschuss vorzugsweise nicht mehr als 50 mol-%, bezogen auf die im Unterschuss vorliegende Komponente, betragen wird.

Die Aufarbeitung des Reaktionsgemisches zur Gewinnung des Zielproduktes I kann nach den hierfür üblichen Methoden erfolgen. In der Regel wird man das verwendete Lösungsmittel nach üblichen Verfahren, beispielsweise destillativ, entfernen. Man kann dann die Zielverbindung I in einem mit Wasser nicht mischbaren organischen Lösungsmittel aufnehmen, extrahiert etwaige Verunreinigungen mit gegebenenfalls angesäuertem Wasser, trocknet und entfernt das Lösungsmittel unter vermindertem Druck. Zur weiteren Reinigung kann man die üblichen Verfahren wie Kristallisation, Fällung oder Chromatographie anwenden. Bei Verwendung eines Zweiphasensystems wird man in der Regel extraktiv aufarbeiten.

Verbindungen der Formel I, worin einer der Reste X¹, X² oder X³ oder die Reste X¹, X² und X³ jeweils für Sauerstoff stehen, können nach bekannten Methoden durch Behandeln mit Schwefelungsmitteln in Verbindungen der allgemeinen Formel I umgewandelt werden, worin einer der Reste X¹, X² oder X³ oder die Reste X¹, X² und X³ jeweils für Schwefel stehen. Beispiele für geeignete Schwefelungsmitteln sind Organophosphorsulfide wie das Lawesson-Reagenz, Organozinnsulfide oder Phosphor(V)sulfide (siehe auch J. March, Advanced Organic Synthesis, 2. Auflage, Wiley Interscience 1985, S. 794 und dort zitierte Literatur). Die Umsetzung kann in einem Lösungsmittel oder in Substanz durchgeführt werden. Geeignete Lösungsmittel sind die oben genannten, inerten Lösungsmittel sowie basische Lösungsmittel wie Pyridin und vergleichbare. Die zur Umsetzung erforderliche Temperatur liegt in der Regel oberhalb Raumtemperatur und liegt insbesondere im Bereich von 50 bis 200 °C. Führt man die Umsetzung des Enamins IIII mit einem Isothiocyanat II, in dem der Rest X¹ für Schwefel steht durch, so erhält man direkt die entsprechenden 2-Thioxouracile mit X¹ = Schwefel.

Die für die Herstellung der 3-Phenyl(thio)uracile und -dithiouracile der Formel I benötigten Carbamate der Formel II sind erhältlich, indem ein Amin der Formel IV mit einer Verbindung der Formel V umgesetzt wird:

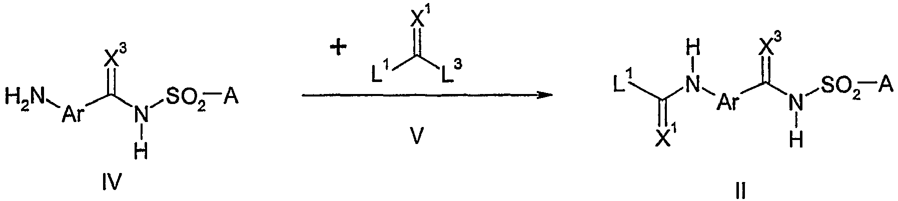

Die Variablen X¹, X³, Ar, A und L¹ haben die zuvor genannten Bedeutungen, insbesonders bevorzugt die in der Beschreibung als bevorzugt genannten Bedeutungen.
- L³: steht für eine nucleophil verdrängbare Abgangsgruppe,
bevorzugt für Chlor oder C₁-C₆-Alkoxy;
besonders bevorzugt für Chlor.

Die Umsetzung der Amine der Formel IV mit Verbindungen der Formel Verfolgt üblicherweise bei Temperaturen von -10 °C bis 160 °C, vorzugsweise 0°C bis 130°C, sehr bevorzugt 25 °C bis 130 °C, in einem inerten organischen Lösungsmittel in gegebenenfalls in Gegenwart einer Base (z B. Houben-Weyl, Methoden der organischen Chemie, E5, 1985, S. 972-980, sowie VIII, S. 655 und XI Teil 2, S. 10).

Der Reaktionsdruck ist für den Erfolg des erfindungsgemäßen Verfahrens von untergeordneter Bedeutung und kann beispielsweise im Bereich von 500 mbar bis 10 bar liegen. Vorzugsweise wird die Reaktion im Bereich des Normaldrucks, d.h. im Bereich von 0,9 bis 1,2 bar durchgeführt.

Die für die Umsetzung erforderliche Reaktionszeit liegt in der Regel im Bereich von 1 h bis 24h, und insbesondere im Bereich von 2h bis 8h.

Die Reaktion kann grundsätzlich in Substanz durchgeführt werden. Vorzugsweise setzt man jedoch die Amine der Formel IV mit den Verbindungen der Formel V in einem organischen Lösungsmittel um. Grundsätzlich geeignet sind alle Lösungsmittel, welche die Verbindungen IV und V zumindest teilweise und vorzugsweise vollständig unter Reaktionsbedingungen zu lösen vermögen.

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Carbonsäureester wie Butylacetat, besonders bevorzugt halogenierte Kohlenwasserstoffe und Ether.

Es können auch Gemische der genannten Lösungsmittel verwendet werden.

Die Umsetzung der Amine der Formel IV mit Verbindungen der Formel V kann in Gegenwart einer Base durchgeführt werden, jedoch ist der Einsatz einer Base nicht erforderlich.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetallamide wie Lithiumamid, Natriumamid und Kaliumamid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, sowie Alkalimetall- und Erdalkalimetallalkoholate wie Natriummethanolat, Natriumethanolat, Kaliumethanolat, Kalium- tert.-Butanolat, Kalium-tert.-Pentanolat und Dimethoxymagnesium, außerdem organische Basen, z.B. tertiäre Amine wie Trimethylamin, Triethylamin, Diisopropylethylamin, N-Methylpiperidin und N-Methylmorpholin, Pyridin, substituierte Pyridine wie Picolin, Collidin, Lutidin und 4-Dimethylaminopyridin sowie bicyclische Amine in Betracht. Besonders bevorzugt werden organische Basen wie Triethylamin, Pyridin und Picolin.

Die Basen werden im allgemeinen in äquimolaren Mengen eingesetzt, sie können aber auch katalytisch, im Überschuß oder gegebenenfalls als Lösungsmittel verwendet werden.

In einer bevorzugten Variante des erfindungsgemäßen Verfahrens erfolgt die Umsetzung der Amine der Formel IV mit Verbindungen der Formel V in Abwesendheit einer Base.

Diese bevorzugte Variante der Reaktionsführung bietet u.a. die Vorteile, dass eine aufwändige Aufarbeitung nach Reaktionsende entfällt und die weitere Umsetzung des so hergestellten Carbamates II zu 3-Phenyl(thio)uracilen und -dithiouracilen I, bevorzugt 3-Phenyluracilen I.A.1, wobei auch noch gegebenenfalls die zuvor genannte Chlorierung des Carbamats II durchgeführt werden kann, als Eintopf-Reaktion erfolgen kann bzw. nur einen Lösungsmitteltausch erfordert.

Die Edukte werden im allgemeinen in äquimolaren Mengen miteinander umgesetzt. Es kann vorteilhaft sein, V in einem Überschuß bezogen auf IV einzusetzen. Vorzugsweise wird man die Verbindungen in einem Molverhältnis IV : V im Bereich von 1,6: 1 bis 1 : 1,6, insbesondere 1: 1,4 bis 1: 1 einsetzen.

Vorzugsweise wird man die bei der Umsetzung der Amine der Formel IV mit den Verbindungen der Formel V gebildete Verbindung L³-H zu wenigstens 80% während der Umsetzung aus der Reaktionsmischung entfernen, insbesondere wenn es sich bei der Verbindung L³-H um ein C₁-C₄-Alkanol wie Methanol oder Ethanol handelt.

Hierzu wird man in an sich bekannter Weise die Reaktion bei einer Temperatur und einem Druck durchführen, bei der die Verbindung L³-H gegebenenfalls als Azeotrop mit dem Lösungsmittel aus der Reaktionsmischung abdestilliert. Gegebenenfalls wird man zum Ausgleich frisches Lösungsmittel in die Reaktion einbringen oder das mit der Verbindung L³-H abdestillierte Lösungsmittel nach ggf. destillativer Abreicherung der Verbindung L³-H in die Reaktion zurückführen.

Aus diesen Gründen ist es von Vorteil, wenn das eingesetzte Lösungsmittel einen Siedepunkt von wenigstens 10 °C und insbesondere wenigstens 30 °C oberhalb des Siedepunktes der bei der Reaktion gebildeten Verbindung L³-H aufweist (jeweils bei Normaldruck).

Zweckmäßigerweise führt man die Umsetzung der Amine der Formel IV mit den Verbindungen der Formel V in einer Apparatur durch, die mit wenigstens einer Destillations- und Rektifikationsvorrichtung, z.B. einer Destillationskolonne versehen ist, welche zum einen ein Abdestillieren der Verbindung L³-H, ggf. zusammen mit Lösungsmittel erlaubt und gleichzeitig eine Abtrennung und Rückführung des gegebenenfalls mit der Verbindung L³-H abdestillierten Lösungsmittels ermöglicht.

Zur Umsetzung können die Amine der Formel IV mit den Verbindungen der Formel V und gegebenenfalls die Base in an sich beliebiger Weise miteinander in Kontakt gebracht werden, d.h., die Reaktanden und gegebenenfalls die Base können getrennt, gleichzeitig oder nacheinander in das Reaktionsgefäß eingebracht und zur Reaktion geführt werden. Beispielsweise kann man die Amine IV und die Verbindungen V in einem Reaktionsgefäß, gegebenenfalls mit dem gewünschten Lösungsmittel, vorlegen und dann die gewünschten Reaktionsbedingungen einstellen.

Man kann jedoch auch die Hauptmenge oder Gesamtmenge an Amin IV und Verbindung V, gegebenenfalls in einem Lösungsmittel unter Reaktionsbedingungen in das Reaktionsgefäß eintragen.

In einer besonders bevorzugten Ausführungsform legt man das Amin IV vor, gibt anschließend gegebenenfalls die Base und danach die Verbindung V zu.

In einer besonders bevorzugten Ausführungsform der Erfindung legt man die Hauptmenge, insbesondere wenigstens 80 % und besonders bevorzugt die Gesamtmenge oder nahezu die Gesamtmenge (> 95 %) der Amine der Formel IV vor, und gibt hierzu im Verlauf der Reaktion, zB. über einen Zeitraum von 0,5 bis 20 h und insbesondere 1 bis 10 h, die Hauptmenge, insbesondere wenigstens 80 % und besonders bevorzugt die Gesamtmenge oder nahezu die Gesamtmenge (> 95 %) der Verbindungen der Formel V unter Reaktionsbedingungen zu. Hierzu wird man die Verbindungen der Formel V vorzugsweise in einem Lösungsmittel lösen. Gegebenenfalls wird man im Anschluß an die Zugabe der Verbindungen der Formel V dann noch eine gewisse Zeit, z.B. 1 h bis 10 h, insbesondere 2 h bis 5 h, nachreagieren lassen.

Die Aufarbeitung und Isolierung der Carbamate der Formel II kann in an sich bekannter Weise erfolgen.

Im Besonderen können auf diesem Weg Carbamate der Formel II.A.1, 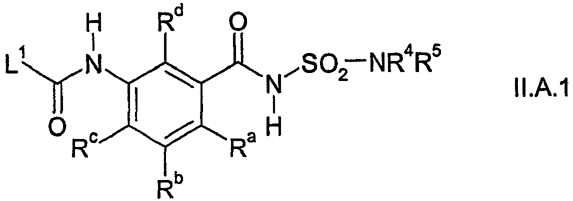 wobei R^{b} und R^{d} Wasserstoff bedeuten,
hergestellt werden, indem entsprechende Amine der Formel IV.A.1, 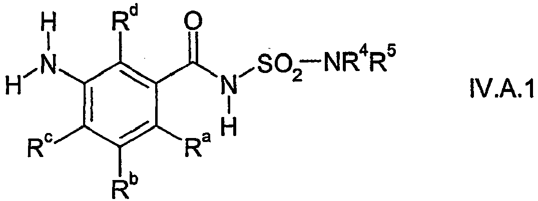 wobei R^{b} und R^{d} Wasserstoff bedeuten,
mit Verbindungen V 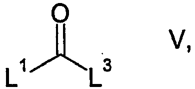 wobei X¹ für Sauerstoff steht,
umgesetzt werden: 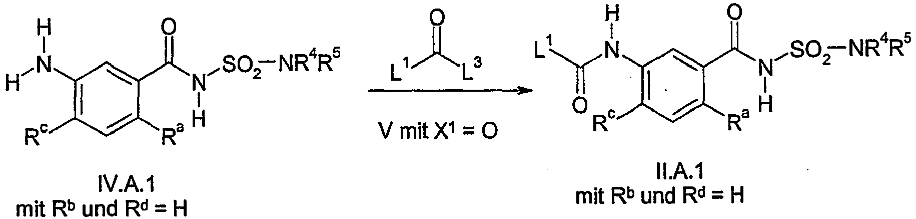
- L³: steht für eine nucleophil verdrängbare Abgangsgruppe,
bevorzugt für Chlor oder C₁-C₆-Alkoxy;
besonders bevorzugt für Chlor.

Dabei ist die Herstellung insbesondere solcher Carbamate der Formel II.A.1 mit R^{b} und R^{d} = Wasserstoff bevorzugt, in denen die Variablen L¹, R^{a}, R^{c} sowie R⁴ und R⁵ jeweils für sich alleine als auch in Kombination miteinander folgende Bedeutungen haben:
- L¹: nucleophil verdrängbare Abgangsgruppe,
bevorzugt C₁-C₆-Alkoxy oder C₁-C₆-Alkylthio, besonders bevorzugt C₁-C₆-Alkoxy,
insbesondere bevorzugt C₁-C₄-Alkoxy,
sehr bevorzugt Methoxy oder Ethoxy;
- R^{a}: Wasserstoff, Halogen oder Cyano,
insbesondere bevorzugt Wasserstoff, Fluor, Chlor oder Cyano,
sehr bevorzugt Wasserstoff, Chlor oder Cyano,
außerordentlich bevorzugt Wasserstoff oder Chlor,
sehr außerordentlich bevorzugt Wasserstoff;
- R^{c}: Wasserstoff oder Halogen,
insbesondere bevorzugt Wasserstoff, Fluor oder Chlor,
sehr bevorzugt Wasserstoff oder Fluor,
außerordentlich bevorzugt Fluor;
- R⁴ und R⁵: unabhängig voneinander
Wasserstoff oder C₁-C₆-Alkyl, das seinerseits durch einen Substituenten ausgewählt aus der Gruppe
Halogen, Cyano und C₁-C₄-Alkoxy, bevorzugt Halogen,
substituiert sein kann;
insbesondere bevorzugt
R⁴ Wasserstoff, C₁-C₆-Alkyl, C₂-C₆-Alkenyl oder C₂-C₆-Alkinyl,
sehr bevorzugt Wasserstoff oder C₁-C₆-Alkyl,
besonders bevorzugt C₁-C₄-Alkyl,
außerordentlich bevorzugt Methyl; und
R⁵ C₁-C₆-Alkyl, C₃-C₈-Cycloalkyl oder Phenyl,
sehr bevorzugt C₁-C₆-Alkyl,
besonders bevorzugt C₁-C₄-Alkyl.

Die für die Herstellung der Carbamate der Formel II benötigten Amine der Formel IV sind in der Literatur bekannt (z. B. WO 04/039768) oder können gemäß der zitierten Literatur hergestellt werden.

Die für die Herstellung der Carbamate der Formel II benötigten Verbindungen der Formel V sind in der Literatur bekannt (z. B. Houben-Weyl, Methoden der organischen Chemie, E4, 1983, S. 6-17) und können käuflich erworben oder gemäß der zitierten Literatur hergestellt werden.

Die auf diesem Weg hergestellten Carbamate der Formel II können anschließend, falls gewünscht, in einem Zwischenschritt vor der Umsetzung mit den Enaminen der Formel III am Rest Ar halogeniert werden.

Bevorzugt können die auf diesem Weg hergestellten Carbamate der Formel II in einem weiteren Schritt am Rest Ar chloriert oder bromiert, sehr bevorzugt chloriert werden.

Die Halogenierung erfolgt üblicherweise bei Temperaturen von 0 °C bis 100 °C, vorzugsweise 20 °C bis 70 °C, in einem inerten organischen Lösungsmittel gegebenenfalls in Gegenwart eines Katalysators (z. B. Buehler, Peason, Survey of Organic Synthesis, Interscience Publishers 1970, S. 392-404).

Der Reaktionsdruck ist für den Erfolg des erfindungsgemäßen Verfahrens von untergeordneter Bedeutung und kann beispielsweise im Bereich von 500 mbar bis 10 bar liegen. Vorzugsweise wird die Reaktion im Bereich des Normaldrucks, d.h. im Bereich von 0,9 bis 1,2 bar durchgeführt

Die für die Umsetzung erforderliche Reaktionszeit liegt in der Regel im Bereich von 1 h bis 24h, und insbesondere im Bereich von 3h bis 12h.

Die Reaktion kann grundsätzlich in Substanz durchgeführt werden. Vorzugsweise setzt man jedoch die Carbamate der Formel V mit dem Halogenierungsmittel in einem organischen Lösungsmittel um. Grundsätzlich geeignet sind alle Lösungsmittel, welche die Verbindung V zumindest teilweise und vorzugsweise vollständig unter Reaktionsbedingungen zu lösen vermögen.

Geeignete Lösungsmittel sind aliphatische Kohlenwasserstoffe wie Pentan, Hexan, Cyclohexan und Gemische von C₅-C₈-Alkanen, halogenierte Kohlenwasserstoffe wie Methylenchlorid, Chloroform, Butylchlorid und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Carbonsäuren wie Ameisensäure oder Essigsäure, Ester wie Butylacetat oder Dimethylcarbonat, sowie Sulfurylchlorid, besonders bevorzugt halogenierte Kohlenwasserstoffe und Sulfurylchlorid.

Es können auch Gemische der genannten Lösungsmittel verwendet werden.

Die für die Halogenierung benötigten Halogenierungsmittel sind in der Literatur bekannt (z. B. Buehler, Peason, Survey of Organic Synthesis, Interscience Publishers 1970, S. 392-404) oder können gemäß der zitierten Literatur hergestellt werden.

Als Halogenierungsmittel finden z.B. Chlor, N-Chlorsuccinimid, SO₂Cl₂, HCl/H₂O₂, 1,3-Dichlor-5,5-dimethylhydanthoin, Brom, N-Bromsuccinimid, HBr/H₂O₂ oder 1,3-Dibrom-5,5-dimethylhydanthoin Verwendung.

Die Halogenierung der Carbamate der Formel II kann, je nach gewähltem Halogenierungsmittel, in Gegenwart eines Katalysators durchgeführt werden.

Als Katalysatoren finden z.B. Lewis-Säuren wie Bortrifluorid, Aluminiumtrichlorid, Eisen-III-chlorid, Zinn-IV-chlorid, Titan-IV-chlorid und Zink-II-chlorid Verwendung.

Das Halogenierungsmittel wird im allgemeinen äquimolar eingesetzt, es kann aber auch im Unterschuß, im Überschuß oder gegebenenfalls als Lösungsmittel verwendet werden.

Die Aufarbeitung und Isolierung der halogenierten Carbamate der Formel II kann in an sich bekannter Weise erfolgen.

Es kann von Vorteil sein, die bei der Reaktion entstandenen Beiprodukte aus dem Halogenierungsreagenz durch Zugabe einer geeigneten Base teilweise bzw. ganz abzufangen.

Im Besonderen können auf diesem Weg para-chlorierte Carbamate der Formel II.A.1,
wobei R^{a} = Chlor und R^{b} und R^{d} = Wasserstoff sind,
hergestellt werden, indem entsprechende Carbamate der Formel II.A.1,
wobei R^{a}, R^{b} und R^{d} = Wasserstoff sind,
chloriert werden:

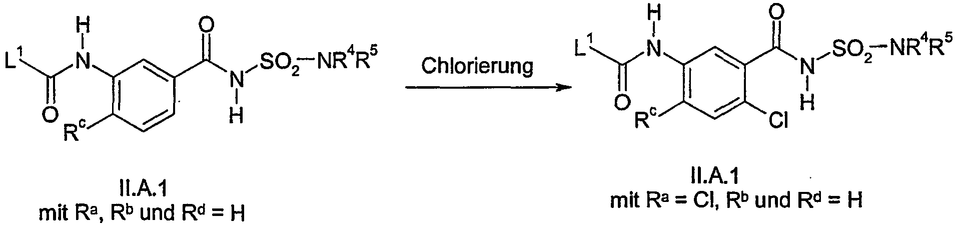

Dabei ist die Chlorierung insbesondere solcher Carbamate der Formel II.A.1 mit R^{a}, R^{b} und R^{d} = Wasserstoff bevorzugt, in denen die Variablen L¹, R^{c} sowie R⁴ und R⁵ jeweils für sich alleine als auch in Kombination miteinander folgende Bedeutungen haben:
- L¹: nucleophil verdrängbare Abgangsgruppe,
bevorzugt C₁-C₆-Alkoxy oder C₁-C₆-Alkylthio,
besonders bevorzugt C₁-C₆-Alkoxy,
insbesondere bevorzugt C₁-C₄-Alkoxy,
sehr bevorzugt Methoxy oder Ethoxy;
- R^{c}: Wasserstoff oder Halogen,
insbesondere bevorzugt Wasserstoff, Fluor oder Chlor,
sehr bevorzugt Wasserstoff oder Fluor,
außerordentlich bevorzugt Fluor;
- R⁴ und R⁵: unabhängig voneinander
Wasserstoff oder C₁-C₆-Alkyl, das seinerseits durch einen Substituenten ausgewählt aus der Gruppe
Halogen, Cyano und C₁-C₄-Alkoxy, bevorzugt Halogen,
substituiert sein kann;
insbesondere bevorzugt
R⁴ Wasserstoff, C₁-C₆-Alkyl, C₂-C₆-Alkenyl oder C₂-C₆-Alkinyl,
sehr bevorzugt Wasserstoff oder C₁-C₆-Alkyl,
besonders bevorzugt C₁-C₄-Alkyl,
außerordentlich bevorzugt Methyl; und
R⁵ C₁-C₆-Alkyl, C₃-C₈-Cycloalkyl oder Phenyl,
sehr bevorzugt C₁-C₆-Alkyl,
besonders bevorzugt C₁-C₄-Alkyl.

Die hier beschriebene Umsetzung der Amine der Formel IV mit Verbindungen der Formel V liefert die Carbamate der Formel II in hoher Ausbeute.

Eine weitere bevorzugte Ausführungsform betrifft daher ein Verfahren, bei dem man in einem ersten Schritt die Amine der Formel IV mit Verbindungen der Formel V zu Carbamaten der Formel II umsetzt, und anschließend die Carbamate der Formel II ohne Isolierung mit den Enaminen der Formel III zu 3-Phenyl(thio)uracilen und-dithiouracilen der Formel I umsetzt. Hierzu kann es von Vorteil sein, wenn man einen Teil oder die Gesamtmenge des zur Herstellung des Carbamats der Formel II verwendeten Lösungsmittels entfernt und durch ein anderes Lösungsmittel substituiert. Insbesondere wird man jedoch die Umsetzung der Carbamate der Formel II mit den Enaminen der Formel III in dem zur Herstellung des Carbamats der Formel II verwendeten Lösungsmittel durchführen.

Als insbesondere bevorzugt werden folgende Schritte des erfindungsgemäßen Verfahrens genannt, wobei die Bevorzugung sowohl für jeden Einzelschritt als auch für das Gesamtverfahren gilt. Die bevorzugten Ausführungsformen der folgenden Sachritte sind wie voranstehend genannt.

In einer Ausführungsform des erfindungsgemäßen Verfahrens können 3-Phenyluracile der Formel I.A.1, wobei R¹ C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl; und R^{b} und R^{d} Wasserstoff bedeuten; hergestellt werden, indem entsprechende Carbamate der Formel II.A.1, wobei R^{a} Chlor und R^{b} und R^{d} Wasserstoff bedeuten, mit Enaminen der Formel III, wobei R¹ für Wasserstoff; und X² für Sauerstoff steht, umgesetzt werden,
und das so entstandene 3-Phenyluracil der Formel I.A.1, wobei R^{a} Chlor und R¹, R^{b} und R^{d} Wasserstoff bedeuten, anschließend mit einem Alkylierungsmittel der Formel VI, worin R¹ C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl, und L⁴ eine nucleophil verdrängbare Abgangsgruppe bedeutet, zu 3-Phenyluracilen der Formel I.A.1, wobei R¹ C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogen-alkenyl, C₃-C₆-Alkinyl, C₃-C₆-Halogenalkinyl oder Phenyl-C₁-C₄-alkyl; und R^{b} und R^{d} Wasserstoff bedeuten; alkyliert wird.

In einer weiteren Ausführungsform des erfindungsgemäßen Verfahrens können 3-Phenyluracile der Formel I.A.1, wobei R¹ C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl; und R^{b} und R^{d} Wasserstoff bedeuten; hergestellt werden, indem entsprechende Carbamate der Formel II.A.1, wobei R^{a} Chlor und R^{b} und R^{d} Wasserstoff bedeuten, mit Enaminen der Formel III, wobei R¹ für C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl und X² für Sauerstoff steht, umgesetzt werden.

In einer weiteren Ausführungsform des erfindungsgemäßen Verfahrens können 3-Phenyluracile der Formel I.A.1, wobei R¹ C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl; und R^{b} und R^{d} Wasserstoff bedeuten, hergestellt werden,
indem Carbamate der Formel II.A.1, wobei R^{a}, R^{b} und R^{d} = Wasserstoff sind, zu para-chlorierten Carbamaten der Formel II.A.1, wobei R^{a} = Chlor und R^{b} und R^{d} = Wasserstoff sind, chloriert werden,
diese anschließend mit Enaminen der Formel III, wobei R¹ für Wasserstoff; und X² für Sauerstoff steht, umgesetzt werden,
und das so entstandene 3-Phenyluracil der Formel I.A.1, wobei R^{a} Chlor und R¹, R^{b} und R^{d} Wasserstoff bedeuten, anschließend mit einem Alkylierungsmittel der Formel VI, worin R¹ C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl, und L⁴ eine nucleophil verdrängbare Abgangsgruppe bedeutet, zu 3-Phenyluracilen der Formel I.A.1, wobei R¹ C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogen-alkenyl, C₃-C₆-Alkinyl, C₃-C₆-Halogenalkinyl oder Phenyl-C₁-C₄-alkyl; und R^{b} und R^{d} Wasserstoff bedeuten; alkyliert wird.

In einer weiteren Ausführungsform des erfindungsgemäßen Verfahrens können 3-Phenyluracile der Formel I.A.1, wobei R¹ C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl; und R^{b} und R^{d} Wasserstoff bedeuten; hergestellt werden,
indem Carbamate der Formel II.A.1, wobei R^{a}, R^{b} und R^{d} = Wasserstoff sind, zu para-chlorierten Carbamaten der Formel II.A.1, wobei R^{a} = Chlor und R^{b} und R^{d} = Wasserstoff sind, chloriert werden, und
diese anschließend mit Enaminen der Formel III, wobei R¹ für C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl und X² für Sauerstoff steht, umgesetzt werden.

In einer weiteren Ausführungsform des erfindungsgemäßen Verfahrens können 3-Phenyluracile der Formel I.A.1, wobei R¹ C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl; und R^{b} und R^{d} Wasserstoff bedeuten; hergestellt werden,
indem entsprechende Amine der Formel IV.A.1, wobei R^{a}, R^{b} und R^{d} Wasserstoff bedeuten, mit Verbindungen V, wobei X¹ für Sauerstoff steht, umgesetzt werden; danach die so erhaltenen Carbamate der Formel II.A.1, wobei R^{a}, R^{b} und R^{d} = Wasserstoff sind, zu para-chlorierten Carbamaten der Formel II.A.1, wobei R^{a} = Chlor und R^{b} und R^{d} = Wasserstoff sind, chloriert werden,
diese anschließend mit Enaminen der Formel III, wobei R¹ für Wasserstoff; und X² für Sauerstoff steht, umgesetzt werden,
und das so entstandene 3-Phenyluracil der Formel I.A.1, wobei R^{a} Chlor und R¹, R^{b} und R^{d} Wasserstoff bedeuten, anschließend mit einem Alkylierungsmittel der Formel VI, worin R¹ C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl, und L⁴ eine nucleophil verdrängbare Abgangsgruppe bedeutet, zu 3-Phenyluracilen der Formel I.A.1, wobei R¹ C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogen-alkenyl, C₃-C₆-Alkinyl, C₃-C₆-Halogenalkinyl oder Phenyl-C₁-C₄-alkyl; und R^{b} und R^{d} Wasserstoff bedeuten; alkyliert wird.

In einer weiteren Ausführungsform des erfindungsgemäßen Verfahrens können 3-Phenyluracile der Formel I.A.1, wobei R¹ C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl; und R^{b} und R^{d} Wasserstoff bedeuten; hergestellt werden,
indem entsprechende Amine der Formel IV.A.1, wobei R^{a}, R^{b} und R^{d} Wasserstoff bedeuten, mit Verbindungen V, wobei X¹ für Sauerstoff steht, umgesetzt werden; danach die so erhaltenen Carbamate der Formel II.A.1, wobei R^{a}, R^{b} und R^{d} = Wasserstoff sind, zu para-chlorierten Carbamaten der Formel II.A.1, wobei R^{a} = Chlor und R^{b} und R^{d} = Wasserstoff sind, chloriert werden, und
diese anschließend mit Enaminen der Formel III, wobei R¹ für C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl und X² für Sauerstoff steht, umgesetzt werden.

In einer weiteren Ausführungsform des erfindungsgemäßen Verfahrens können 3-Phenyluracile der Formel I.A.1, wobei R¹ C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl; und R^{b} und R^{d} Wasserstoff bedeuten; hergestellt werden,
indem entsprechende Amine der Formel IV.A.1, wobei R^{a} = Chlor und R^{b} und R^{d} Wasserstoff bedeuten, mit Verbindungen V, wobei X¹ für Sauerstoff steht, umgesetzt werden;
danach die so erhaltenen Carbamate der Formel IIA.1, wobei R^{a} = Chlor und R^{b} und R^{d} = Wasserstoff sind, mit Enaminen der Formel III, wobei R¹ für Wasserstoff; und X² für Sauerstoff steht, umgesetzt werden,
und das so entstandene 3-Phenyluracil der Formel I.A.1, wobei R^{a} Chlor und R¹, R^{b} und R^{d} Wasserstoff bedeuten, anschließend mit einem Alkylierungsmittel der Formel VI, worin R¹ C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl, und L⁴ eine nucleophil verdrängbare Abgangsgruppe zu 3-Phenyluracilen der Formel I.A.1, wobei R¹ C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogen-alkenyl, C₃-C₆-Alkinyl, C₃-C₆-Halogenalkinyl oder Phenyl-C₁-C₄-alkyl; und R^{b} und R^{d} Wasserstoff bedeuten; alkyliert wird.

In einer weiteren Ausführungsform des erfindungsgemäßen Verfahrens können 3-Phenyluracile der Formel I.A.1, wobei R¹ C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl; und R^{b} und R^{d} Wasserstoff bedeuten; hergestellt werden,
indem entsprechende Amine der Formel IV.A.1, wobei R^{a} = Chlor und R^{b} und R^{d} Wasserstoff bedeuten, mit Verbindungen V, wobei X¹ für Sauerstoff steht, umgesetzt werden; und
danach die so erhaltenen Carbamate der Formel II.A.1, wobei R^{a} = Chlor und R^{b} und R^{d} = Wasserstoff sind, mit Enaminen der Formel III, wobei R¹ für C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl und X² für Sauerstoff steht, umgesetzt werden.

In einer weiteren Ausführungsform des erfindungsgemäßen Verfahrens können 3-Phenyluracile der Formel I.A.1, wobei R¹, R^{b} und R^{d} Wasserstoff bedeuten; hergestellt werden,
indem entsprechende Amine der Formel IV.A.1, wobei R^{a} = Chlor und R^{b} und R^{d} Wasserstoff bedeuten, mit Verbindungen V, wobei X¹ für Sauerstoff steht, umgesetzt werden; und
danach die entstandenen Carbamate der Formel II.A.1, wobei R^{a} Chlor und R^{b} und R^{d} Wasserstoff bedeuten, mit Enaminen der Formel III, wobei R¹ für Wasserstoff steht, umgesetzt werden.

Das erfindungsgemäße Verfahren erlaubt erstmalig die Herstellung von Carbamaten der Formel II 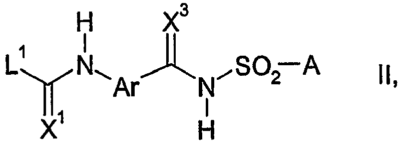 wobei X¹ und X³ sowie Ar und A die zuvor genannten Bedeutungen haben und L¹ für eine nucleophil verdrängbare Abgngsgruppe, bevorzugt für C₁-C₆-Alkoxy oder C₁-C₆-Alkylthio, besonders bevorzugt für C₁-C₆-Alkoxy steht.

Diese Verbindungen sind neu und ebenfalls Gegenstand der vorliegenden Erfindung.

Unter den Carbamaten der Formel II sind solche der Formel II.A (= Carbamate der Formel II, wobei X¹ und X³ für Sauerstoff und Ar für Ar-1 stehen) 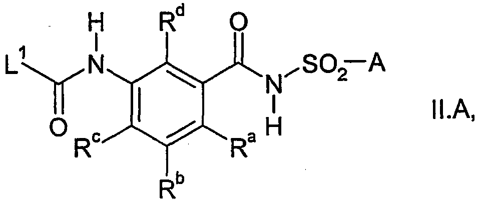 worin die Variablen R^{a}, R^{b}, R^{c} und R^{d} sowie A und L¹ die zuvor genannten Bedeutungen haben, bevorzugt.

Ganz besonders bevorzugt sind die Carbamate der Formel II.A.1 (= Carbamate der Formel II, wobei X¹ und X³ für Sauerstoff, Ar für Ar-1 und A für NR⁴R⁵ stehen) 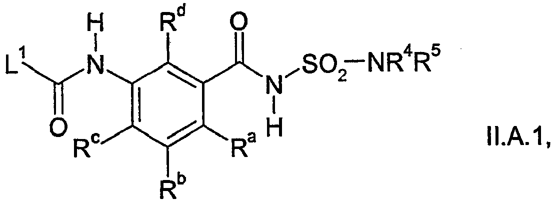 worin die Variablen R^{a}, R^{b}, R^{c} und R^{d} sowie R⁴, R⁵ und L¹ die zuvor genannten Bedeutungen haben.

Unter den Carbamaten der Formal II.A.1 sind insbesondere solche bevorzugt, in denen die Variablen L¹, R^{a}, R^{b}, R^{c} und R^{d} sowie R⁴ und R⁵ jeweils für sich allein oder in Kombination die folgenden Bedeutungen aufweisen:
- L¹: nucleophil verdrängbare Abgangsgruppe,
bevorzugt C₁-C₆-Alkoxy oder C₁-C₆-Alkylthio, besonders bevorzugt C₁-C₆-Alkoxy,
insbesondere bevorzugt C₁-C₄-Alkoxy,
sehr bevorzugt Methoxy oder Ethoxy;
- R^{a}: Wasserstoff, Halogen oder Cyano,
insbesondere bevorzugt Wasserstoff, Fluor, Chlor oder Cyano,
sehr bevorzugt Wasserstoff, Chlor oder Cyano,
außerordentlich bevorzugt Wasserstoff oder Chlor;
- R^{b}: Wasserstoff;
- R^{c}: Wasserstoff oder Halogen,
insbesondere bevorzugt Wasserstoff, Fluor oder Chlor,
sehr bevorzugt Wasserstoff oder Fluor,
außerordentlich bevorzugt Fluor;

- R^{d}: Wasserstoff;
- R⁴ und R⁵: unabhängig voneinander
Wasserstoff oder C₁-C₆-Alkyl, das seinerseits durch einen Substituenten ausgewählt aus der Gruppe
Halogen, Cyano und C₁-C₄-Alkoxy, bevorzugt Halogen,
substituiert sein kann;
insbesondere bevorzugt
R⁴ Wasserstoff, C₁-C₆-Alkyl, C₂-C₆-Alkenyl oder C₂-C₆-Alkinyl,
sehr bevorzugt Wasserstoff oder C₁-C₆-Alkyl,
besonders bevorzugt C₁-C₄-Alkyl,
außerordentlich bevorzugt Methyl; und
R⁵ C₁-C₆-Alkyl, C₃-C₈-Cycloalkyl oder Phenyl,
sehr bevorzugt C₁-C₆-Alkyl,
besonders bevorzugt C₁-C₄-Alkyl.

Außerordentlich bevorzugt sind die Carbamate der Formel IIA.1.a (entspricht Carbamaten der Formel II mit L¹ = Methoxy, X¹, X³ = Sauerstoff, Ar = Ar-1 mit R^{b} und R^{d} = Wasserstoff und R^{c} = Fluor sowie A = NR⁴R⁵⁾, insbesondere die Carbamate der Formel IIA.1.a.1 bis II.A.1.a.60 der Tabelle 1, wobei die Definitionen der Variablen R^{a}, R⁴ und R⁵ nicht nur in Kombination miteinander sondern auch jeweils für sich allein betrachtet für die erfindungsgemäßen Verbindungen eine besondere Rolle spielen.

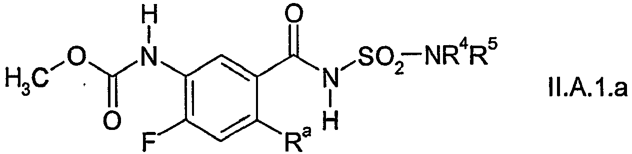

**Tabelle 1**

| Nr. | R^{a} | R⁴ | R⁵ |
|---|---|---|---|
| IIA.1.a.1 | H | H | H |
| II.A.1.a.2 | H | H | CH₃ |
| II.A.1.a.3 | H | H | C₂H₅ |
| II.A.1.a.4 | H | H | (CH₂)₂CH₃ |
| IIA.1.a.5 | H | H | CH(CH₃)₂ |
| IIA.1.a.6 | H | H | (CH₂)₃CH₃ |
| IIA.1.a.7 | H | H | CH(CH₃)CH₂CH₃ |
| II.A.1.a.8 | H | H | CH₂CH(CH₃)CH₃ |
| II.A.1.a.9 | H | H | C(CH₃)₃ |
| II.A.1.a.10 | H | H | CH₂CH=CH₂ |
| II.A.1.a.11 | H | H | CH₂-C≡CH |
| II.A.1.a.12 | H | CH₃ | CH₃ |
| II.A.1.a.13 | H | CH₃ | C₂H₅ |
| II.A.1.a.14 | H | CH₃ | (CH₂)₂CH₃ |
| II.A.1.a.15 | H | CH₃ | CH(CH₃)₂ |
| II.A.1.a.16 | H | CH₃ | (CH₂)₃CH₃ |
| II.A.1.a.17 | H | CH₃ | CH(CH₃)CH₂CH₃ |
| II.A.1.a.18 | H | CH₃ | CH₂CH(CH₃)CH₃ |
| II.A.1.a.19 | H | CH₃ | C(CH₃)₃ |
| II.A.1.a.20 | H | CH₃ | CH₂CH=CH₂ |
| II.A.1.a.21 | H | CH₃ | CH₂-C≡CH |
| II.A.1.a.22 | H | C₂H₅ | C₂H₅ |
| II.A.1.a.23 | H | C₂H₅ | (CH₂)₂CH₃ |
| II.A.1.a.24 | H | C₂H₅ | CH(CH₃)₂ |
| II.A.1.a.25 | H | C₂H₅ | (CH₂)₃CH₃ |
| II.A.1.a.26 | H | C₂H₅ | CH(CH₃)CH₂CH₃ |
| II.A.1.a.27 | H | C₂H₅ | CH₂CH(CH₃)CH₃ |
| II.A.1.a.28 | H | C₂H₅ | C(CH₃)₃ |
| II.A.1.a.29 | H | C₂H₅ | CH₂CH=CH₂ |
| II.A.1.a.30 | H | C₂H₅ | CH₂-C≡CH |
| II.A.1.a.31 | Cl | H | H |
| II.A.1.a.32 | Cl | H | CH₃ |
| II.A.1.a.33 | Cl | H | C₂H₅ |
| II.A.1.a.34 | Cl | H | (CH₂)₂CH₃ |
| II.A.1.a.35 | Cl | H | CH(CH₃)₂ |
| II.A.1.a.36 | Cl | H | (CH₂)₃CH₃ |
| II.A.1.a.37 | Cl | H | CH(CH₃)CH₂CH₃ |
| II.A.1.a.38 | Cl | H | CH₂CH(CH₃)CH₃ |
| II.A.1.a.39 | Cl | H | C(CH₃)₃ |
| II.A.1.a.40 | Cl | H | CH₂CH=CH₂ |
| II.A.1.a.41 | Cl | H | CH₂-C≡CH |
| II.A.1.a.42 | Cl | CH₃ | CH₃ |
| II.A.1.a.43 | Cl | CH₃ | C₂H₅ |
| II.A.1.a.44 | Cl | CH₃ | (CH₂)₂CH₃ |
| II.A.1.a.45 | Cl | CH₃ | CH(CH₃)₂ |
| II.A.1.a.46 | Cl | CH₃ | (CH₂)₃CH₃ |
| II.A.1.a.47 | Cl | CH₃ | CH(CH₃)CH₂CH₃ |
| II.A.1.a.48 | Cl | CH₃ | CH₂CH(CH₃)CH₃ |
| II.A.1.a.49 | Cl | CH₃ | C(CH₃)₃ |
| II.A.1.a.50 | Cl | CH₃ | CH₂CH=CH₂ |
| II.A.1.a.51 | Cl | CH₃ | CH₂-C≡CH |
| II.A.1.a.52 | Cl | C₂H₅ | C₂H₅ |
| II.A.1.a.53 | Cl | C₂H₅ | (CH₂)₂CH₃ |
| II.A.1.a.54 | Cl | C₂H₅ | CH(CH₃)₂ |
| II.A.1.a.55 | Cl | C₂H₅ | (CH₂)₃CH₃ |
| II.A.1.a.56 | Cl | C₂H₅ | CH(CH₃)CH₂CH₃ |
| II.A.1.a.57 | Cl | C₂H₅ | CH₂CH(CH₃)CH₃ |
| II.A.1.a.58 | Cl | C₂H₅ | C(CH₃)₃ |
| II.A.1.a.59 | Cl | C₂H₅ | CH₂CH=CH₂ |
| II.A.1.a.60 | Cl | C₂H₅ | CH₂-C≡CH |

Ebenso außerordentlich bevorzugt sind die Carbamate der Formel II.A.1.b, insbesondere die Verbindungen der Formel II.A.1.b.1 bis IIA.1.b.60, die sich von den entsprechenden Verbindungen der Formel II.A.1.a.1 bis IIA.1.a.60 dadurch unterscheiden, daß L¹ für Ethoxy steht

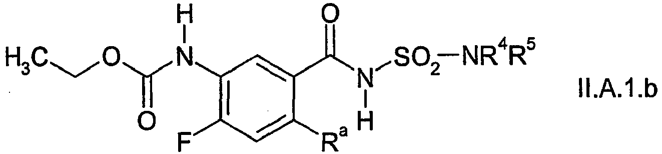

Ebenso außerordentlich bevorzugt sind die Carbamate der Formel II.A.1.c, insbesondere die Verbindungen der Formel II.A.1.c.1 bis II.A.1.c.60, die sich von den entsprechenden Verbindungen der Formel IIA.1.a.1 bis II.A.1.a.60 dadurch unterscheiden, daß L¹ für n-Propyloxy steht.

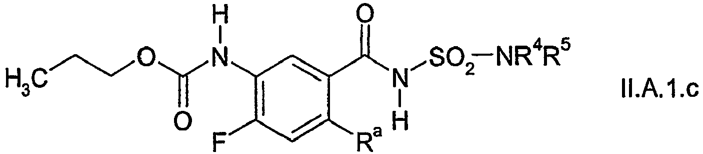

Ebenso außerordentlich bevorzugt sind die Carbamate der Formel II.A.1.d, insbesondere die Verbindungen der Formel II.A.1.d.1 bis II.A.1.d.60, die sich von den entsprechenden Verbindungen der Formel IIA.1.a.1 bis II.A.1.a.60 dadurch unterscheiden, daß L¹ für iso-Propyloxy steht

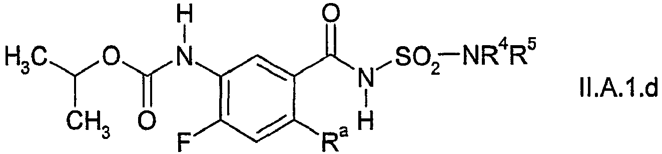

Ebenso außerordentlich bevorzugt sind die Carbamate der Formel II.A.1.e, insbesondere die Verbindungen der Formel IIA.1.e.1 bis II.A.1.e.60, die sich von den entsprechenden Verbindungen der Formel II.A.1.a.1 bis IIA.1.a.60 dadurch unterscheiden, daß L¹ für sec-Butyloxy steht.

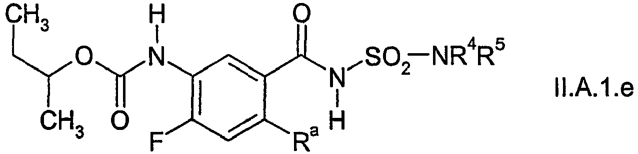

Ebenso außerordentlich bevorzugt sind die Carbamate der Formel II.A.1.f, insbesondere die Verbindungen der Formel II.A.1.f.1 bis IIA.1.f.60, die sich von den entsprechenden Verbindungen der Formel IIA.1.a.1 bis II.A.1.a.60 dadurch unterscheiden, daß L¹ für iso-Butyloxy steht.

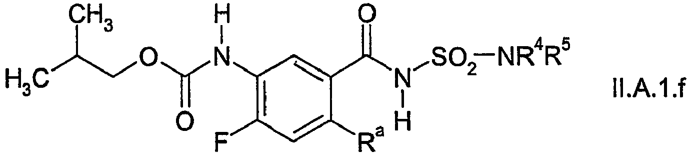

Ebenso außerordentlich bevorzugt sind die Carbamate der Formel II.A.1.g, insbesondere die Verbindungen der Formel II.A.1.g.1 bis II.A.1.g.60, die sich von den entsprechenden Verbindungen der Formel II.A.1.a.1 bis II.A.1.a.60 dadurch unterscheiden, daß L¹ für tert-Butyloxy steht.

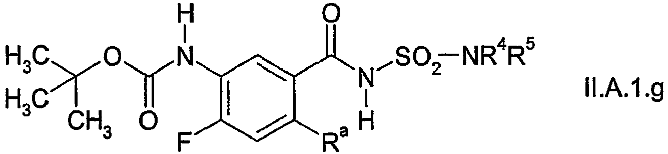

Ebenso außerordentlich bevorzugt sind die Carbamate der Formel II.A.1.h, insbesondere die Verbindungen der Formel II.A.1.h.1 bis IIA.1.h.60, die sich von den entsprechenden Verbindungen der Formel II.A.1.a.1 bis IIA.1.a.60 dadurch unterscheiden, daß L¹ für Methylthio steht.

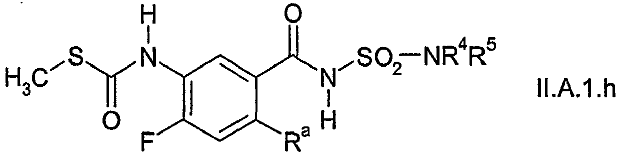

Ebenso außerordentlich bevorzugt sind die Carbamate der Formel II.A.1.i, insbesondere die Verbindungen der Formel II.A.1.i.1 bis II.A.1.i.60, die sich von den entsprechenden Verbindungen der Formel II.A.1.a.1 bis II.A.1.a.60 dadurch unterscheiden, daß L¹ für Ethylthio steht.

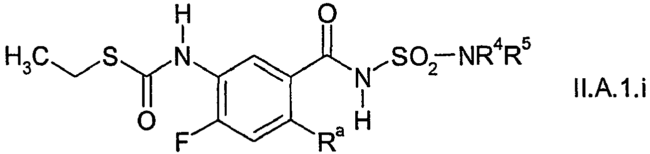

### Synthesebeispiele

Die folgenden Beispiele dienen der Erläuterung der Erfindung.

### 1. Herstellung der Carbamate der Formel II

### Beispiel 1.1:

### N-{4-Fluor-3-[(ethoxycarbonyl)-amino]-benzoyl}-N'-iso-propyl-N'-methylsulfamid (Verbindung II.A.1.b.15)

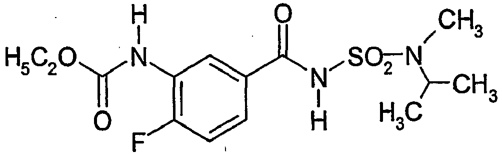

### Beispiel 1.1.a

Zu einer Lösung aus 41,0 g (0,138 mol) N-(4-Fluor-3-amino-benzoyl)-N'-*iso*-propyl-N'-methylsulfamid in Methylenchlorid wurden bei Raumtemperatur 14,13 g (0,179 mol) Pyridin zugetropft und anschließend auf 0-5°C gekühlt. Bei dieser Temperatur wurden 19,83 g (0,183 mol) Ethylchloroformiat in Methylenchlorid portionsweise hinzugefügt und anschließend 60 min gerührt. Die Reaktionsmischung wurde hydrolysiert und die abgetrennte organische Phase mit H₂O sowie 10% Salzsäure extrahiert. Anschließend wurde die organische Phase gewaschen, getrocknet und das Lösungsmittel entfernt Man erhielt 47.9 g (96% der Theorie) der Titelverbindung (Schmp.:142-144 °C) ¹H-NMR (500 MHz, d-DMSO) δ[ppm] = 11.9 (s, 1 H), 9.50 (s, 1 H), 8.30 (d, 1 H), 7.65-7.70 (m, 1 H), 7.35 (t, 1H), 4.10-4.25 (m, 3H), 2.90 (s, 3H), 1.28 (t, 3H), 1.10 (d, 6H).

Analog zu Beispiel 1.1.a wurden die folgenden Beispiele 1.1.b bis 1.1.e durchgeführt:

### Beispiel 1.1.b

6,10 g (0,020 mol) N-(4-Fluor-3-amino-benzoyl)-N'-*iso*-propyl-N'-methylsulfamid
3,87 g (0,041 mol) Picolin
2,97 g (0,027 mol) Ethylchloroformiat
Man erhielt 6,1 g (95% der Theorie) der Titelverbindung.

### Beispiel 1.1.c

6,00 g (0,020 mol) N-(4-Fluor-3-amino-benzoyl)-N'-*iso*-propyl-N'-methylsulfamid
2,47 g (0,062 mol) NaOH
2,97 g (0,027 mol) Ethylchloroformiat
Man erhielt 6,4 g (95% der Theorie) der Titelverbindung.

### Beispiel 1.1.d

6,00 g (0,020 mol) N-(4-Fluor-3-amino-benzoyl)-N'-*iso*-propyl-N'-methylsulfamid
3,72 g (0,027 mol) K₂CO₃
3,00 g (0,027 mol) Ethylchloroformiat
Man erhielt 6,0 g (76% der Theorie) der Titelverbindung.

### Beispiel 1.1.e

5,90 g (0,020 mol) N-(4-Fluor-3-amino-benzoyl)-N'-*iso*-propyl-N'-methylsulfamid
2,47 g (0,062 mol) Triethylamin
0.24 g (0,062 mol) Dimethylaminopyridin (DMAP)
2,89 g (0,027 mol) Ethylchloroformiat
Man erhielt 6,1 g (52% der Theorie) der Titelverbindung.

### Beispiel 1.1.f

Zu einer Lösung von 300,0 g (0,897 mol) N-(4-Fluor-3-amino-benzoyl)-N'-iso-propyl-N'-methylsulfamid in Chlorbenzol wurden bei 115-125°C 134,7 g (1,22 mol) Ethylchloroformiat portionsweise zugefügt und anschließend bei 125°C 2 h gerührt. Danach wurden das Lösungsmittel und der Überschuß Ethylchloroformiat entfernt.

Man erhielt 312,8 g (96% der Theorie) der Titelverbindung.

### Beispiel 1.2

### N-{6-Chlor-4-fluor-3-[(ethoxycarbonyl)-amino]-benzoyl}-N'-iso-propyl-N'-methylsulfamid (Verbindung II.A.1.b.45)

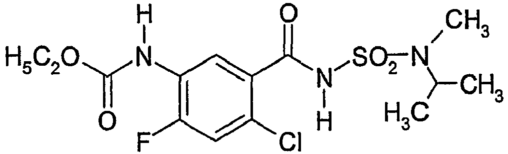

Zu einer Lösung aus 200.0 g (0.565 mol) N-(6-Chlor-4-fluor-3-amino-benzoyl)-N'-*iso*propyl-N'-methylsulfamid in Methylenchlorid wurden bei Raumtemperatur 57.7 g (0.729 mol) Pyridin zugetropft und anschließend auf 0-5°C gekühlt Anschließend gab man portionsweise 80.99 g (0.746 mol) Ethylchloroformiat in Methylenchlorid hinzu und ließ 60 min rühren. Dann wurde die Reaktionsmischung hydrolysiert und die abgetrennte organische Phase mit H₂O sowie 10% Salzsäure extrahiert. Anschließend wurde die organische Phase gewaschen, getrocknet und das Lösungsmittel entfernt.

Man erhielt 156.4 g (86% der Theorie) der Titelverbindung.
¹H-NMR (400 MHz, CDCl₃): δ = 8.70 (s, 1 H), 8.45 (d, 1 H), 7.20 (d, 1 H), 6.90 (s, 1 H) 4.20-4.40 [m, 3 H), 3.00 (s, 3H), 1.35 (t, 3H), 1.20 (d, 6H).

### Beispiel 1.3

### N-{4-Fluor-3-[(methoxycarbonyl)-amino]-benzoyl}-N'-iso-propyl-N'-methylsulfamid (Verbindung II.A.1.a.15)

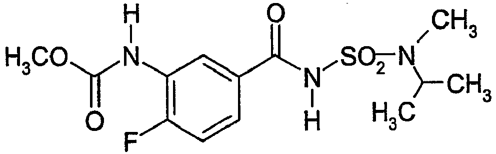

Zu einer Lösung von 12,50 g (0,042 mol) N-(4-Fluor-3-amino-benzoyl)-N'-*iso*-propyl-N'-methylsulfamid in Chlorbenzol wurden bei 115-125°C 5,52 g (0,057 mol) Methylchloroformiat portionsweise zugefügt und anschließend bei 125°C 2 h gerührt. Danach wurden das Lösungsmittel und der Überschuß Methylchloroformiat entfernt.

Man erhielt 14,9 g (99% der Theorie) der Titelverbindung.
¹H-NMR (500MHz, CDCl₃): δ = 9.25 (s, 1 H), 8.55 (d, 1 H), 7.55-7.60 (m, 1 H), 7.15 (t, 1 H), 6.95 (s, 1 H), 4.20-4.30 (m, 1 H), 3.00 (s, 3 H), 2.95 (s, 3 H), 1.18 ppm (d, 6 H).

### Beispiel 1.4

### N-{4-Fluor-3-[(phenoxycarbonyl)-amino]-benzoyl}-N'-iso-propyl-N'-methylsulfamid

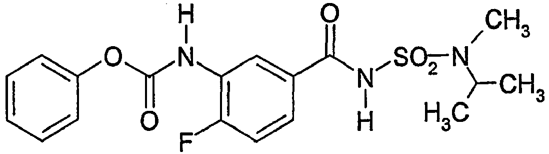

Zu einer Lösung von 12,50 g (0,042 mol) N-(4-Fluor-3-amino-benzoyl)-N'-*iso*-propyl-N'-methylsulfamid in Chlorbenzol wurden bei 115-125°C 6,69 g (0,057 mol) Phenylchloroformiat portionsweise zugefügt und anschließend bei 125°C 2 h gerührt. Danach wurden das Lösungsmittel und der Überschuß Phenylchloroformiat entfernt.

Man erhielt 17,7 g (98% der Theorie) der Titelverbindung.
¹H-NMR (500MHz, CDCl₃): δ = 9.05 (s, 1 H), 8.55 (d, 1 H), 7.55-7.60 (m, 1 H), 7.25-7.40 (m, 7 H), 4.20-4.30 (m, 1 H), 3.00 (s, 3 H), 1.20 ppm (d, 6 H).

### Beispiel 1.5

### N-{4-Fluor-3-[(n-butyloxycarbonyl)-amino]-benzoyl)-N'-iso-propyl-N'-methylsulfamid

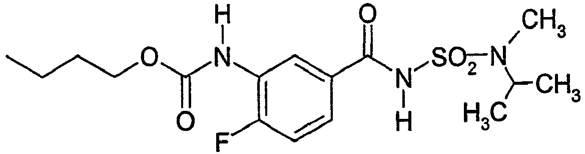

Zu einer Lösung von 12,50 g (0,042 mol) N-(4-Fluor-3-amino-benzoyl)-N'-iso-propyl-N'-methylsulfamid in Chlorbenzol wurden bei 115-125°C 7,36 g (0,053 mol) n-Butylchloroformiat portionsweise zugefügt und anschließend bei 125°C 2 h gerührt Danach wurden das Lösungsmittel und der Überschuß *n*-Butylchloroformiat entfernt.

Man erhielt 18,0 g (93% der Theorie) der Titelverbindung.
¹H-NMR (500MHz, CDCl₃): δ = 9.15 (s, 1 H), 8.55 (d, 1 H), 7.55-7.60 (m, 1 H), 7.15 (t, 1 H), 6.95 (s, 1 H), 4.20-4.35 (m, 3 H), 3.00 (s, 3 H), 2.95 (s, 3 H), 1,60 (q, 2 H), 1,35 (q, 2H), 1.18 ppm (6 H), 0.95 (t, 3 H).

### Beispiel 1.6

### N-{4-Fluor-3-[(iso-propyloxycarbonyl)-amino]-benzoyl}-N'-iso-propyl-N'-methylsulfamid (Verbindung II.A.1.d.15)

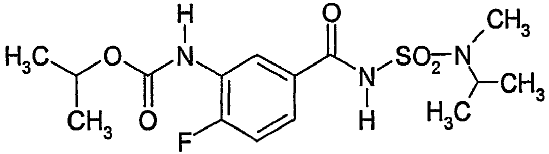

Zu einer Lösung von 12,50 g (0,042 mol) N-(4-Fluor-3-amino-benzoyl)-N'-*iso*-propyl-N'-methylsulfamid in Toluol wurden bei 115-125°C 83 g einer 1M-LÖsung von *iso-*Propylchloroformiat in Toluol (entspricht 0,097 mol *iso*-Propylchloroformiat) portionsweise zugefügt und anschließend bei 125°C 2 h gerührt. Danach wurden das Lösungsmittel und der Überschuß *iso*-Propylchloroformiat entfernt.

Man erhielt 16,1 g (97% der Theorie) der Titelverbindung.
¹H-NMR (500MHz, CDCl₃): δ = 9.10 (s, 1 H), 8.55 (d, 1 H), 7.55-7.60 (m, 1 H), 7.10 (t, 1 H), 6.90 (s, 1 H), 4.95-5.10 (m, 1 H), 4.20-4.30 (m, 1 H), 2.95 (s, 3 H), 1.35 (d, 6 H), 1.18 ppm (d, 6 H).

### 2. Herstellung der chlorierten Carbamate der Formel II

### Beispiel 2.1:

### N-{2-Chlor-4-fluor-5-[(ethoxycarbonyl)amino]-benzoyl}-N'-iso-propyl-N'-methylsulfamid (Verbindung II.A.1.b.45)

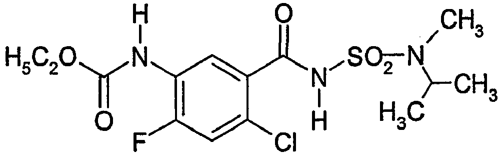

### Beispiel 2.1.a

150 g (0,407 mol) N-(3-[(Ethoxycarbonyl)amino]-4-fluor-benzoyl)-N'-iso-propyl-N'-methylsulfamid suspendiert in Chlorbenzol wurden in einem Reaktionsgefäß unter Vakuum aufkonzentriert (Innentemperatur < 100°C). Danach wurde das Vakuum gebrochen und die Suspension auf 65 °C abgekühlt. Anschließend wurden 760 g (5,517 mol) Sulfurylchlorid portionsweise zugegeben, wobei die Temperatur auf 50 °C gehalten wurde. Anschließend wurde 16 h nachgeführt.

Dann entfernte man überschüssiges Sulfurylchlorid destillativ. Der Destillationsrückstand wurde mit Chlorbenzol sowie Wasser versetzt , auf Raumtemperatur abgekühlt und der pH-Wert der so erhaltenen Suspension mit 2N Natronlauge auf pH = 5 eingestellt. Anschließend wurde das Produkt abfiltriert, gewaschen und getrocknet.

Man erhielt 126,5 g (75% der Theorie) der Titelverbindung (Schmp.: 98-100 °C).

### Beispiel 2.1.b

40 g (0,11 mol) N-{3-[(Ethoxycarbonyl)amino]-4-fluor-benzoyl}-N'-iso-propyl-N'-methylsulfamid wurden zu 179,3 g (1,33 mol) Sulfurylchlorid gegeben und 10 h bei 40 °C gerührt. Überschüssiges Sulfurylchlorid und Chlorbenzol wurden anschließend destillativ entfernt.Man versetzte den Rückstand bei 75 °C unter Rühren zunächst mit Toluol, dann mit Wasser. Bei 80 - 85 °C wurde der pH-Wert mittels 2 N Natronlauge auf pH = 4 eingestellt und der Ansatz unter Rühren auf 20 °C abgekühlt. Das ausgefallene Produkt wurde abfiltriert, gewaschen und getrocknet

Man erhielt 33 g (71 % der Theorie) der Titelverbindung (Schmp.: 98-100 °C).

### Beispiel 2.2

### N-{2-Chlor-4-fluor-5-[(methoxycarbonyl)amino]-benzoyl}-N'-iso-propyl-N'-methylsulfamid (Verbindung IIA.1.b.45)

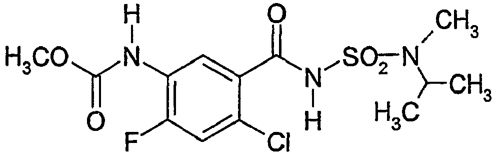

40 g (0,112 mol) N-(3-[(Methoxycarbonyl)amino]-4-fluor-benzoyl)-N'-iso-propyl-N'-methylsulfamid suspendiert in Chlorbenzol wurden in einem Reaktionsgefäß unter Vakuum aufkonzentriert. Danach wurde das Vakuum gebrochen und die Suspension auf 65 °C abgekühlt. Anschließend wurden 197 g (1,430 mol) Sulfurylchlorid portionsweise zugegeben, wobei die Temperatur auf 50 °C gehalten wurde. Anschließend wurde 16 h nachgerührt.

Dann entfernte man überschüssiges Sulfurylchlorid destillativ. Der Destillationsrückstand wurde mit Chlorbenzol sowie Wasser versetzt, auf Raumtemperatur abgekühlt und der pH-Wert der so erhaltenen Suspension mit 2N Natronlauge auf pH = 5 eingestellt. Anschließend wurde das Produkt abfiltriert, gewaschen und getrocknet.

Man erhielt 14,6 g (34% der Theorie) der Titelverbindung.
¹H-NMR (500MHz, d-DMSO): δ = 11.1 (s, 1 H), 9.80 (s, 1 H), 7.90-7.95 (m, 1 H), 7.60-7.70 (m, 1 H), 4.25-4.30 (m, 1 H), 3.80 (s, 3 H), 2.85 (s, 3 H), 1.15 (d, 6 H).

### 3. Herstellung der Phenyl(thio)uracile und -dithiouracile der Formel I

### Beispiel 3.1:

### 2-Chlor-5-[3,6-dihydro-2,6-dioxo-4-(trifluormethyl)-1(2H)-pyrimidinyl]-4-fluor-N-{(methyl(1-methylethyl)amino]sulfonyl}-benzamid

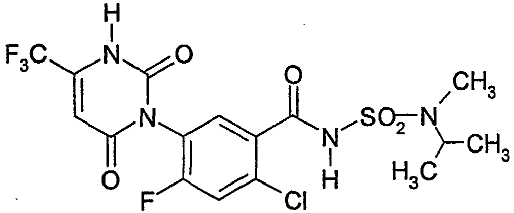

### Beispiel 3.1.a

9,13 g (0,049 mol) 3-Amino-4,4,4-trifluor-2-butensäureethylester wurden in DMF bei Raumtemperatur vorgelegt. Man gab 12,84 g (0,059 mol) Kaliummethylatlösung (32 %ig in Methanol) zu und ließ 30 min nachrühren. Anschließend wurden 20 g (0,049 mol) N-(2-Chlor-4-fluor-5-[(ethoxycarbonyl)amino]-benzoyl)-N'-*iso*-propyl-N'-methylsulfamid zugegeben. Die Reaktionsmischung wurde erwärmt und soviel Alkohol abdestilliert, bis 119 °C erreicht waren. Dann gab man unter Abdestillation von Alkohol in Portionen innerhalb von einigen Stunden 11,75 g (0,054 mol) Kaliummethylatlösung (32 % in Methanol) zu. Zur Aufarbeitung tropfte man die Reaktionsmischung unter Kühlung in verdünnte Salzsäure , wobei am Ende der pH < 2 war. Das ausgefallene Produkt wurde abfiltriert, gewaschen und getrocknet.

Man erhielt 22,5 g (92,7 % der Theorie) der Titelverbindung [Schmp. 238 °C (Zersetzung)].

### Beispiel 3.1.b

1,2 g (6,8 mmol), 3-Amino-4,4,4-trifluor-2-butensäureethylester wurden in DMF bei Raumtemperatur vorgelegt Man gab 1,9 g (13,7 mmol) Kaliumcarbonat zu und ließ 1 h bei 50 °C nachrühren. Anschließend wurden 2,4 g (5,7 mmol) N-(2-Chlor-4-fluor-5-[(ethoxycarbonyl)amino]-benzoyl)-N'-*iso*-propyl-N'-methylsulfamid zugegeben, die Temperatur auf 120 °C erhöht und 4,5 h nachgerührt. Zur Aufarbeitung wurde die Reaktionsmischung unter Kühlung in verdünnte Salzsäure getropft, wobei am Ende der pH < 2 war. Das ausgefallene Produkt wurde abfiltriert, gewaschen und getrocknet. Man erhielt 2,3 g (73 % der Theorie) der Titelverbindung.

### Beispiel 3.2:

### 2-Chlor-5-[3,6-dihydro-3-methyl-2,6-dioxo-4-(trifluormethyl)-1(2H)-pyrimidinyl]-4-fluor-N-[methyl-(1-methylethyl)amino]sulfonyl]-benzamid

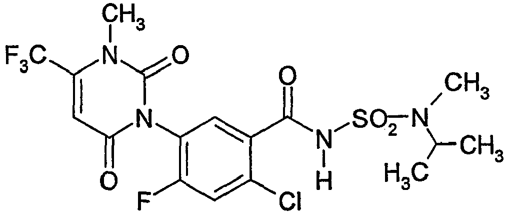

Zu 2,0 g (4,11 mmol) 2-Chlor-5-[3,6-dihydro-2,6-dioxo-4-(trifluoromethyl)-1(2H)-pyrimidinyl]-4-fluor-N-[[methyl(1-methylethyl)amino]sulfonyl]benzamid aus Beispiel 1 in 80 ml N,N-Dimethylformamid gab man 1,14 g (9,04 mmol) Dimethylsulfat und 0,283 g (2,055 mmol) K₂CO₃ und rührte danach 16 Stunden bei 25°C. Anschließend destillierte man bei 30 °C und reduziertem Druck das N,N-Dimethylformamid ab und nahm den Rückstand in etwa 250 ml Ethylacetat auf. Man säuerte das Reaktionsgemisch mit 10 %iger HCl an und extrahierte danach zweimal mit Wasser. Man trocknete die organische Phase über MgSO₄ und destillierte das Lösungsmittel ab, wobei man 1,95 g des rohen Produktes erhielt. Die Reinheit des Wertproduktes betrug laut ¹H-NMR und HPLC 77 % (entspricht einer Ausbeute von 73%). Zur Reinigung chromatographierte man 0,92 g dieses rohen Produktes an Kieselgel (Säule 28 x 4,5 cm) mit Cyclohexan/Essigester 9/1 bis 1/1, wobei man vier Fraktionen erhielt. Die 3. Fraktion (0,58 g; entspricht 59% isolierter Ausbeute) enthielt das gewünschte Wertprodukt in reiner Form.
¹H-NMR-Daten (DMSO-d₆) δ(ppm) : 12,2 (NH), 7,8 (d, 1 H), 7,7 (d, 1 H), 6,6 (s, 1 H), 4,1 (sept, 1 H), 3,5 (s, 3 H), 3,3 (s, 3 H), 2,9 (s, 3 H), 1,2 (d, 6 H)

### Beispiel 3.3:

### 2-Chlor-5-[3,6-dihydro-3-methyl-2,6-dioxo-4-(trifluormethyl)-1(2H)-pyrimidinyl]-4-fluor-N-[[methyl-(1-methylethyl)amino]sulfonyl]-benzamid

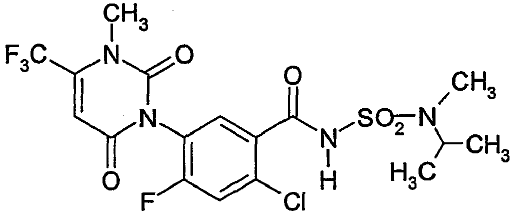

Zu einem Lösungsmittelgemisch aus 135 g Toluol und 27 g Tetrahydrofuran gab man bei 25 °C 12,45 g (0,024 mol) 2-Chlor-5-[3,6-dihydro-2,6-dioxo-4-(trifluormethyl)-1(2H)-pyrimidinyl]-4-fluor-N-[[methyl(1-methylethyl)amino]sulfonyl]benzamid (93,9%ig) aus Beispiel 1 und versetzte danach das Gemisch mit einer Lösung aus 2,3 g (0,0288 mol) Natriumhydroxid (50%ig) in 57,5 g Wasser. Zu dem Reaktionsgemisch gab man 0,77 g (0,0024 mol) Tetrabutylammoniumbromid und 3,69 g (0,0293 mol) Dimethylsulfat zu.. Das zweiphasige Reaktionsgemisch rührte man 23 Stunden intensiv bei 25°C.

Danach trennte man die wässrige Phase ab und wusch die organische Phase zweimal mit jeweils 100 ml Wasser. Nach dem Trocknen der vereinten organischen Phase destillierte man das Lösungsmittel bei reduziertem Druck ab, wobei man 13,8 g eines rohen Produktes erhielt, welches nach quantitativer HPLC die Titelverbindung zu 77,5% enthielt (entspricht einer Ausbeute von 88,9%).

### Beispiel 3.4:

### 2-Chlor-5-[3,6-dihydro-3-methyl-2,6-dioxo-4-(trifluormethyl)-1(2H)-pyrimidinyl]-4-fluor-N-[[methyl-(1-methylethyl)amino]sulfonyl]-benzamid

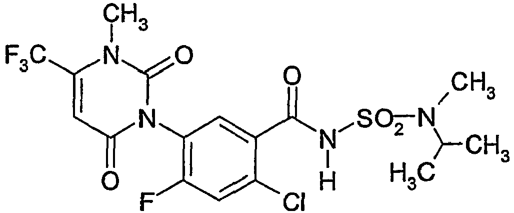

Zu einem Lösungsmittelgemisch aus 250 ml Dichlormethan und 125 ml Tetrahydrofuran gab man 5 g (10,3 mmol) 2-Chlor-5-[3,6-dihydro-2,6-dioxo-4-(trifluoromethyl)-1(2H)-pyrimidinyl]-4-fluor-N-[[methyl(1-methylethyl)amino]sulfonyl]benzamid aus Beispiel 1 und versetzte danach mit einer Lösung von 0,411 g (10,3 mmol) NaOH in 375 ml Wasser. Zu dem Reaktionsgemisch gab man 0,38 g (1,03 mmol) Tetrabutylammoniumiodid und 1,36 g (10,8 mmol) Dimethylsulfat und rührte das Zwei-Phasen-Gemisch 14 Stunden mit 1000 Umdrehungen/min.

Man trennte die wässrige Phase ab und engte die organische Phase unter vermindertem Druck bis zur Trockne ein. Die chromatographische Reinigung an Kieselgel erfolgte auf die in Beispiel 5 beschriebene Weise, wobei man 4 Fraktionen erhielt. Nach Entfernen des Lösungsmittels enthielt die erste Fraktion 0,54 g eines Gemisches, das gemäß ¹H-MNR zu 90 % aus dem gewünschten Wertprodukt bestand und die zweite Fraktion 2 enthielt 2,4 g des Wertprodukts mit einer Reinheit > 95 % (Ausbeute bezogen auf beide Fraktionen: 56%).

## Patentansprüche

1. Verfahren zur Herstellung von 3-Phenyl(thio)uracilen und dithiouracilen der Formel I 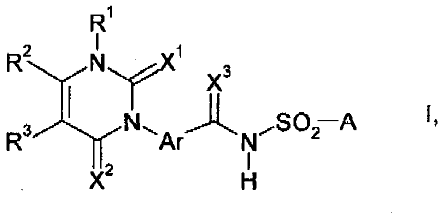 worin die Variablen die folgenden Bedeutungen haben:
R¹ Wasserstoff, C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₈-Halogenalkinyl, Phenyl-C₁-C₄-alkyl oder Amino;
R² und R³ unabhängig voneinander Wasserstoff, C₁-C₆-Alkyl, C₁-C₆-Halogenalkyl, C₃-C₈-Cycloalkyl, C₂-C₆-Alkenyl, C₂-C₆-Halogenalkenyl, C₃-C₆-Alkinyl oder C₃-C₆-Halogenalkinyl;
X¹, X² und X³ unabhängig voneinander Sauerstoff oder Schwefel;
Ar Phenyl, das partiell oder vollständig halogeniert sein kann und/oder ein bis drei Reste aus der Gruppe Cyano, C₁-C₄-Alkyl oder C₁-C₄-Halogenalkyl tragen kann, und
A NR⁴R⁵, worin R⁴ und R⁵ unabhängig voneinander Wasserstoff oder C₁-C₆-Alkyl bedeuten;
**dadurch gekennzeichnet, dass** Carbamate der Formel II 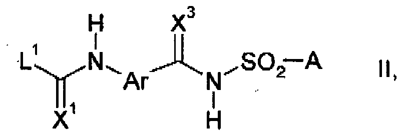 wobei die Variablen X¹, X³, Ar und A die zuvor genannten Bedeutungen haben und L¹ für C₁-C₆-Alkoxy oder C₁-C₆-Alkylthio steht,
mit Enaminen der Formel III 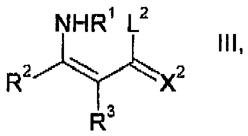 wobei die Variablen X², R¹, R² und R³ die zuvor genannten Bedeutungen haben und L² für C₁-C₆-Alkoxy, C₁-C₆-Halogenalkoxy, C₁-C₄-Alkoxy-C₂-C₄-alkoxy, C₁-C₄-Alkylthio-C₂C₄-alkoxy, C₂-C₆-Alkenyloxy, C₂-C₆-Halogen-alkenyloxy, C₃-C₆-Alkinyloxy, C₃-C₆-Halogenalkinyloxy, C₃-C₈-Cycloalkyloxy, C₁-C₆-Cyanoalkoxy oder Benzyloxy, das seiner-seits am Phenylring partiell oder vollständig halogeniert sein kann und/oder durch ein bis drei Reste aus der Gruppe Cyano, Nitro, C₁-C₄-Alkyl, C₁-C₄-Alkoxy und C₁-C₄-Alkylthio substituiert sein kann;
steht,
in Gegenwart von 2,2 bis 2,4 Äquivalenten Base bezogen auf das Carbamat der Formel **II** umgesetzt werden.

2. Verfahren zur Herstellung von 3-Phenyl(thio)uracilen und -dithiouracilen der Formel I gemäß Anspruch 1, **dadurch gekennzeichnet, dass** R¹ für Wasserstoff oder C₁-C₄-Alkyl steht.

3. Verfahren zur Herstellung von 3-Phenyl(thio)uracilen und -dithiouracilen der Formel I gemäß Ansprüchen 1 und 2, **dadurch gekennzeichnet, dass** R² für Wasserstoff, C₁-C₄-Alkyl oder C₁-C₄-Halogenalkyl steht.

4. Verfahren zur Herstellung von 3-Phenyl(thio)uracilen und -dithiouracilen der Formel I gemäß Ansprüchen 1 bis 3, **dadurch gekennzeichnet, dass** R³ für Wasserstoff oder C₁-C₄-Alkyl steht.

5. Verfahren zur Herstellung von 3-Phenyl(thio)uracilen und -dithiouracilen der Formel I gemäß Ansprüchen 1 bis 4, **dadurch gekennzeichnet, dass** X¹, X² und X³ für Sauerstoff stehen.

6. Verfahren gemäß Ansprüchen 1 bis 5, **dadurch gekennzeichnet, dass** Ar für eine Gruppe der allgemeinen Formel Ar-1 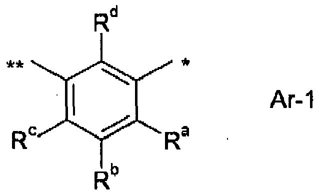 steht, wobei
* die Verknüpfung von Ar mit der C(X³)-Gruppe;
** die Verknüpfung von Ar mit dem direkt benachbarten Stickstoffatom kennzeichnet;
R^{a} und R^{c} unabhängig voneinander Wasserstoff, Halogen, Cyano oder C₁-C₄-Halogenalkyl; und
R^{b} und R^{d} Wasserstoff
bedeuten.

7. Verfahren gemäß Ansprüchen 1 bis 6, **dadurch gekennzeichnet, dass** die Base über einen Zeitraum versetzt zugegeben wird.

8. Verfahren zur Herstellung von 3-Phenyl(thio)uracilen und -dithiouracilen der Formel I gemäß Ansprüchen 1 bis 7, **dadurch gekennzeichnet, dass** die Carbamate der Formel II durch Umsetzung von Aminen der Formel IV 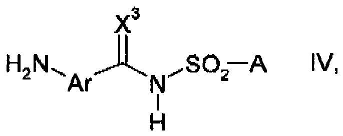 wobei X³, Ar und A die unter Anspruch 1 genannten Bedeutungen haben,
mit Verbindungen der Formel V 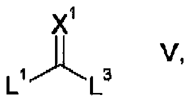 wobei X¹ und L¹ die unter Anspruch 1 genannten Bedeutungen haben und L³ für Chlor oder C₁-C₆-Alkoxy steht, hergestellt werden.

9. Verfahren zur Herstellung von 3-Phenyl(thio)uracilen und -dithiouracilen der Formel I gemäß Ansprüchen 1 bis 8, **dadurch gekennzeichnet, dass** die Carbamate der Formel II in einem Zwischenschritt mit einem Halogenierungsmittel am Rest Ar halogeniert werden.

10. Carbamate der Formel II 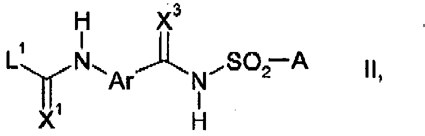 wobei X¹, X³, Ar, A und L¹ die unter Anspruch 1 genannten Bedeutungen haben.

11. Verfahren zur Herstellung von 3-Phenyl(thio)uracilen und -dithiouracilen der Formel I gemäß Anspruch 1 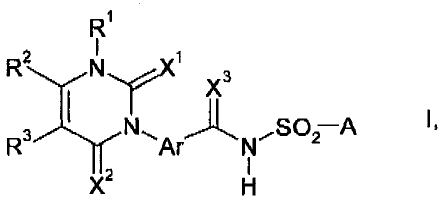 worin R¹ die folgenden Bedeutungen hat:
R¹ C₁-C₆-Alkyl, C₁-C₄-Cyanoalkyl, C₁-C₆-Halogenalkyl, C₃-C₈-Cycloalkyl, C₂-C₆-Alkenyl, C₂-C₆-Halogenalkenyl, C₃-C₆-Alkinyl, C₃-C₆-Haogenalkinyl, Phenyl-C₁-C₄-alkyl oder Amino;
und die Variablen R², R³, X¹, X², X³, Ar und A die in Ansprüchen 1 bis 7 genannten Bedeutungen haben,
**dadurch gekennzeichnet, dass** Carbamate der Formel II 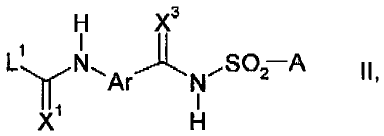 wobei die Variablen X¹, X³, Ar und A die zuvor genannten Bedeutungen haben und L¹ für C₁-C₆-Alkoxy oder C₁-C₆-Alkylthio steht,
mit Enaminen der Formel III 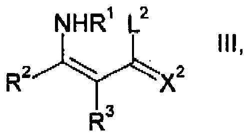 wobei R¹ für Wasserstoff steht, die Variablen X² , R², R³ und L² die zuvor genannten Bedeutungen haben,
umgesetzt werden; und das entstandene 3-Phenyl(thio)uracil und -dithioracil der Formel I, wobei R¹ für Wasserstoff steht, anschließend
- mit einem Alkylierungsmittel der Formel VI
R¹-L⁴ VI,
worin
R¹ C₁-C₆-Alkyl, C₁-C₄-Cyanoalkyl, C₁-C₆-Halogenalkyl, C₃-C₆-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl; und
L⁴ Halogen, Hydrogensulfat, C₁-C₆-Alkylsulfat, Sulfat, C₁-C₆-Alkylsulfonyloxy, C₁-C₆-Halogenalkylsulfonyloxy oder Phenylsulfonyloxy, wobei der Phenylring gegebenenfalls mit Halogen, Nitro, C₁-C₆-Alkyl oder C₁-C₆-Halogenalkyl ein- oder mehrfach substituiert ist;
bedeutet
zu 3-Phenyl(thio)uracilen und -dithloracilen der Formel I worin R¹ für C₁-C₆-Alkyl, C₁-C₄-Cyanoalkyl, C₁-C₆-Halogenalkyl, C₃-C₆-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl, steht, alkyliert;
oder
- mit einem Aminierungsmittel der Formel VII
H₂N-L⁵ VII,
worin L⁵ für Halogen, Hydrogensulfat, C₁-C₆-Alkylsulfonyloxy, C₁-C₆-Halogenalkylsulfonyloxy, Phenylsulfonyloxy oder Phenyloxy,
wobei der Phenylring gegebenenfalls mit Halogen, Nitro, C₁-C₆-Alkyl oder C₁-C₆-Halogenalkyl ein- oder mehrfach substitutiert ist, steht,
zu 3-Phenyl(thio)uracilen und -dithioracilen der Formel I, worin R¹ für NH₂ steht, aminiert.

12. Verfahren zur Herstellung von 3-Phenyl(thio)uracilen und -dithiouracilen der Formel I gemäß Anspruch 11, wobei R¹ für C₁-C₆-Alkyl oder Amino steht.

13. Verfahren zur Herstellung von 3-Phenyl(thio)uracilen und -dithiouracilen der Formel I gemäß Anspruch 11, wobei R¹ für C₁-C₆-Alkyl steht.

## Claims

1. A process for preparing 3-phenyl(thio)uracils and -dithiouracils of the formula I 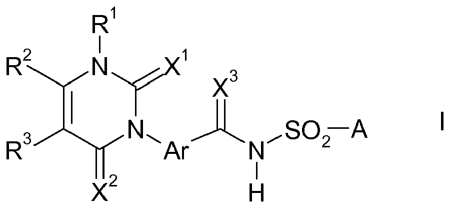 where the variables are each defined as follows:
R¹ is hydrogen, C₁-C₆-alkyl, C₁-C₄-cyanoalkyl, C₁-C₆-haloalkyl, C₃-C₈-cycloalkyl, C₂-C₆-alkenyl, C₂-C₆-haloalkenyl, C₃-C₆-alkynyl, C₃-C₆-haloalkynyl, phenyl-C₁-C₄-alkyl or amino,
R² and R³ are each independently hydrogen, C₁-C₆-alkyl, C₁-C₆-haloalkyl, C₃-C₈-cycloalkyl, C₂-C₆-alkenyl, C₂-C₆-haloalkenyl, C₃-C₆-alkynyl or C₃-C₆-haloalkynyl;
X¹, X² and X³ are each independently oxygen or sulfur;
Ar is phenyl which may be partly or fully halogenated and/or may carry from one to three radicals from the group of cyano, C₁-C₄-alkyl or C₁-C₄-haloalkyl, and
A is NR⁴R⁵ where R⁴ and R⁵ are each independently hydrogen or C₁-C₆-alkyl;
which comprises reacting carbamates of the formula II 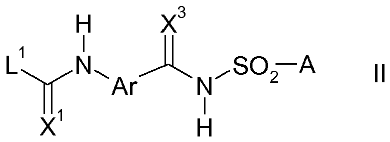 where the variables X¹, X³, Ar and A are each as defined above and L¹ is C₁-C₆-alkoxy or C₁-C₆-alkylthio
with enamines of the formula III 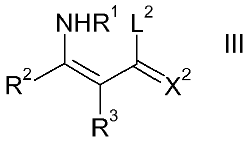 where the variables X², R¹, R² and R³ are each as defined above and L² is C₁-C₆-alkoxy, C₁-C₆-haloalkoxy, C₁-C₄-alkoxy-C₂-C₄-alkoxy,
C₁-C₄-alkylthio-C₂C₄-alkoxy, C₂-C₆-alkenyloxy, C₂-C₆-haloalkenyl-oxy, C₃-C₆-alkynyloxy, C₃-C₆-haloalkynyloxy, C₃-C₈-cycloalkyloxy, C₁-C₆-cyanoalkoxy or benzyloxy which in turn may be partially or fully halogenated on the phenyl ring and/or may be substituted by from one to three radicals from the group of cyano, nitro, C₁-C₄-alkyl, C₁-C₄-alkoxy and C₁-C₄-alkylthio;
in the presence of from 2.2 to 2.4 equivalents of base, based on the carbamate of the formula II.

2. The process for preparing 3-phenyl(thio)uracils and -dithiouracils of the formula I according to claim 1, wherein R¹ is hydrogen or C₁-C₄-alkyl.

3. The process for preparing 3-phenyl(thio)uracils and -dithiouracils of the formula I according to claims 1 and 2, wherein R² is hydrogen, C₁-C₄-alkyl or C₁-C₄-haloalkyl.

4. The process for preparing 3-phenyl(thio)uracils and -dithiouracils of the formula I according to claims 1 to 3, wherein R³ is hydrogen or C₁-C₄-alkyl.

5. The process for preparing 3-phenyl(thio)uracils and -dithiouracils of the formula I according to claims 1 to 4, wherein X¹, X² and X³ are each oxygen.

6. The process according to claims 1 to 5, wherein Ar is a group of the general
formula Ar-1 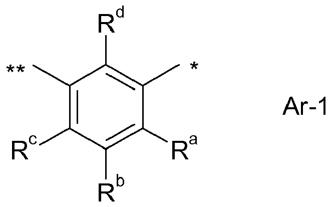 where
* represents the bond of Ar to the C(X³) group;
** represents the bond of Ar to the directly adjacent nitrogen atom; R^{a} and R^{c} are each independently hydrogen, halogen, cyano or C₁-C₄-haloalkyl; and
R^{b} and R^{d} are each hydrogen.

7. The process according to claims 1 to 6, wherein the base is added offset over a period of time.

8. The process for preparing 3-phenyl(thio)uracils and -dithiouracils of the formula I according to claims 1 to 7, wherein the carbamates of the formula II are prepared by reacting amines of the formula IV 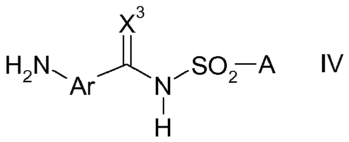 where X³, Ar and A are each as defined under claim 1
with compounds of the formula V 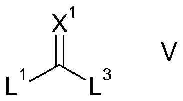 where
X¹ and L¹ are each as defined under claim 1 and
L³ is chlorine or C₁-C₆-alkoxy.

9. The process for preparing 3-phenyl(thio)uracils and -dithiouracils of the formula I according to claims 1 to 8, wherein the carbamates of the formula II are halogenated on the Ar radical with a halogenating agent in an intermediate step.

10. A carbamate of the formula II 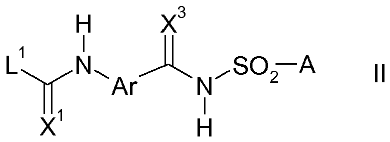 where X¹, X³, Ar, A and L¹ are each as defined under claim 1.

11. A process for preparing 3-phenyl(thio)uracils and -dithiouracils of the formula I according to claim 1 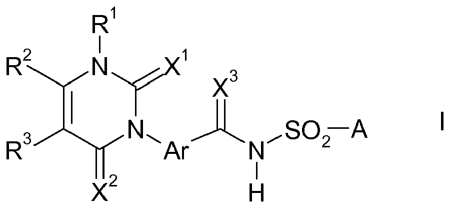 where R¹ is defined as follows:
R¹ is C₁-C₆-alkyl, C₁-C₄-cyanoalkyl, C₁-C₆-haloalkyl, C₃-C₈-cycloalkyl, C₂-C₆-alkenyl, C₂-C₆-haloalkenyl, C₃-C₆-alkynyl, C₃-C₆-haloalkynyl, phenyl-C₁-C₄-alkyl or amino;
and the variables R², R³, X¹, X², X³, Ar and A are each as defined in claims 1 to 7,
which comprises reacting carbamates of the formula II 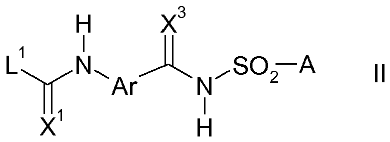 where the variables X¹, X³, Ar and A are each as defined above and L¹ is C₁-C₆-alkoxy or C₁-C₆-alkylthio
with enamines of the formula III 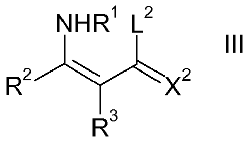 where R¹ is hydrogen, the variables X², R², R³ and L² are each as defined above; and then reacting the resulting 3-phenyl(thio)uracil and -dithiouracil of the formula I where R¹ is hydrogen,
- with an alkylating agent of the formula VI
R¹-L⁴ VI
where
R¹ is C₁-C₆-alkyl, C₁-C₄-cyanoalkyl, C₁-C₆-haloalkyl, C₃-C₈-cycloalkyl, C₂-C₆-alkenyl, C₂-C₆-haloalkenyl, C₃-C₆-alkynyl, C₃-C₆-haloalkynyl or phenyl-C₁-C₄-alkyl; and
L⁴ is halogen, hydrogensulfate, C₁-C₆-alkylsulfate, sulfate, C₁-C₆-alkylsulfonyloxy, C₁-C₆-haloalkylsulfonyloxy or phenylsulfonyloxy where the phenyl ring is optionally mono- or polysubstituted by halogen, nitro, C₁-C₆-alkyl or C₁-C₆-haloalkyl;
to give 3-phenyl(thio) uracils and -dithiouracils of the formula I where R¹ is C₁-C₆-alkyl, C₁-C₄-cyanoalkyl, C₁-C₆-haloalkyl, C₃-C₈-cycloalkyl, C₂-C₆-alkenyl, C₂-C₆-haloalkenyl, C₃-C₆-alkynyl, C₃-C₆-haloalkynyl or phenyl-C₁-C₄-alkyl;
or
- with an aminating agent of the formula VII
H₂N-L⁵ VII
where L⁵ is halogen, hydrogensulfate, C₁-C₆-alkylsulfonyloxy, C₁-C₆-haloalkylsulfonyloxy, phenylsulfonyloxy or phenyloxy, where the phenyl ring is optionally mono- or polysubstituted by halogen, nitro, C₁-C₆-alkyl or C₁-C₆-haloalkyl
to give 3-phenyl(thio)uracils and -dithiouracils of the formula I where R¹ is NH₂.

12. A process for preparing 3-phenyl(thio)uracils and -dithiouracils of the formula I according to claim 11 where R¹ is C₁-C₆-alkyl or amino.

13. A process for preparing 3-phenyl(thio)uracils and -dithiouracils of the formula I according to claim 11 where R¹ is C₁-C₆-alkyl.

## Revendications

1. Procédé de fabrication de 3-phényl(thio)uraciles et -dithiouraciles de formule I 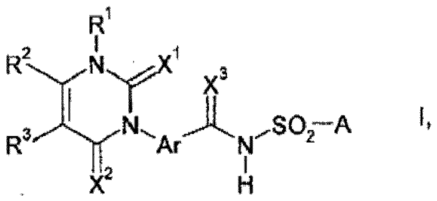 dans laquelle les variables ont les significations suivantes :
R¹ hydrogène, alkyle en C₁-C₆, cyanoalkyle en C₁-C₄, halogénoalkyle en C₁-C₆, cycloalkyle en C₃-C₈, alcényle en C₂-C₆, halogénoalcényle en C₂-C₆, alcynyle en C₃-C₆, halogénoalcynyle en C₃-C₆, phényl-alkyle en C₁-C₄ ou amino ;
R² et R³ indépendamment l'un de l'autre hydrogène, alkyle en C₁-C₆, halogénoalkyle en C₁-C₆, cycloalkyle en C₃-C₈, alcényle en C₂-C₆, halogénoalcényle en C₂-C₆, alcynyle en C₃-C₆ ou halogénoalcynyle en C₃-C₆ ;
X¹, X² et X³ indépendamment les uns des autres oxygène ou soufre ;
Ar phényle, qui peut être halogéné en partie ou en totalité et/ou qui peut porter un à trois radicaux du groupe constitué par cyano, alkyle en C₁-C₄ ou halogénoalkyle en C₁-C₄, et
A NR⁴R⁵, R⁴ et R⁵ signifiant indépendamment l'un de l'autre hydrogène ou alkyle en C₁-C₆ ;
**caractérisé en ce que** des carbamates de formule II 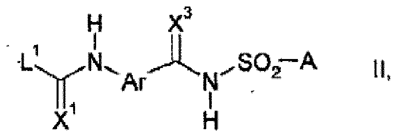 dans laquelle les variables X¹, X³, Ar et A ont les significations indiquées précédemment et L¹ représente alcoxy en C₁-C₆ ou alkylthio en C₁-C₆,
sont mis en réaction avec des énamines de formule III 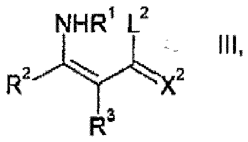 dans laquelle les variables X², R¹, R² et R³ ont les significations indiquées précédemment et L² représente alcoxy en C₁-C₆, halogénoalcoxy en C₁-C₆, alcoxy en C₁-C₄-alcoxy en C₂-C₄, alkylthio en C₁-C₄-alcoxy en C₂-C₄, alcényloxy en C₂-C₆, halogénoalcényloxy en C₂-C₆, alcynyloxy en C₃-C₆, halogénoalcynyloxy en C₃-C₆, cycloalkyloxy en C₃-c₈, cyanoalcoxy en C₁-C₆ ou benzyloxy, qui peut lui-même être halogéné en partie ou en totalité sur le cycle phényle et/ou qui peut être substitué par un à trois radicaux du groupe constitué par cyano, nitro, alkyle en C₁-C₄, alcoxy en C₁-C₄ et alkylthio en C₁-C₄ ;
en présence de 2,2 à 2,4 équivalents de base par rapport au carbamate de formule II.

2. Procédé de fabrication de 3-phényl(thio)uraciles et -dithiouraciles de formule I selon la revendication 1, **caractérisé en ce que** R¹ représente hydrogène ou alkyle en C₁-C₄.

3. Procédé de fabrication de 3-phényl(thio)uraciles et -dithiouraciles de formule I selon les revendications 1 et 2, **caractérisé en ce que** R² représente hydrogène, alkyle en C₁-C₄ ou halogénoalkyle en C₁-C₄.

4. Procédé de fabrication de 3-phényl(thio)uraciles et -dithiouraciles de formule I selon les revendications 1 à 3, **caractérisé en ce que** R³ représente hydrogène ou alkyle en C₁-C₄.

5. Procédé de fabrication de 3-phényl(thio)uraciles et -dithiouraciles de formule I selon les revendications 1 à 4, **caractérisé en ce que** X¹, X² et X³ représentent l'oxygène.

6. Procédé selon les revendications 1 à 5, **caractérisé en ce qu'**Ar représente un groupe de formule générale Ar-1 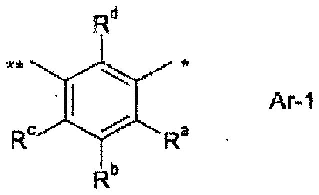 dans laquelle
* désigne la liaison d'Ar avec le groupe C(X³) ;
** désigne la liaison d'Ar avec l'atome d'azote directement voisin ;
R^{a} et R^{c} signifient indépendamment l'un de l'autre hydrogène, halogène, cyano ou halogénoalkyle en C₁-C₄ ; et
R^{b} et R^{d} signifient hydrogène.

7. Procédé selon les revendications 1 à 6, **caractérisé en ce que** la base est ajoutée de manière échelonnée pendant une période de temps.

8. Procédé de fabrication de 3-phényl(thio)uraciles et -dithiouraciles de formule I selon les revendications 1 à 7, **caractérisé en ce que** les carbamates de formule II sont fabriqués par mise en réaction d'amines de formule IV 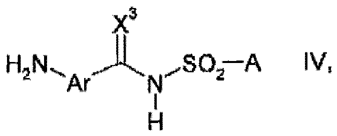 dans laquelle X³, Ar et A ont les significations indiquées dans la revendication 1,
avec des composés de formule V 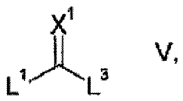 dans laquelle X¹ et L¹ ont les significations indiquées dans la revendication 1 et L³ représente chlore ou alcoxy en C₁-C₆.

9. Procédé de fabrication de 3-phényl(thio)uraciles et -dithiouraciles de formule I selon les revendications 1 à 8, **caractérisé en ce que** les carbamates de formule II sont halogénés lors d'une étape intermédiaire sur le radical Ar avec un agent d'halogénation.

10. Carbamates de formule II 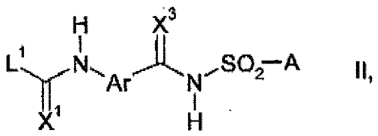 dans laquelle X¹, X³, Ar, A et L¹ ont les significations indiquées dans la revendication 1.

11. Procédé de fabrication de 3-phényl(thio)uraciles et -dithiouraciles de formule I selon la revendication 1 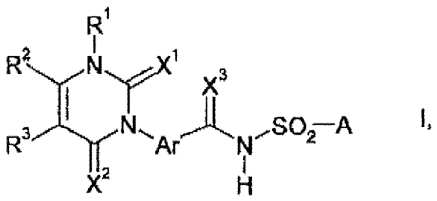 dans laquelle R¹ a les significations suivantes :
R¹ alkyle en C₁-C₆, cyanoalkyle en C₁-C₄, halogénoalkyle en C₁-C₆, cycloalkyle en C₃-C₈, alcényle en C₂-C₆, halogénoalcényle en C₂-C₆, alcynyle en C₃-C₆, halogénoalcynyle en C₃-C₆, phényl-alkyle en C₁-C₄ ou amino ;
et les variables R², R³, X¹, X², X³, Ar et A ont les significations indiquées dans les revendications 1 à 7,
**caractérisé en ce que** des carbamates de formule II 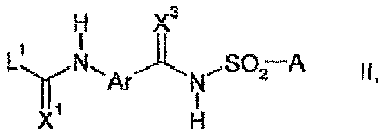 dans laquelle les variables X¹, X³, Ar et A ont les significations indiquées précédemment et L¹ représente alcoxy en C₁-C₆ ou alkylthio en C₁-C₆, sont mis en réaction avec des énamines de formule III 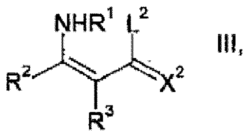 dans laquelle R¹ représente hydrogène, les variables X², R², R³ et L² ont les significations indiquées précédemment ;
et le 3-phényl(thio)uracile et -dithiouracile de formule I formé, dans laquelle R¹ représente hydrogène,
est ensuite
- alkylé avec un agent d'alkylation de formule VI
R¹-L⁴ VI
dans laquelle
R¹ signifie alkyle en C₁-C₆, cyanoalkyle en C₁-C₄, halogénoalkyle en C₁-C₅, cycloalkyle en C₃-C₈, alcényle en C₂-C₆, halogénoalcényle en C₂-C₆, alcynyle en C₃-C₆, halogénoalcynyle en C₃-C₆ ou phényl-alkyle en C₁-C₄ ; et
L⁴ signifie halogène, hydrogénosulfate, sulfate d'alkyle en C₁-C₆, sulfate, alkylsulfonyloxy en C₁-C₆, halogénoalkylsulfonyloxy en C₁-C₆ ou phénylsulfonyloxy, le cycle phényle étant éventuellement substitué une ou plusieurs fois avec halogène, nitro, alkyle en C₁-C₆ ou halogénoalkyle en C₁-C₆ ; en 3-phényl(thio)uraciles et -dithiouraciles de formule I
dans laquelle R¹ représente alkyle en C₁-C₆, cyanoalkyle en C₁-C₄, halogénoalkyle en C₁-C₆, cycloalkyle en C₃-C₈, alcényle en C₂-C₆, halogénoalcényle en C₂-C₆, alcynyle en C₃-C₆, halogénoalcynyle en C₃-C₆ ou phényl-alkyle en C₁-C₄;
ou
- aminé avec un agent d'amination de formule VII
H₂N-L⁵ VII
dans laquelle L⁵ représente halogène, hydrogénosulfate, alkylsulfonyloxy en C₁-C₆, halogénoalkylsulfonyloxy en C₁-C₆, phénylsulfonyloxy ou phényloxy, le cycle phényle étant éventuellement substitué une ou plusieurs fois avec halogène, nitro, alkyle en C₁-C₆ ou halogénoalkyle en C₁-C₆,
en 3-phényl(thio)uraciles et -dithiouraciles de formule I, dans laquelle R¹ représente NH₂.

12. Procédé de fabrication de 3-phényl(thio)uraciles et -dithiouraciles de formule I selon la revendication 11, dans lequel R¹ représente alkyle en C₁-C₆ ou amino.

13. Procédé de fabrication de 3-phényl(thio)uraciles et -dithiouraciles de formule I selon la revendication 11, dans lequel R¹ représente alkyle en C₁-C₆.
